# EUROPEAN PATENT APPLICATION

(11) **EP 4 790 422 A1**
(43) Date of publication of application: **12.08.2026**
(21) Application number: 24874688.5
(22) Date of filing: 03.10.2024
(51) Int. Cl.: G01N 33/86

(54) **METHOD FOR ESTIMATING FACTOR OF BLOOD COAGULATION ABNORMALITY**

(30) Priority: 03.10.2023 JP 2023172352
(71) Applicant: Sekisui Medical Co., Ltd., Tokyo 1030027 (JP); Public University Corporation Nara Medical University, Nara 6348521 (JP)
(72) Inventor: KAWABE, Toshiki, Tokyo 103-0027 (JP); ONISHI, Kengo, Tokyo 103-0027 (JP); NOGAMI, Keiji, Kashihara-shi, Nara 634-8521 (JP); OGIWARA, Kenichi, Kashihara-shi, Nara 634-8521 (JP); SHIMONISHI, Naruto, Kashihara-shi, Nara 634-8521 (JP)
(74) Representative: Wächtershäuser & Hartz Patentanwaltspartnerschaft mbB
(86) International application number: PCT/JP2024/035446
(87) International publication number: WO 2025/075088

(57) **Abstract**

Provided is a method for estimating a cause of coagulation disorders in a blood specimen. The method comprises 1) calculating a parameter Ps related to a coagulation reaction of a specimen S, and a parameter Pm related to a coagulation reaction of a specimen M; 2) estimating whether the specimen S is coagulation factor inhibitor-positive based on Ps; and 3) estimating that the specimen S which has not been estimated to be coagulation factor inhibitor-positive in 2) is lupus anticoagulant-positive or coagulation factor-deficient based on Pm or based on Ps and Pm. The specimen S is a subject blood specimen with a prolonged coagulation time, the specimen M is a mixed specimen of the specimen S and a specimen N, and the specimen N is a normal blood specimen. Ps is calculated based on a measurement point or time at which a coagulation reaction curve or coagulation rate curve of the specimen S reaches a predetermined value, and Pm is calculated based on a measurement point or time at which a coagulation reaction curve or coagulation rate curve of the specimen M reaches a predetermined value.

## Description

### Technical Field

The present invention relates to a method for estimating the cause of blood coagulation disorders.

### Background Art

A blood coagulation test is a test for diagnosing a patient's blood coagulation ability by adding a predetermined reagent to a patient's blood specimen, and measuring the blood coagulation time and the like. Typical examples of the blood coagulation time include prothrombin time (PT), activated partial thromboplastin time (APTT), thrombin time, and the like. In general, abnormalities in blood coagulation ability cause a prolonged coagulation time. Causes of abnormalities in blood coagulation ability include the effects of anticoagulants, decreased components involved in coagulation, the effect of autoantibodies on phospholipids which contribute to coagulation reactions (lupus anticoagulant), congenital deficiencies of blood coagulation factors (e.g., congenital hemophilia), appearance of autoantibodies (inhibitors) against blood coagulation factors, and the like. Examples of coagulation disorders caused by inhibitors include appearance of antibodies against FVIII (FVIII inhibitors) in patients with congenital hemophilia A, following the administration of factor VIII (FVIII) preparations. Another example is acquired hemophilia A (AHA). In AHA, autoantibodies against FVIII appear.

Conventionally, when a prolonged coagulation time is observed in a subject specimen in a blood coagulation test, and when the specimen is not suspected to contain an anticoagulant, a cross-mixing test is generally performed to estimate the cause of the prolonged coagulation time. In the cross-mixing test, a mixed specimen of a subject specimen and a normal specimen is prepared, and the APTT (immediate reaction) of the subject specimen, normal specimen, and mixed specimen immediately after mixing, and separately from these specimens, the APTT (delayed reaction) of each specimen after incubation at 37°C for 2 hours are measured. Based on the APTT patterns of the immediate reaction and delayed reaction, it is determined whether the cause of the prolonged APTT of the subject specimen is a coagulation factor inhibitor (anticoagulant factor), lupus anticoagulant (LA), or a coagulation factor deficiency such as hemophilia. However, the cross-mixing test requires a large amount of specimens as described above, takes time and requires effort, and does not allow quantitative determination.

Patent Literature 1 describes a method for acquiring information on the cause of the prolongation of coagulation time, the method including a step of acquiring first, second, and third coagulation times of a subject blood specimen, a normal blood specimen, and a mixed specimen of these specimens; a step of acquiring fourth, fifth, and sixth coagulation times of the subject blood specimen, normal blood specimen, and mixed specimen after incubation at a predetermined temperature for 45 minutes to 4 hours; a step of acquiring a first quantification index based on the first, second, and third coagulation times; a step of acquiring a second quantification index based on the fourth, fifth, and sixth coagulation times; and a step of acquiring a value of the ratio or difference between the first quantification index value and the second quantification index value, or a value combining the ratio value and the difference value; wherein the value is information suggesting whether the subject blood specimen is suspected to contain a coagulation factor inhibitor.

Patent Literature 2 describes a blood specimen determination method, including a step of preparing a mixed plasma by mixing a test plasma and a normal plasma; a step of acquiring a maximum coagulation rate, maximum coagulation acceleration, and maximum coagulation deceleration, which are parameters related to the derivative of the coagulation waveform of the mixed plasma; and a step of determining whether the test plasma is suspected to be a coagulation factor-deficient plasma, or a plasma containing lupus anticoagulant (LA) or a coagulation factor inhibitor, based on the acquired parameter values.

Patent Literature 3 describes a blood analysis method, including preparing a mixed specimen by mixing a subject blood specimen and a normal blood specimen, heating the mixed specimen, acquiring coagulation reaction data on the heated mixed specimen and an unheated mixed specimen, acquiring derivative curves of the coagulation reaction data, calculating a parameter related to the centroid point of the derivative curve for the unheated mixed specimen as a first parameter, calculating a parameter related to the centroid point of the derivative curve for the heated mixed specimen as a second parameter, and evaluating the coagulation properties of the subject blood specimen based on the ratio or difference between the first parameter and the second parameter.

Patent Literature 4 describes a method for estimating the cause of prolonged coagulation time, the method including detecting a coagulation reaction end point Pe in a coagulation reaction curve of a subject blood specimen with a prolonged coagulation time, calculating a curve T(X) which represents a measurement point or time at which the coagulation reaction curve reaches X% of Pe, and estimating the cause of the prolonged coagulation time of the subject blood specimen based on the shape of T(X).

Patent Literature 5 describes a method for analyzing a blood coagulation reaction, the method including determining points pk and qk at which the coagulation rate curve V(i) of a subject blood specimen reaches predetermined values before and after reaching a maximum value Vmax, and estimating the cause of blood coagulation disorders in the blood specimen based on parameters calculated using pk and qk.

Patent Literature 6 states that it is possible to distinguish between an LA-positive specimen and a coagulation factor inhibitor-positive specimen based on the maximum coagulation rate, maximum coagulation acceleration, or maximum coagulation deceleration calculated from the coagulation waveform of a blood specimen.

### Citation List

### Patent Literature

Patent Literature 1: JP-B-6696963
Patent Literature 2: JP-B-6871673
Patent Literature 3: WO 2021/229556
Patent Literature 4: WO 2022/186381
Patent Literature 5: WO 2022/054819
Patent Literature 6: JP-A-2016-118442

### Summary of Invention

### Technical Problem

The present invention provides a method for estimating the cause of coagulation disorders in blood specimens.

### Solution to Problem

That is, the present invention provides the following.
[1] A method for estimating a cause of coagulation disorders in a blood specimen, the method comprising:
   1) calculating a parameter Ps related to a coagulation reaction of a specimen S, and a parameter Pm related to a coagulation reaction of a specimen M,
      wherein
      the specimen S is a subject blood specimen with a prolonged coagulation time,
      the specimen M is a mixed specimen of the specimen S and a specimen N,
      the specimen N is a normal blood specimen,
      Ps is calculated based on Ts(X), or vT1s(X) and vT2s(X), and
      Pm is calculated based on Tm(X), vT2m(X) , vT1m(X), and vT2m(X), or based on a first derivative curve of a coagulation reaction curve of the specimen M,
      wherein
      Ts(X) represents a measurement point or time at which a coagulation reaction curve of the specimen S reaches X% of Es, and Es is a coagulation reaction end point in the coagulation reaction curve of the specimen S,
      vT1s(X) represents a measurement point or time at which a coagulation rate curve of the specimen S reaches X% of a maximum value before reaching the maximum value,
      vT2s(X) represents a measurement point or time at which the coagulation rate curve of the specimen S reaches X% of a maximum value after reaching the maximum value,
      Tm(X) represents a measurement point or time at which a coagulation reaction curve of the specimen M reaches X% of Em, and Em is a coagulation reaction end point in the coagulation reaction curve of the specimen M,
      vT1m(X) represents a measurement point or time at which a coagulation rate curve of the specimen M reaches X% of a maximum value before reaching the maximum value,
      vT2m(X) represents a measurement point or time at which the coagulation rate curve of the specimen M reaches X% of a maximum value after reaching the maximum value, and
         $0 < X \leq 100 ;$
   2) estimating whether the specimen S is coagulation factor inhibitor-positive based on Ps; and
   3) estimating that the specimen S which has not been estimated to be coagulation factor inhibitor-positive in 2) is lupus anticoagulant-positive or coagulation factor-deficient based on Pm or based on Ps and Pm.
[2] The method according to [1], wherein Ps is selected from the group consisting of the following (a) to (j):
   (a) rTs(X1)
      (wherein $rTs \left(X1\right) = Ts \left(X1\right) / Ts \left(As\right) \times Qs$ X1 = 35 to 95, As = 3 to 75, X1>As, and Qs>0);
   (b) $\left\{rTs\left(X2\right)-rTs\left(X1\right)\right\} / \left(X2-X1\right) ,$ (wherein rTs(X1) and rTs(X2) are defined in accordance with the above equation (IIa'), provided that X1 = 1 to 85, X2 = 25 to 95, (X2-X1) = 5 to 94, As = 5 to 95, and Qs>0) ;
   (c) rSLs[X1:X2]
      (wherein $rSLs \left[X1:X2\right] = \frac{{\sum }_{X = X 1}^{X 2} \left\{\left(X-UX\right)\times \left(rTs\left(X\right)-UrTs\right)\right\}}{{\sum }_{X = X 1}^{X 2} {\left(X-UX\right)}^{2}} \times Qs$
      UX is an average from X1 to X2,
      UrTs is an average from rTs(X1) to rTs(X2),
      rTs(X2 is defined in accordance with the above equation (IIa'),
      X1 = 1 to 85, X2 = 25 to 100, (X2-X1) = 5 to 99, As = 5 to 95, and Qs>0);
   (d) wXs[X1:X2]
      (wherein $wXs \left[X1:X2\right] = \frac{{\sum }_{X = X 1}^{X 2} \left\{X\times Ts\left(X\right)\right\}}{{\sum }_{X = X 1}^{X 2} Ts \left(X\right)} \times Qs$ X1 = 1 to 75, X2 = 30 to 100, (X2-X1) = 10 to 99, and Qs>0);
   (e) $vT 2 s \left(X1\right) / vT 1 s \left(X2\right)$ (wherein X1 = 3 to 58 and X2 = 13 to 98);
   (f) $vT 2 s \left(X1\right) / vT 2 s \left(X2\right)$ (wherein X1 = 13 to 63, X2 = 58 to 83, and X2>X1);
   (g) $\left(vT2s\left(X1\right)-vT1s\left(X2\right)\right) / vT 2 s \left(Bs\right)$ (wherein X1 = 3 to 58, X2 = 13 to 98, and Bs = 3 to 100);
   (h) $\left(vT2s\left(X1\right)-vT1s\left(X2\right)\right) / vT 1 s \left(Bs\right)$ (wherein X1 = 3 to 58, X2 = 3 to 93, and Bs = 3 to 100);
   (i) $\left(vT2s\left(X1\right)-vT2s\left(X2\right)\right) / vT 2 s \left(Bs\right)$ (wherein X1 = 8 to 58, X2 = 48 to 83, X2>X1, and Bs = 3 to 100); and
   (j) $\left(vT2s\left(X1\right)-vT2s\left(X2\right)\right) / vT 1 s \left(Bs\right)$ (wherein X1 = 8 to 58, X2 = 48 to 98, X2>X1, and Bs = 3 to 100), and
      wherein Pm is selected from the group consisting of the following (a') to (p'):
      (a') Tm(X1) (wherein X1 = 90 to 100);
      (b') rTm(X1)
         (wherein $rTm \left(X1\right) = Tm \left(X1\right) / Tm \left(Am\right) \times Qm$ X1 = 30 to 95, Am = 1 to 35, X1-Am = 10 to 94, and Qm>0);
      (c') aTm(X1,X2)
         (wherein $aTm \left(X1,X2\right) = \left\{Tm\left(X2\right)-Tm\left(X1\right)\right\} / \left(X2-X1\right) \times Qm ,$ X1 = 1 to 90, X2 = 25 to 100, X2-X1 = 5 to 99, and Qm>0);
      (d') SLm[X1:X2]
         (wherein $SLm \left[X1:X2\right] = \frac{{\sum }_{X = X 1}^{X 2} \left\{\left(X-UX\right)\times \left(Tm\left(X\right)-UTm\right)\right\}}{{\sum }_{X = X 1}^{X 2} {\left(X-UX\right)}^{2}} \times Qm$
         UX is an average from X1 to X2,
         UTm is an average from Tm(X1) to Tm(X2),
         X1 = 1 to 95, X2 = 30 to 100, (X2-X1) = 5 to 99, and Qm>0) ;
      (e') rSLm[X1:X2]
         (wherein $rSLm \left[X1:X2\right] = \frac{{\sum }_{X = X 1}^{X 2} \left\{\left(X-UX\right)\times \left(rTm\left(X\right)-UrTm\right)\right\}}{{\sum }_{X = X 1}^{X 2} {\left(X-UX\right)}^{2}} \times Qm$
         rTm(X) is defined in accordance with the above equation (IIb'),
         UX is an average of X from X1 to X2,
         UrTm is an average of rTm(X) from rTm(X1) to rTm(X2),
         X1 = 1 to 70, X2 = 25 to 100, (X2-X1) = 5 to 99, Am = 5 to 95, and Qm>0);
      (f') wXm[X1:X2]
         (wherein $wXm \left[X1:X2\right] = \frac{{\sum }_{X = X 1}^{X 2} \left\{X\times Tm\left(X\right)\right\}}{{\sum }_{X = X 1}^{X 2} Tm \left(X\right)} \times Qm$ X1 = 1 to 45, X2 = 30 to 100, (X2-X1) = 10 to 99, and Qm>0) ;
      (g') wTm[X1:X2]
         (wherein $wTm \left[X1:X2\right] = \frac{{\sum }_{X = X 1}^{X 2} \left\{X\times Tm\left(X\right)\right\}}{{\sum }_{X = X 1}^{X 2} X} \times Qm$ X1 = 75 to 95, X2 = 95 to 100, (X2-X1) = 5 to 25, and Qm>0) ;
      (h') wrTm[X1:X2]
         (wherein $wrTm \left[X1:X2\right] = \frac{{\sum }_{X = X 1}^{X 2} \left\{X\times rTm\left(X\right)\right\}}{{\sum }_{X = X 1}^{X 2} X}$
         rTm(X) is defined in accordance with the above equation (IIb'),
         X1 = 1 to 90, X2 = 30 to 100, (X2-X1) = 5 to 99, Am = 3 to 34, and Qm>0);
      (i') $vT 2 m \left(X1\right) / vT 1 m \left(X2\right)$ (wherein X1 = 3 to 93 and X2 = 3 to 88);
      (j') $vT 2 m \left(X1\right) / vT 2 m \left(X2\right)$ (wherein X1 = 38 to 63, X2 = 63 to 93, and X2>X1);
      (k') $\left(vT2m\left(X1\right)-vT1m\left(X2\right)\right) / vT 2 s \left(Bs\right)$ (wherein X1 = 3 to 93, X2 = 3 to 88, and Bm = 3 to 100);
      (l') $\left(vT2m\left(X1\right)-vT2m\left(X2\right)\right) / vT 2 s \left(Bs\right)$ (wherein X1 = 28 to 63, X2 = 68 to 93, X2>X1, and Bm = 3 to 100);
      (m') $\left(vT2m\left(X1\right)-vT1m\left(X2\right)\right) / vT 1 s \left(Bs\right)$ (wherein X1 = 3 to 93, X2 = 3 to 93, and Bm = 3 to 100);
      (n') $\left(vT2m\left(X1\right)-vT2m\left(X2\right)\right) / vT 1 s \left(Bs\right)$ (wherein X1 = 23 to 63, X2 = 63 to 93, X2>X1, and Bm = 3 to 100);
      (o') iCmax_m
         (wherein iCmax_m is a reciprocal of Cmax_m,
         Cmax_m is a maximum value of Cm(i), $Cm \left(i\right) = Vm \left(i\right) \times \left(Q/Em\right)$
         in the equation (XIb), Vm(i) is a first derivative curve of a coagulation reaction curve of the specimen M, i is time or the number of measurement points, and Q>0); and
      (p') iwCm[X1:X2]
         (wherein $iwCm \left[X1:X2\right] = \frac{{\sum }_{i = X 1}^{X 2} i}{{\sum }_{i = X 1}^{X 2} \left\{i\times Cm\left(i\right)\right\}} \times Qm$
         Cm(i) is defined in accordance with the above equation (XIb),
         X1 and X2 satisfy Cm(X1) = Cm(X2) = Vm(i)×[Q/(Em×S%)], provided that X1<VmaxT_m<X2 and Qm>0,
         S = 1 to 100, and
         VmaxT_m is time or a measurement point at which the first derivative curve of the coagulation reaction curve of the specimen M reaches a maximum value).
[3] The method according to [1] or [2], wherein
   2) comprises estimating that the specimen S is coagulation factor inhibitor-positive when Ps is equal to or larger than a threshold value for Ps; and
   3) comprises estimating that the specimen S is lupus anticoagulant-positive when Pm is equal to or larger than a threshold value for Pm, or that the specimen S is coagulation factor-deficient when Pm is equal to or less than the threshold value for Pm.
[4] The method according to any one of [1] to [3], which comprises, as 2) and 3), plotting Ps and Pm of the specimen S on a two-dimensional map, and estimating that the specimen S is coagulation factor inhibitor-positive, lupus anticoagulant-positive, or coagulation factor-deficient, based on the position of the plotted point on the two-dimensional map.
[5] The method according to any one of [1] to [4], which comprises:
   calculating a parameter Pd for a specimen S which has been estimated to be lupus anticoagulant-positive, and
   estimating whether the specimen S is suspected to be derived from a patient with acquired hemophilia A based on Pd,
   wherein
   Pd is calculated based on Td(X), $Td \left(X\right) = Ts \left(X\right) - Tm \left(X\right) ,$ and $0 < X \leq 100 .$
[6] The method according to [5], wherein Pd is selected from the group consisting of the following (a") to (e"):
   (a") Td(X1) (wherein X1 = 43 to 100);
   (b") Td(X2)-Td(X1) (wherein X1 = 1 to 80, X2 = 15 to 100, and X2-X1 = 5 to 99);
   (c") SLd[X1:X2]
      (wherein $SLd \left[X1:X2\right] = \frac{{\sum }_{X = X 1}^{X 2} \left\{\left(X-UX\right)\times \left(Td\left(X\right)-UTd\right)\right\}}{{\sum }_{X = X 1}^{X 2} {\left(X-UX\right)}^{2}} \times Qd$ X1 = 1 to 95, X2 = 25 to 100, X2-X1 = 5 to 99, UX is an average of X from X1 to X2, UTd is an average of Td(X) from Td(X1) to Td(X2), and Qd>0);
   (d") wXd[X1:X2]
      (wherein $wXd \left[X1:X2\right] = \frac{{\sum }_{X = X 1}^{X 2} \left\{X\times Td\left(X\right)\right\}}{{\sum }_{X = X 1}^{X 2} Td \left(X\right)} \times Qd$ X1 = 1 and X2 = 50 to 90, or X1 = 15 to 25, X2 = 25 to 45, and X2-X1 = 5 to 30, and Qd>0); and
   (e") wTd[X1:X2]
      (wherein $wTd \left[X1:X2\right] = \frac{{\sum }_{X = X 1}^{X 2} \left\{X\times Td\left(X\right)\right\}}{{\sum }_{X = X 1}^{X 2} X} \times Qd$ X1 = 1 to 95, X2 = 45 to 100, X2-X1 = 5 to 99, and Qd>0).
[7] The method according to [5] or [6], which comprises estimating that the specimen S is suspected to be derived from a patient with acquired hemophilia A when Pd is equal to or larger than a threshold value for Pd.
[8] A program for implementing the method according to any one of [1] to [7].
[9] A device for implementing the method according to any one of [1] to [7].

### Advantageous Effects of Invention

The method of the present invention makes it possible to quantitatively estimate the cause of coagulation disorders in blood specimens without creating APTT patterns of immediate reaction and delayed reaction for each specimen as in conventional cross-mixing tests. The method of the present invention enables quicker and simpler estimation of the cause of coagulation disorders in blood specimens.

### Brief Description of Drawings

Fig. 1 illustrates an example of the coagulation reaction curve.
Fig. 2A illustrates the explanation of T(X). The time points at which T(X) reaches 10%, 50%, and 90% of a coagulation reaction end point E on a coagulation reaction curve are shown as T(10), T(50), and T(90), respectively. Fig. 2B illustrates the changes in T(X) and vT(X) with respect to X. vT(X) is shown divided into vT1(X) and vT2(X).
Fig. 3 illustrates T(X) in which APTT is about 100 seconds by specimen type. A: Ts(X), B: Tm(X) at a mixing ratio of 1:1. Solid line: LA, dashed line: dFVIII, chain line: iFVIII. C: iFVIII (APTT: 107 seconds), D: dFVIII (APTT: 101 seconds), E: LA (APTT: 108 seconds). Solid line: Ts, dashed line: Tm.
Fig. 4 illustrates a conceptual diagram showing the configuration of an automated analyzer for performing the method for estimating the cause of coagulation disorders according to the present invention.
Fig. 5 illustrates rTs(X1) of subject specimens. LA: LA-positive specimen. Def: coagulation factor-deficient specimen, and 8, 9, and 5 indicate FVIII, FIX, and FV, respectively. Inh: coagulation factor inhibitor-positive specimen, and 8 and 9 indicate FVIII and FIX, respectively.
Fig. 6 illustrates the difference between the minimum value of rTs(X1) in the Inh group and the maximum value of rTs(X1) in the LA and Def groups. The gray columns indicate that the minimum value in the Inh group is larger than the maximum value in the LA and Def groups; otherwise, it is indicated with ×.
Fig. 7 illustrates rTs(X2)-rTs(X1) of subject specimens. The horizontal axis label is the same as in Fig. 5.
Fig. 8 illustrates the difference between the minimum value of rTs(X2)-rTs(X1) in the Inh group and the maximum value of rTs(X2)-rTs(X1) in the LA and Def groups. The gray columns indicate that the minimum value in the Inh group is larger than the maximum value in the LA and Def groups; otherwise, it is indicated with ×.
Fig. 9 illustrates arTs(X1,X2) of subject specimens. The horizontal axis label is the same as in Fig. 5.
Fig. 10 illustrates the difference between the minimum value of arTs(X1,X2) in the Inh group and the maximum value of arTs(X2,X1) in the LA and Def groups. As = 5. The gray columns indicate that the minimum value in the Inh group is larger than the maximum value in the LA and Def groups; otherwise, it is indicated with ×.
Fig. 11: the same as Fig. 10. As = 50.
Fig. 12: the same as Fig. 10. As = 95.
Fig. 13 illustrates rSLs[X1:X2] of subject specimens. The horizontal axis label is the same as in Fig. 5.
Fig. 14 illustrates the difference between the minimum value of rSLs[X1:X2] in the Inh group and the maximum value of rSLs[X1:X2] in the LA and Def groups. As = 5. The gray columns indicate that the minimum value in the Inh group is larger than the maximum value in the LA and Def groups; otherwise, it is indicated with ×.
Fig. 15: the same as Fig. 14. As = 50.
Fig. 16: the same as Fig. 14. As = 95.
Fig. 17 illustrates wXs[X1:X2] of subject specimens. The horizontal axis label is the same as in Fig. 5.
Fig. 18 illustrates the difference between the minimum value of wXs[X1:X2] in the Inh group and the maximum value of wXs[X1:X2] in the LA and Def groups. The gray columns indicate that the minimum value in the Inh group is larger than the maximum value in the LA and Def groups; otherwise, it is indicated with ×.
Fig. 19 illustrates vT2s(X1)/vT1s(X2) of subject specimens. The horizontal axis label is the same as in Fig. 5.
Fig. 20 illustrates the difference between the minimum value in the Inh group and the maximum value in the LA and Def groups for vT2s(X1) /vT1s(X2) and vT2s(X1)/vT2s(X2). The gray columns indicate that the minimum value in the Inh group is larger than the maximum value in the LA and Def groups; otherwise, it is indicated with ×.
Fig. 21 illustrates the relative value of the difference in vTs(X) for subject specimens. The horizontal axis label is the same as in Fig. 5.
Fig. 22 illustrates the difference between the minimum value in the Inh group and the maximum value in the LA and Def groups for the relative value of the difference in vTs(X). The gray columns indicate that the minimum value in the Inh group is larger than the maximum value in the LA and Def groups; otherwise, it is indicated with ×.
Fig. 23: the same as Fig. 22.
Fig. 24: the same as Fig. 22.
Fig. 25 illustrates Tm(X1) of subject specimens. The horizontal axis label is the same as in Fig. 5.
Fig. 26 illustrates rTm(X1) of subject specimens. The horizontal axis label is the same as in Fig. 5.
Fig. 27 illustrates the difference between the minimum value of rTm(X1) in the LA group and the maximum value of rTm(X1) in the Def group. The gray columns indicate that the minimum value in the LA group is larger than the maximum value in the Def group; otherwise, it is indicated with ×.
Fig. 28 illustrates SLm[X1:X2] of subject specimens. The horizontal axis label is the same as in Fig. 5.
Fig. 29 illustrates the difference between the minimum value of SLm[X1:X2] in the LA group and the maximum value of SLm[X1:X2] in the Def group. The gray columns indicate that the minimum value in the LA group is larger than the maximum value in the Def group; otherwise, it is indicated with ×.
Fig. 30 illustrates rSLm[X1:X2] of subject specimens. The horizontal axis label is the same as in Fig. 5.
Fig. 31 illustrates the difference between the minimum value of rSLm[X1:X2] in the LA group and the maximum value of rSLm[X1:X2] in the Def group. Am = 5. The gray columns indicate that the minimum value in the LA group is larger than the maximum value in the Def group; otherwise, it is indicated with ×.
Fig. 32: the same as Fig. 31. Am = 50.
Fig. 33: the same as Fig. 31. Am = 95.
Fig. 34 illustrates the following: A: wXm[X1:X2], B: wTm[X1:X2], and C: wrTm[X1:X2] of subject specimens. The horizontal axis label is the same as in Fig. 5.
Fig. 35 illustrates the difference between the minimum value of wXm[X1:X2] in the LA group and the maximum value of wXm[X1:X2] in the Def group. The gray columns indicate that the minimum value in the LA group is larger than the maximum value in the Def group; otherwise, it is indicated with ×.
Fig. 36 illustrates the difference between the minimum value of wTm[X1:X2] in the LA group and the maximum value of wTm[X1:X2] in the Def group. The gray columns indicate that the minimum value in the LA group is larger than the maximum value in the Def group; otherwise, it is indicated with ×.
Fig. 37A illustrates the difference between the minimum value of wrTm[X1:X2] in the LA group and the maximum value of wrTm[X1:X2] in the Def group. Am = 3. The gray columns indicate that the minimum value in the LA group is larger than the maximum value in the Def group; otherwise, it is indicated with ×.
Fig. 37B: the same as Fig. 37. Am = 10.
Fig. 37C: the same as Fig. 37. Am = 34.
Fig. 38 illustrates aTm(X1,X2) of subject specimens. The horizontal axis label is the same as in Fig. 5.
Fig. 39 illustrates the difference between the minimum value of aTm(X1,X2) in the LA group and the maximum value of aTm(X1,X2) in the Def group. The gray columns indicate that the minimum value in the LA group is larger than the maximum value in the Def group; otherwise, it is indicated with ×.
Fig. 40A illustrates the ratio of vTm(X) and vTm(X) for subject specimens. The horizontal axis label is the same as in Fig. 5.
Fig. 40B illustrates the difference between the minimum value in the LA group and the maximum value in the Def group for vT2m(X1)/vT1m(X2) and vT2m(X1)/vT2m(X2). The gray columns indicate that the minimum value in the LA group is larger than the maximum value in the Def specimen group; otherwise, it is indicated with ×.
Fig. 41 illustrates the relative value of the difference in vTm(X) for subject specimens. The horizontal axis label is the same as in Fig. 5.
Fig. 42 illustrates the difference between the minimum value in the LA group and the maximum value in the Def group for the relative value of the difference in vTm(X). The gray columns indicate that the minimum value in the LA group is larger than the maximum value in the Def group; otherwise, it is indicated with ×.
Fig. 43: the same as Fig. 42.
Fig. 44: the same as Fig. 42.
Fig. 45 illustrates iCmax_m and iwCm[X] of subject specimens.
Fig. 46 illustrates two-dimensional maps plotting (Ps, Pm) for subject specimens. In the figures, the circle indicates LA, the triangle indicates iFVIII, the square indicates iFIX, the cross indicates dFVIII, the plus sign indicates dFIX, and the asterisk indicates dFV. The dotted line parallel to the Y-axis indicates a threshold value Ls, and the dotted line parallel to the X-axis indicates a threshold value Lm. The diagonal line indicates a threshold line Lc.
Fig. 47 illustrates the following: A: Ts(50), B: Vmax, C: (1/Vmax)×100, and D: VmaxT for subject specimens. The horizontal axis label is the same as in Fig. 5.
Fig. 48 illustrates the following: A: Tm(50), B: Vmax, C: (1/Vmax)×100, and D: VmaxT for mixed specimens. The horizontal axis label is the same as in Fig. 5.
Fig. 49A illustrates two-dimensional maps plotting (Ps, Pm) of subject specimens using Pm of mixed specimens with a mixing ratio (subject specimen:NPP) of 1:1. dFVIII, dFIX, and dFV indicate FVIII-deficient, FIX-deficient, and FV-deficient specimens, respectively, and iFVIII and iFIX indicate FVIII inhibitor-positive and FIX inhibitor-positive specimens, respectively. The dotted line parallel to the Y-axis indicates a threshold value Ls, and the dotted line parallel to the X-axis indicates a threshold value Lm.
Fig. 49B illustrates two-dimensional maps plotting (Ps, Pm) of subject specimens using Pm of mixed specimens with a mixing ratio (subject specimen:NPP) of 9:1. The display and dotted lines in each figure are the same as those in Fig. 49A.
Fig. 49C illustrates two-dimensional maps plotting (Ps, Pm) of subject specimens using Pm of mixed specimens with a mixing ratio (subject specimen:NPP) of 1:9. The display and dotted lines in each figure are the same as those in Fig. 49A.
Fig. 50 illustrates two-dimensional maps plotting (Ps, Pm) for AHA and LA. The dotted line parallel to the Y-axis indicates a threshold value Ls, and the dotted line parallel to the X-axis indicates a threshold value Lm. The mixing ratios of the mixed specimens used (subject specimen:NPP) are 9:1 (A), 1:1 (B), and 1:9 (C).
Fig. 51 illustrates the comparison of T(X) between AHA-a estimated to be LA, AHA-b estimated to be Inh, and LA in the AHA group. Solid line: AHA-a, dashed line: AHA-b, chain line: LA. A: Ts(X), B: Tm(X) of mixed specimens with a mixing ratio (subject specimen:NPP) of 1:1, C: Td(X).
Fig. 52 illustrates Td(X) of specimen S and NPP by mixing ratio for AHA-a, AHA-b, and LA. The mixing ratios of specimen S to NPP are 9:1 (A), 1:1 (B), and 1:9 (C).
Fig. 53A illustrates Pd = Td(X2)-Td(X1) in the AHA group and LA group. The horizontal axis labels are as follows: LA: LA-positive specimen, AHA: acquired hemophilia A specimen. Fig. 53B illustrates the difference between the maximum value in the LA group and the minimum value in the AHA group for Pd (= Td(X2)-Td(X1)). The gray columns indicate that the minimum value in the AHA group is larger than the maximum value in the LA group; otherwise, it is indicated with ×.
Fig. 54A illustrates Pd = SLd[X1:X2] in the AHA group and LA group, and Fig. 54B illustrates the difference between the maximum value in the LA group and the minimum value in the AHA group for Pd (= SLd[X1:X2]). The horizontal axis labels of Fig. A and the explanation of Fig. B are same as those in Fig. 53.
Fig. 55A illustrates Pd = wXd[X1:X2] in the AHA group and LA group, and Fig. 55B illustrates the difference between the maximum value in the LA group and the minimum value in the AHA group for Pd (= wXd[X1:X2]). The horizontal axis labels of Fig. A and the explanation of Fig. B are same as those in Fig. 53.
Fig. 56A illustrates Pd = wTd[X1:X2] in the AHA group and LA group, and Fig. 56B illustrates the difference between the maximum value in the LA group and the minimum value in the AHA group for Pd (= wTd[X1:X2]). The horizontal axis labels of Fig. A and the explanation of Fig. B are same as those in Fig. 53.
Fig. 57 illustrates two-dimensional maps plotting (Pd, Pm) for AHA and LA. The dotted line parallel to the Y-axis indicates a threshold value Ld, and the dotted line parallel to the X-axis indicates a threshold value Lm. The mixing ratios of the mixed specimens used (subject specimen: NPP) are 9:1 in Fig. A, 1:1 in Fig. B, and 1:9 in Fig. C.
Fig. 58 illustrates two-dimensional maps created by converting the two-dimensional maps of Fig. 49A into index ratios (Ps ratio, Pm ratio).
Figs. 59A to C illustrate the relationship between the factor activity and the Ps ratio (A: FVIII, B: FIX, C: FV), and Fig. 59D illustrates the relationship between the titer and the Pm ratio (triangle: iFVIII, square: iFIX).
Fig. 60 illustrates a two-dimensional map plotting (Ps ratio, Pm ratio) for LA, iFVIII, and AHA.
Fig. 61 illustrates two-dimensional maps for LA and AHA. A: plotted point of (Ps ratio, Pm ratio), B: plotted point of (Pd ratio, Pm ratio).
Fig. 62 illustrates a flow for estimating the cause of coagulation disorders in specimens with a known APTT prolongation.
Fig. 63 illustrates a flow for measuring APTT and estimating the cause of coagulation disorders for specimens with an unknown APTT.
Fig. 64 illustrates a flow for, after finding an APTT prolongation specimen, subsequently estimating the cause of coagulation disorders in the specimen.
Fig. 65 illustrates a detailed flow for comparing parameters and estimating the cause of coagulation disorders.
Fig. 66 illustrates a flow for estimating the cause of coagulation disorders, including Inh-X estimation.

### Description of Embodiments

In a blood coagulation test, a predetermined reagent is added to a subject blood specimen, the subsequent blood coagulation reaction is measured, and the blood coagulation time is measured from the coagulation reaction. Many abnormal specimens with blood coagulation disorders have a prolonged coagulation time in comparison to normal specimens. In the present specification, the blood coagulation reaction, blood coagulation time, blood coagulation disorders, and blood specimen are also simply referred to as the "coagulation reaction," "coagulation time," "coagulation disorders," and "specimen," respectively. The prolonged coagulation time is an indicator of the presence or absence of coagulation disorders. On the other hand, the cause of coagulation disorders (e.g., cause of prolonged coagulation time) cannot be estimated from the coagulation time.

In a blood coagulation test, if a prolongation of the coagulation time, such as APTT, is observed in a subject specimen, a cross-mixing test is then performed in conventional general procedures to determine the cause of the prolonged coagulation time (hereinafter simply referred to as the "cause of prolongation") unless the subject specimen contains an anticoagulant, such as heparin. Specifically, in the cross-mixing test, it is determined whether the cause of APTT prolongation is a coagulation factor inhibitor (anticoagulant factor), lupus anticoagulant (LA), or a coagulation factor deficiency such as hemophilia. In the cross-mixing test, the APTT (immediate reaction) of a normal specimen, a subject specimen, and a mixed specimen of test and normal specimens immediately after preparation of the specimen, and the APTT (delayed reaction) of each specimen after incubation at 37°C for 2 hours are measured. The cause of APTT prolongation is determined based on the APTT patterns of these immediate and delayed reactions. With conventional methods, it is not possible to determine the cause of prolongation in the subject specimen without performing a cross-mixing test separately from coagulation time measurement. However, the cross-mixing test requires a large amount of specimens to create APTT patterns of immediate reaction and delayed reaction for each specimen, takes time and requires effort, and requires a lot of time to obtain the results.

In the present invention, it is possible to estimate the cause of coagulation disorders in specimens using a smaller amount of specimens without the need to further prepare specimens which have been subjected to long-term incubation treatment as specimen pretreatment for delayed reaction measurement in the conventional cross-mixing test described above. In addition, the series of analysis steps can be easily automated.

### [Method for estimating cause of coagulation disorders]

The present invention provides a method for estimating the cause of coagulation disorders in blood specimens. In the method for estimating the cause of coagulation disorders in blood specimens according to the present invention (hereinafter also referred to as the method of the present invention), a blood specimen with a prolonged coagulation time is used as a subject blood specimen (hereinafter also referred to as a subject specimen), and the cause of coagulation disorders (i.e., the cause of the prolonged coagulation time) in the subject specimen is estimated.

### 1. Specimen

Plasma is preferably used as the blood specimen. Any anticoagulant generally used for coagulation tests can be added to the specimen. For example, blood is collected using a blood collection tube containing a sodium citrate aqueous solution, and then centrifuged to obtain plasma.

In a general blood coagulation test, time-series data of blood coagulation reactions (e.g., coagulation reaction curves) are obtained from specimens, and the coagulation time is calculated based on the data. A specimen with a longer coagulation time than a normal value (e.g., a reference value determined based on the coagulation time of a normal specimen group) is selected as a specimen with a prolonged coagulation time, and can be used as a subject specimen in the method of the present invention. Examples of the calculated coagulation time include, but are not limited to, prothrombin time (PT) and activated partial thromboplastin time (APTT).

In the method of the present invention, the coagulation reaction curves of the subject specimen and a mixed specimen of the subject specimen and a normal specimen are used. The normal specimen refers to a normal blood specimen which does not have a prolonged coagulation time. The normal specimen for use can be a blood specimen from a person without blood coagulation disorders, for example, a blood specimen collected from a healthy person, preferably plasma, a pooled specimen thereof, or commercially available normal plasma. In the preparation of a mixed specimen, a subject specimen and a normal specimen are mixed at a predetermined ratio. The mixing ratio of the subject specimen and the normal specimen, subject specimen:normal specimen, may be in the range from 1:9 to 9:1 as the capacity ratio.

In the present specification, the subject specimen is also referred to as the specimen S, the normal specimen as the specimen N, and the mixed specimen as the specimen M. Further, the subject specimen and mixed specimen, or the subject specimen, normal specimen, and mixed specimen, are also collectively referred to simply as the "specimens."

### 2. Measurement of coagulation reaction

The blood coagulation reactions of the subject specimen and mixed specimen are measured. The coagulation reaction curve of each specimen can be obtained from the time-series data of the coagulation reactions obtained by this measurement. The coagulation reaction measurement can be performed in accordance with the procedure of general coagulation reaction measurement performed in the measurement of coagulation times, such as prothrombin time (PT) and activated partial thromboplastin time (APTT), or the measurement of fibrinogen concentration (Fbg). In the present specification below, the method of the present invention will be explained mainly based on the measurement of coagulation reaction by APTT measurement. A person skilled in the art would be able to implement modifications of the method of the present invention to other measurement methods (e.g., measurement of coagulation reaction by PT measurement).

In the measurement of coagulation reaction, a coagulation time measurement reagent is added to the specimen to initiate the blood coagulation reaction. The coagulation reaction of the mixture (reaction liquid) after the addition of the reagent can be measured. The reagent for use can be selected freely depending on the purpose of measurement. Reagents for measuring various coagulation times are commercially available (e.g., APTT reagent Coagpia APTT-N; manufactured by SEKISUI MEDICAL CO., LTD.). For the measurement of coagulation reaction, a general method, such as an optical method for measuring the amount of scattered light, transmittance, absorbance, or the like, or a mechanical method for measuring the viscosity of plasma, may be used. In the present specification below, the method of the present invention will be explained using, as an example, the measurement of coagulation reaction based on the amount of scattered light.

The start point of the coagulation reaction measurement may typically be a time point at which the specimen is mixed with a reagent to initiate the coagulation reaction; however, another time point may be defined as the start point of the coagulation reaction measurement. The time for which the coagulation reaction measurement is continued can be, for example, from several tens of seconds to about 10 minutes from the time of mixing the specimen and the reagent. This measurement time may be a fixed time determined freely, or may be up to a time point at which the end of the coagulation reaction of each specimen is detected. During the measurement time, the progress of the coagulation reaction can be repeatedly measured (photometrically detected in the case of optical detection) at predetermined intervals. For example, measurements may be taken at intervals of 0.1 seconds. The temperature of the mixture during the measurement is under general conditions in which the coagulation reaction occurs, for example, 30°C or higher and 40°C or lower, and preferably 35°C or higher and 39°C or lower. In addition, various measurement conditions can be set appropriately depending on the specimen, reagent, measurement means, and the like.

Unlike conventional methods, such as the cross-mixing test or the method described in Patent Literature 1, the method of the present invention does not require the preparation of additional specimens which have been subjected to long-term incubation treatment. For example, in the present invention, during coagulation reaction measurement, long-term incubation treatment, which is required for the delayed reaction in the conventional cross-mixing test, is not necessary before the addition of a coagulation time measurement reagent (e.g., a first reagent for APTT measurement). In the present specification, the "long-term incubation treatment" of specimens refers to incubation treatment preferably at 15°C or higher and 40°C or lower, and more preferably 30°C or higher and 40°C or lower, preferably for 2 minutes or more, and more preferably 5 minutes or more. Further, in the present specification, the "incubation treatment" of specimens refers to active temperature control using a heater, a cooler, or an incubator, and does not include passive temperature changes or maintenance, such as leaving specimens at room temperature.

On the other hand, in the present invention, the specimen to be subjected to coagulation reaction measurement may undergo preliminary heating treatment for general coagulation reaction measurement, for example, heating treatment at 30°C or higher and 40°C or lower for 1 minute or less, before the addition of a coagulation time measurement reagent. Therefore, in the present invention, the specimen to be subjected to coagulation reaction measurement does not undergo incubation treatment at 15°C or higher and 40°C or lower for 5 minutes or more before the addition of a coagulation time measurement reagent, preferably does not undergo incubation treatment at 15°C or higher and 40°C or lower for 2 minutes or more, and more preferably undergoes incubation treatment at 15°C or higher and 40°C or lower for 1 minute or less. In the present invention, after being mixed with a coagulation time measurement reagent (prepared into a reaction liquid), the specimen to be subjected to coagulation reaction measurement may undergo a heating process as in general coagulation reaction measurement, i.e., heating treatment to maintain the reaction liquid at 30°C or higher and 40°C or lower.

A series of operations in the coagulation reaction measurement described above can be performed using an automated analyzer. An example of automated analyzers is a blood coagulation automated analyzer CP3000 (manufactured by SEKISUI MEDICAL CO., LTD.). Alternatively, some of the operations may be performed manually. For example, specimen preparation can be performed manually, and the subsequent operations can be performed using an automated analyzer.

### 3. Acquisition of coagulation reaction curve

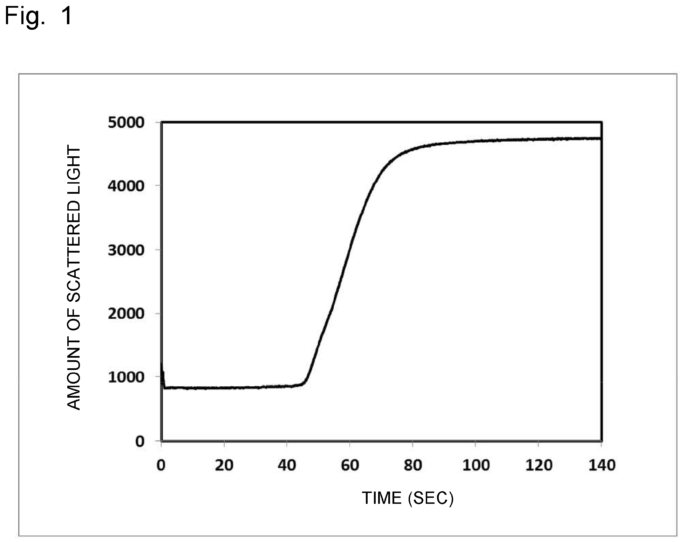

A coagulation reaction curve R(i) of the specimen is acquired from the coagulation reaction measurement described above. Here, i represents time or the number of measurement points from the start of the coagulation reaction measurement of the specimen (hereinafter also referred to simply as the "time" or "number of measurement points," respectively). For example, when the measurement (photometric) interval is 0.1 seconds, the time is represented by 0.1 × the number of measurement points. That is, R(i) may be a function of the number of measurement points or a function of the time. In the present specification below, R(i) may be abbreviated simply as R. In general, a coagulation reaction curve R is obtained by subjecting coagulation reaction measurement values to noise removal or smoothing processing by known means, or optionally zero-point adjustment or normalization of the curve to adjust the initial coagulation reaction measurement value. Fig. 1 shows an example of the coagulation reaction curve R. In Fig. 1, the horizontal axis represents the time, and the vertical axis represents the amount of scattered light. Since the coagulation reaction of the reaction liquid progresses over time, the coagulation reaction curve R rises due to the increase in the amount of scattered light, reaches a plateau as the coagulation reaction approaches completion, and takes on a sigmoidal shape over the entire process.

From the obtained coagulation reaction curve R, its derivative, e.g., a first derivative curve, may be obtained. The differential processing of the coagulation reaction curve can be performed by any method, for example, by calculating the average slope value within the interval. In the present specification, the first derivative curve of the coagulation reaction curve R is also referred to as the "coagulation rate curve" and is represented by V(i) (also abbreviated simply as V).

### 4. Calculation of coagulation time

The blood coagulation time of the specimen can be calculated based on R(i) or V(i). The coagulation time can be calculated in accordance with any method. Examples of the method for calculating the coagulation time include a method of calculating a time point at which R(i) reaches N% of a coagulation reaction end point E (described later) as the coagulation time; a method of calculating a time point at which V(i) reaches N% of its maximum value as the coagulation time; a method of using the time when the ratio of the integrated value of R(i) in a minute time zone reaches a predetermined value as a calculation start point Te, and calculating a time point at which R(i) reaches N% of R(Te) as the coagulation time (JP-A-H6-249855); a method of calculating the coagulation time based on changes over time in the integrated value of R(i) in a minute time zone (see WO 2021/132552); a method of calculating the coagulation time based on the weighted average time of V(i) (see WO 2021/177452); and a method of using the time when V(i) reaches a predetermined value after reaching a maximum value as a calculation start point Te, and calculating a time point at which R(i) reaches N% of R(Te) as the coagulation time (see WO 2021/206107).

When the coagulation time calculated for a specimen is longer than a normal value (e.g., a standard value determined based on the coagulation time of a normal specimen group), it is determined that the coagulation time of the specimen is prolonged. The specimen with a prolonged coagulation time can be used as a subject specimen (specimen S) in the method of the present invention. In the method of the present invention, when it is necessary to estimate the cause of coagulation disorders (or the cause of prolongation) in a subject specimen with a prolonged coagulation time, this subject specimen is used as the specimen S. A mixed specimen (specimen M) is prepared from the specimen S and a normal specimen (specimen N), and coagulation reaction curves of the specimens S and M can be acquired.

### 5. Acquisition of T(X)

### 5-1) Coagulation reaction end point E

In an embodiment of the method of the present invention, T(X), which represents a measurement point or time at which the coagulation reaction curve R(i) of a specimen reaches X% of a coagulation reaction end point E, is acquired. The coagulation reaction end point E in the coagulation reaction curve R of the specimen is detected. The coagulation reaction end point E can be detected in accordance with any standard, such as a time point at which R(i) reaches a plateau, a time point at which V(i) reaches a peak and then decreases to 0 or a fixed value (see WO 2021/206107), or the earliest point at which the ratio of the integrated value of R(i) is less than a threshold value (e.g., 1.001) in a minute time zone (see WO 2021/132552 or the Examples described later). The detection of the coagulation reaction end point E may be performed after the acquisition of R up to a predetermined measurement time, or the detection of E may be performed in parallel with the acquisition of R, and the acquisition of R may be terminated at the time when E is detected. For example, the procedure described in WO 2021/206107 or WO 2021/132552 makes it possible to detect the coagulation reaction end point E in parallel with the acquisition of R (so-called real-time detection of E).

As an embodiment of the method of the present invention, an example of the procedure for detecting the coagulation reaction end point E based on the method described in WO 2021/132552 is described in detail. The ratio of the integrated value of R(i) in a minute time zone is defined as an integration ratio Z(i), and is calculated by the following equation: 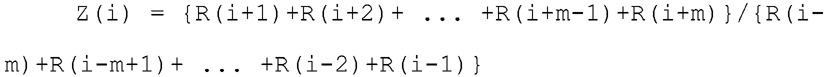 Z(i) = {R(i+1)+R(i+2)+ ... +R(i+m-1)+R(i+m)}/{R(i-m)+R(i-m+1)+ ... +R(i-2)+R(i-1)}

In the above equation, i represents a measurement point number, and m can be set appropriately depending on the coagulation reaction measurement conditions, analysis items, and the like. For example, when the measurement interval is 0.1 seconds, m = 10 to 30. R(i) at the earliest measurement point or time point at which Z(i) is less than a threshold value Zs is detected as the coagulation reaction end point E. Zs can be set appropriately depending on the analysis items, but is larger than 1 and 1.100 or less. For example, in the case of APTT measurement, Zs is preferably 1.050 or less, and more preferably within the range from 1.010 to 1.001. In order to prevent the false detection of E due to early reaction errors or other factors, it is preferable to calculate Z(i) after i reaches a predetermined calculation start point and after R(i) is equal to or larger than a predetermined value. In this procedure, while measuring the coagulation reaction, R(i) is acquired and Z(i) is calculated in parallel, enabling the detection of E.

In the method of the present invention, the coagulation reaction end point E in the coagulation reaction curve can be acquired for each of the specimen S and specimen M. Hereinafter, the coagulation reaction end point in the coagulation reaction curve of the specimen S is referred to as Es, and the coagulation reaction end point in the coagulation reaction curve of the specimen M is referred to as Em.

### 5-2) Ts(X) and Tm(X)

T(X) can be acquired for each of the specimen S and specimen M. In the present specification, R(i) and T(X) for the specimen S are referred to as Rs(i) (or simply as Rs) and Ts(X), respectively, and R(i) and T(X) for the specimen M are referred to as Rm(i) (or simply as Rm) and Tm(X), respectively. More specifically, Ts(X) represents a measurement point or time at which the coagulation reaction curve Rs of the specimen S reaches X% of Es, and Tm(X) represents a measurement point or time at which the coagulation reaction curve Rm of the specimen M reaches X% of Em. On the other hand, T(X) in the present specification is used as a generic term which includes Ts(X) and Tm(X), and the explanation about T(X) also applies to Ts(X) and Tm(X), unless otherwise specified. In the present specification and drawings, T(X), Ts(X), and Tm(X) may be written without parentheses around X as "TX," "TsX," and "TmX," respectively, or without X and parentheses as "T," "Ts," and "Tm," respectively.

More specifically, T(X) is the number of measurement points or time until R reaches X% of E from the coagulation reaction measurement start point (time 0). That is, the following equation (I) holds for T(X). $R \left(T \left(X\right)\right) = E \times X %$

Therefore, the following equations (Ia) and (Ib) hold for Ts(X) and Tm(X), respectively. $Rs \left(Ts \left(X\right)\right) = Es \times X %$ $Rm \left(Tm \left(X\right)\right) = Em \times X %$

Here, Rs and Rm represent the coagulation reaction curves of the specimens S and M, respectively, and 0<X≤100. Therefore, T(X) can be expressed as a function of the variable X. In the method of the present invention, X is preferably from 1 to 100. For example, each X may be a value separated by 0.1 or more (i.e., increment of X≥0.1), or a value separated by 5 or more (i.e., increment of X≥5). In an example, X is a variable which changes in increments of 1 in the range from 1 to 100 (i.e., X = {1, 2, 3, ..., 97, 98, 99, 100}). In another example, X is a variable which changes in increments of 3 in the range from 3 to 99 (i.e., X = {3, 6, 9, ..., 96, 99}). In still another example, X is a variable which changes in increments of 3 in the range from 1 to 97 (i.e., X = {1, 4, 7, ..., 94, 97}). In still another example, X is a variable which changes in increments of 5 in the range from 5 to 100 (i.e., X = {5, 10, 15, ..., 90, 95, 100}). In another example, X may be a variable which changes in increments of 0.5 in the range from 0.5 to 100 (i.e., X = {0.5, 1.0, 1.5, 2.0, ..., 98.5, 99.0, 99.5, 100.0}).

Alternatively, T(X) may be expressed as an average value from T(X-K) to T(X+K). In this case, K is preferably less than the increment of X described above. For example, when K = 2, T(X) can be expressed as an average value from T(8) to T(12) when X = 10, or can be expressed as an average value from T(13) to T(17) when X = 15. Alternatively, T(X) may be expressed as an average value from T(X-K+1) to T(X). For example, when K = 5, T(X) can be expressed as an average value from T(6) to T(10) when X = 10, or can be expressed as an average value from T(91) to T(95) when X = 95.

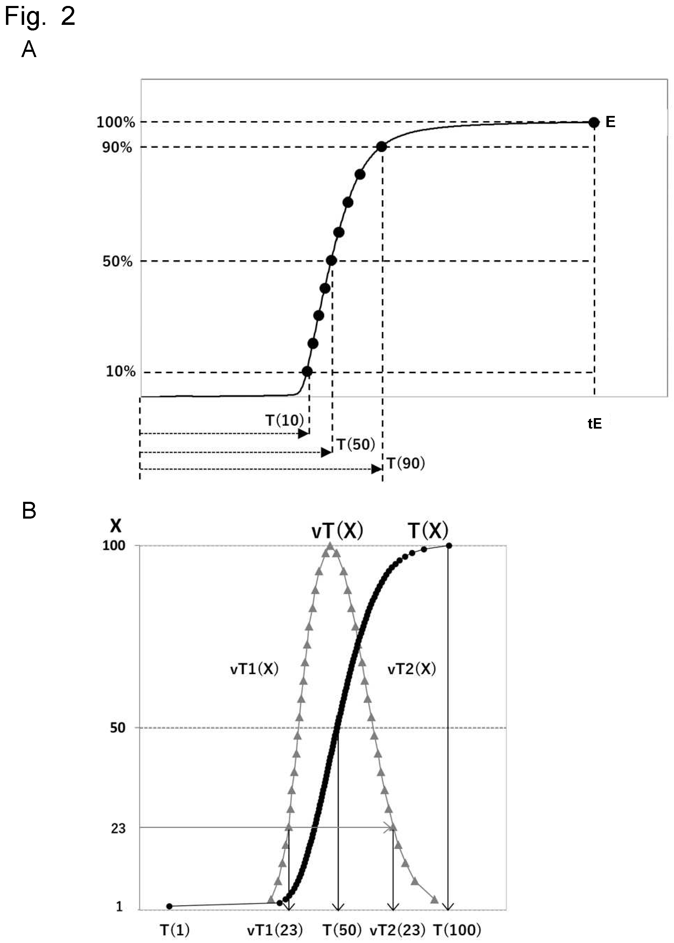

Fig. 2A illustrates a diagram explaining T(X). The figure shows a coagulation reaction curve with points at which T(X) reaches from 10 to 90% of E, and the time points at which T(X) reaches 10%, 50%, and 90% of E are shown as T(10), T(50), and T(90), respectively. tE at the time point E corresponds to T(100).

In order to calculate parameters for estimating the cause of coagulation disorders described later, Ts(X) and Tm(X) may be further converted to relative values. When the relative values of Ts(X) and Tm(X) are expressed as rTs(X) and rTm(X), respectively, rTs(X) and rTm(X) are preferably defined by the following equations (IIa) and (IIb), respectively. $rTs \left(X\right) = Ts \left(X\right) / Ts \left(As\right) \times Qs$ $rTm \left(X\right) = Tm \left(X\right) / Tm \left(Am\right) \times Qm$

Here, As and Am may each independently be in the range of X values described above, and is preferably from 3 to 97, and more preferably from 40 to 60 (e.g., 50). Qs>0 and Qm>0. Qs and Qm are coefficients for adjusting the size of the parameters, and can be selected from, for example, a range from 1 to 100. It is not necessary for Qs and Qm to be the same value.

The method of the present invention may include a step of calculating Ts(X) or rTs(X), and Tm(X) or rTm(X), from the coagulation reactions of the specimen S and specimen M. Alternatively, Ts(X), Tm(X), rTs(X), or rTm(X) used in the method of the present invention may be calculated or stored in advance.

### 6. Acquisition of function related to V(i)

### 6-1) vT1(X) and vT2(X)

In an embodiment of the method of the present invention, a point at which the coagulation rate curve V(i) of a specimen reaches a predetermined value is acquired. The predetermined value can be set to X% (0<X≤100) of the maximum value Vmax of V(i). Since V(i) is a carve with a peak at Vmax, points at which V(i) reaches the predetermined value are detected before and after the peak. In the present specification, the measurement point or time at which V(i) reaches Vmax is referred to as VmaxT. The measurement point or time at which V(i) reaches X% of Vmax before VmaxT is defined as vT1(X), and the measurement point or time at which V(i) reaches X% of Vmax after VmaxT is defined as vT2(X). Therefore, vT1(X)≤vT2(X), provided that when X = 100, vT1(X) = vT2(X) = VmaxT. In the present specification, vT1(X) and vT2(X) may be collectively expressed as vT(X). Fig. 2B illustrates the changes in T(X) and vT(X) with respect to X. In the figure, vT(X) is shown divided into vT1(X) and vT2(X).

In the method of the present invention, vT1(X) and vT2(X) can be acquired for each of the specimen S and specimen M. In the present specification, V(i), Vmax, VmaxT, vT1(X), and vT2(X) for the specimen S are referred to as Vs(i), Vmax_s, VmaxT_s, vT1s(X), and vT2s(X), respectively, and V(i), Vmax, VmaxT, vT1(X), and vT2(X) for the specimen M are referred to as Vm(i), Vmax_m, VmaxT_m, vT1m(X), and vT2m(X), respectively. vT1s(X) and vT2s(X) may be collectively expressed as vTs(X), and vT1m(X) and vT2m(X) may be collectively expressed as vTm(X). Further, in the present specification and drawings, vT1s(X), vT2s(X), vT1m(X), and vT2m(X) may be written without parentheses around X as "vT1sX," "vT2sX," "vT1mX," and "vT2mX," respectively, or without X and parentheses as "vT1s," "vT2s," "vT1m," and "vT2m," respectively.

Therefore, the following equations (Xa) to (Xd) hold for vT1s(X), vT2s(X), vT1m(X), and vT2m(X), respectively. $Vs \left(vT1s \left(X\right)\right) = Vmax _ s \times X %$ (provided that vT1s(X)≤VmaxT_s) $Vs \left(vT2s \left(X\right)\right) = Vmax _ s \times X %$ (provided that vT2s(X)≥VmaxT_s) $Vm \left(vT1m \left(X\right)\right) = Vmax _ m \times X %$ (provided that vT1m(X)≤VmaxT_m) $Vm \left(vT2m \left(X\right)\right) = Vmax _ m \times X %$ (provided that vT2m(X)≥VmaxT_m)

That is, vT1s(X) and vT2s(X) each represent a measurement point or time at which Vs(i) reaches X% of Vmax_s before and after VmaxT_s, and vT1m(X) and vT2m(X) each represent a measurement point or time at which Vm(i) reaches X% of Vmax_m before and after VmaxT_m. In the equations (Xa) to (Xd), 0<X≤100.

In the calculation of vT1(X) and vT2(X), when V(i) shows a bimodal distribution, multiple points at which V(i) is Vmax×X% after VmaxT may be detected. In that case, the largest among the detected points can be selected as vT2(X). Similarly, when multiple points at which (i) is Vmax×X% before VmaxT are detected, the smallest among the detected points (excluding points in the initial noise) can be selected as vT1(X). Alternatively, V(i)<(Vmax×X%)<V(i+1) or V(i-1)<(Vmax×X%)<V(i) in some times. In those cases, i can be selected as the number of measurement points or time which satisfies V(i) = Vmax×X%.

### 6-2) C(i)

V(i) may be corrected based on the coagulation reaction end point E. In the present specification, the corrected V(i) is referred to as the corrected V(i) and expressed as C(i). For example, the corrected Vs(i) and corrected Vm(i) are expressed as Cs(i) and Cm(i), respectively, and can be calculated in accordance with the following equations. $Cs \left(i\right) = Vs \left(i\right) \times \left(Q/Es\right)$ $Cm \left(i\right) = Vm \left(i\right) \times \left(Q/Em\right)$ (in each equation, Q>0)

In the calculation of vT1s(X), vT2s(X), vT1m(X), and vT2m(X) described above, C(i) (Cs(i) and Cm(i)) can be used in place of Vs(i) and Vm(i).

In the present specification, the maximum values of Cs(i) and Cm(i) are expressed as Cmax_s and Cmax_m, respectively. Further, the reciprocals of Cs(i) and Cm(i) are expressed as iCs(i) and iCm(i), respectively; that is, iCs(X) = 1/Cs(X) and iCm(X) = 1/Cm(X). Further, the reciprocals of Cmax_s and Cmax_m are expressed as iCmax_s and iCmax_m, respectively; that is, iCmax_s = 1/Cmax_s and iCmax_m = 1/Cmax_m.

### 7. Calculation of parameters Ps and Pm

In the method of the present invention, a parameter Ps related to the coagulation reaction of the specimen S, and a parameter Pm related to the coagulation reaction of the specimen M are calculated.

In an embodiment, Ps is a parameter calculated based on Ts(X), and preferably a parameter calculated based on rTs(X). In another embodiment, Ps is a parameter calculated based on vTs(X) (vT1s(X) and/or vT2s(X)). Preferably, at least two Ts(X) or vTs(X) in at least two X (e.g., X1 and As, or X1 and X2) are used for the calculation of Ps.

In an embodiment, Pm is a parameter calculated based on Tm(X), and preferably a parameter calculated based on rTm(X). In another embodiment, Pm is a parameter calculated based on vTm(X) (vT1m(X) and/or vT2m(X)). In another embodiment, Pm is a parameter calculated based on vT2m(X). Preferably, at least two Tm(X) or vTm(X) in at least two X (e.g., X1 and Am, or X1 and X2) are used for the calculation of Pm.

### 7-1) Ps

In an embodiment, Ps is rTs(X1) defined by the following equation (IIa'). $rTs \left(X1\right) = Ts \left(X1\right) / Ts \left(As\right) \times Qs$

Here, preferably X1 = 35 to 95, As = 3 to 75, provided that X1>As, preferably (X1-As) = 5 to 92, and Qs is as described above.

In an embodiment, Ps is rTs(X2)-rTs(X1) defined by the following equation (IIIa'). $rTs \left(X2\right) - rTs \left(X1\right)$ $\left(=\left\{Ts \left(X2\right)-Ts \left(X1\right)\right\}/Ts \left(As\right)\times Qs\right)$

Here, X1 and X2 may each independently be in the range of X values described above, provided that X1<X2, preferably X1 = 2 to 80, X2 = 50 to 95, and (X2-X1) = 5 to 93. As and Qs are as described above.

In an embodiment, Ps is arTs(X1,X2), which is defined as the ratio of the difference between rTs(X1) and rTs(X2) to the difference between X1 and X2 by the following equation (IVa'). $\begin{matrix}arTs \left(X1,X2\right) \\ = \left\{rTs \left(X2\right)-rTs \left(X1\right)\right\} / \left(X2-X1\right) \\ = \left[\left\{Ts \left(X2\right)-Ts \left(X1\right)\right\}/Ts \left(As\right)\times Qs\right] / \left(X2-X1\right)\end{matrix}$

Here, X1 and X2 may each independently be in the range of X values described above, provided that X1<X2, preferably X1 = 1 to 85, X2 = 25 to 95, (X2-X1) = 5 to 94, and As = 5 to 95. More preferably, X1 = 4 to 80, X2 = 55 to 95, and X2-X1 = 5 to 91. Qs is as described above.

In an embodiment, Ps is the slope of a regression line of rTs(X) in a predetermined interval of X. The slope of the regression line of rTs(X) in the interval from X1 to X2 is represented by rSLs[X1:X2], and is calculated by the following equation (Va'). $rSLs \left[X1:X2\right] = \frac{{\sum }_{X = X 1}^{X 2} \left\{\left(X-UX\right)\times \left(rTs\left(X\right)-UrTs\right)\right\}}{{\sum }_{X = X 1}^{X 2} {\left(X-UX\right)}^{2}} \times Qs$

Here, UX is an average of X from X1 to X2, and UrTs is an average of rTs(X) from rTs(X1) to rTs(X2). For the calculation of rTs, As = 5 to 95. X1 and X2 may each independently be in the range of X values described above, provided that X1<X2, preferably X1 = 1 to 85, X2 = 25 to 100, and (X2-X1) = 5 to 99, and more preferably X1 = 1 to 80, X2 = 55 to 100, and (X2-X1) = 5 to 99. Qs is as described above.

In an embodiment, Ps is a weighted average of X in a predetermined interval of Ts(X). The weighted average of X in the interval from Ts(X1) to Ts(X2) is represented by wXs[X1:X2], and is calculated by the following equation (VIa). $wXs \left[X1:X2\right] = \frac{{\sum }_{X = X 1}^{X 2} \left\{X\times Ts\left(X\right)\right\}}{{\sum }_{X = X 1}^{X 2} Ts \left(X\right)} \times Qs$

Here, X1 and X2 may each independently be in the range of X values described above, provided that X1<X2, preferably X1 = 1 to 75, X2 = 30 to 100, and (X2-X1) = 10 to 99. Qs is as described above.

In an embodiment, Ps is the ratio of vT(X), and is vT2s(X1)/vT1s(X2). Here, preferably X1 = 3 to 58 and X2 = 13 to 98. In another embodiment, Ps is vT2s(X1)/vT2s(X2). Here, preferably X1 = 13 to 63 and X2 = 58 to 83 (provided that X2>X1).

In an embodiment, Ps is the ratio to the difference in vT(X), and is (vT2s(X1) -vT1s(X2))/vT2s(Bs). Here, preferably X1 = 3 to 58, X2 = 13 to 98, and Bs = 3 to 100. More preferably X1 = 13 to 63 and X2 = 58 to 83.

In another embodiment, Ps is (vT2s(X1)-vT2s(X2))/vT2s(Bs). Here, preferably X1 = 8 to 58, X2 = 48 to 83, provided that X2>X1, and Bs = 3 to 100. More preferably X1 = 8 to 58 and X2 = 68 to 83.

In another embodiment, Ps is (vT2s(X1)-vT2s(X2))/vT1s(Bs). Here, preferably X1 = 8 to 58, X2 = 48 to 98, provided that X2>X1, and Bs = 3 to 100. More preferably X2 = 38 to 83.

In another embodiment, Ps is (vT2s(X1)-vT1s(X2))/vT1s(Bs). Here, preferably X1 = 3 to 58, X2 = 3 to 93, and Bs = 3 to 100. More preferably, X2 = 78 to 83.

### 7-2) Pm

In an embodiment, Pm is Tm(X1), wherein X1 may be in the range of X values described above, for example, X1 = 90 to 100.

In an embodiment, Pm is the ratio of Tm(X), and is rTm(X1) defined by the following equation (IIb'). $rTm \left(X1\right) = Tm \left(X1\right) / Tm \left(Am\right) \times Qm$

Here, preferably X1 = 30 to 95, Am = 1 to 35, provided that X1>Am, preferably (X1-Am) = 10 to 94, and Qm is as described above.

In an embodiment, Pm is aTm(X1,X2), which is defined as the ratio of the difference between Tm(X1) and Tm(X2) to the difference between X1 and X2 by the following equation (IVb). $\begin{matrix}aTm \left(X1,X2\right) \\ = \left\{Tm \left(X2\right)-Tm \left(X1\right)\right\} / \left(X2-X1\right) \times Qm\end{matrix}$

Here, X1 and X2 may each independently be in the range of X values described above, provided that X1<X2, preferably X1 = 1 to 90, X2 = 25 to 100, and X2-X1 = 5 to 99. Qm is as described above.

In an embodiment, Pm is the slope of a regression line of Tm(X) in a predetermined interval of X. The slope of the regression line in the interval from X1 to X2 is represented by SLm[X1:X2], and is calculated by the following equation (Vb). $SLm \left[X1:X2\right] = \frac{{\sum }_{X = X 1}^{X 2} \left\{\left(X-UX\right)\times \left(Tm\left(X\right)-UTm\right)\right\}}{{\sum }_{X = X 1}^{X 2} {\left(X-UX\right)}^{2}} \times Qm$

Here, UX is an average of X from X1 to X2, and UTm is an average of Tm(X) from Tm(X1) to Tm(X2). X1 and X2 may each independently be in the range of X values described above, provided that X1<X2, preferably X1 = 1 to 95, X2 = 30 to 100, and (X2-X1) = 5 to 99. Qm is as described above.

In an embodiment, Pm is the slope of a regression line of rTm(X) in a predetermined interval of X. The slope of the regression line of rTm(X) in the section from X1 to X2 is represented by rSLm[X1:X2], and is calculated by the following equation (Vb'). $rSLm \left[X1:X2\right] = \frac{{\sum }_{X = X 1}^{X 2} \left\{\left(X-UX\right)\times \left(rTm\left(X\right)-UrTm\right)\right\}}{{\sum }_{X = X 1}^{X 2} {\left(X-UX\right)}^{2}} \times Qm$

Here, UX is an average of X from X1 to X2, and UrTm is an average of rTm(X) from rTm(X1) to rTm(X2). X1 and X2 may each independently be in the range of X values described above, provided that X1<X2, preferably X1 = 1 to 70, X2 = 25 to 100, and (X2-X1) = 5 to 99, and more preferably X1 = 1 to 55, X2 = 25 to 95, and (X2-X1) = 5 to 94. rTm is as described above, Am for the calculation of rTm is as described above, and preferably Am = 5 to 95. Qm is as described above.

In an embodiment, Pm is a weighted average of X in a predetermined interval of Tm(X). The weighted average of X in the interval from Tm(X1) to Tm(X2) is represented by wXm[X1:X2], and is calculated by the following equation (VIb). $wXm \left[X1:X2\right] = \frac{{\sum }_{X = X 1}^{X 2} \left\{X\times Tm\left(X\right)\right\}}{{\sum }_{X = X 1}^{X 2} Tm \left(X\right)} \times Qm$

Here, X1 and X2 may each independently be in the range of X values described above, provided that X1<X2, preferably X1 = 1 to 45, X2 = 30 to 100, and (X2-X1) = 10 to 99. Qm is as described above.

In an embodiment, Pm is a weighted average of Tm(X) in a predetermined interval of X. The weighted average of Tm(X) in the interval from X1 to X2 is represented by wTm[X1:X2], and is calculated by the following equation (VIIb). $wTm \left[X1:X2\right] = \frac{{\sum }_{X = X 1}^{X 2} \left\{X\times Tm\left(X\right)\right\}}{{\sum }_{X = X 1}^{X 2} X} \times Qm$

Here, X1 and X2 may each independently be in the range of X values described above, provided that X1<X2, preferably X1 = 75 to 95, X2 = 95 to 100, and (X2-X1) = 5 to 25. Qm is as described above.

In an embodiment, Pm is a weighted average of rTm(X) in a predetermined interval of X. The weighted average of rTm(X) in the interval from X1 to X2 is represented by wrTm[X1:X2], and is calculated by the following equation (VIIb'). $\begin{matrix}wrTm \left[X1:X2\right] = \frac{{\sum }_{X = X 1}^{X 2} \left\{X\times rTm\left(X\right)\right\}}{{\sum }_{X = X 1}^{X 2} X} \\ = \frac{{\sum }_{X = X 1}^{X 2} \left\{X\times Tm\left(X\right)/Tm\left(Am\right)\times Qm\right\}}{{\sum }_{X = X 1}^{X 2} X}\end{matrix}$

Here, X1 and X2 may each independently be in the range of X values described above, provided that X1<X2, preferably X1 = 1 to 90, X2 = 30 to 100, and (X2-X1) = 5 to 99, and more preferably X1 = 15 to 75, X2 = 50 to 85, and (X2-X1) = 5 to 65. rTm is as described above, preferably Am = 3 to 34, and Qm is as described above.

In an embodiment, Pm is vT2m(X1). Here, preferably X1 = 3 to 18. In an embodiment, Pm is vT2m(X1)/vT1m(X2). Here, preferably X1 = 3 to 93 and X2 = 3 to 88. In an embodiment, Pm is vT2m(X1)/vT2m(X2). Here, preferably X1 = 38 to 63, X2 = 63 to 93, and X2>X1.

In an embodiment, Pm is the ratio to the difference in vT(X), and is (vT2m(X1)-vT1m(X2)) /vT2m(Bm). Here, preferably X1 = 3 to 93, X2 = 3 to 88, and Bm = 3 to 100. More preferably X1 = 18 to 93 and X2 = 3 to 78.

In another embodiment, Pm is (vT2m(X1)-vT2m(X2))/vT2m(Bm). Here, preferably X1 = 28 to 63, X2 = 68 to 93, provided that X2>X1, and Bm = 3 to 100. More preferably X1 = 43 to 58 and X2 = 63 to 68.

In another embodiment, Pm is (vT2m(X1)-vT1m(X2))/vT1m(Bm). Here, preferably X1 = 3 to 93, X2 = 3 to 93, and Bm = 3 to 100. More preferably X1 = 18 to 93 and X2 = 3 to 78.

In another embodiment, Pm is (vT2m(X1)-vT2m(X2))/vT1m(Bm). Here, preferably X1 = 23 to 63, X2 = 63 to 93, provided that X2>X1, and Bm = 3 to 100. More preferably X1 = 43 to 58 and X2 = 63 to 68.

In an embodiment, Pm is a reciprocal iCm(X1) of Cm(X1), wherein preferably X1 = 100. Therefore, a preferred example of iCm(X1) used for Pm is iCmax_m. In another embodiment, Pm is a reciprocal of the weighted average of Cm(X) in the interval of X = X1 to X2, and this is represented by iwCm[X1:X2], and is calculated by the following equation (XIIb). $iwCm \left[X1:X2\right] = \frac{{\sum }_{i = X 1}^{X 2} i}{{\sum }_{i = X 1}^{X 2} \left\{i\times Cm\left(i\right)\right\}} \times Qm$

Here, X1 and X2 satisfy Cm(X1) = Cm(X2) = Vm(i)×[Q/(Em×S%)] (S = 3 to 100), and X1<VmaxT_m<X2. Qm is as described above. iwCm[X1:X2] may be represented by iwCm[S].

### 8. Estimation of cause of coagulation disorders using Ps and Pm

In the method of the present invention, Ps and Pm described above, which are parameters for the specimen S and specimen M, are used to estimate the cause of coagulation disorders (or the cause of prolongation) in the specimen S.

Examples of the cause of coagulation disorders which can be estimated by the method of the present invention include coagulation factor inhibitor positivity (Inh), lupus anticoagulant positivity (LA), and coagulation factor deficiency (Def). Coagulation factor deficiency (Def) includes coagulation factor V (FV) deficiency, coagulation factor VIII (FVIII) deficiency, coagulation factor IX (FIX) deficiency, coagulation factor X (FX) deficiency, coagulation factor XI (FXI) deficiency, coagulation factor XII (FXII) deficiency, and the like. Preferably, coagulation factor deficiency refers to a condition in which the coagulation factor (e.g., FVIII) activity level (IU/dL) is less than about 40% of the normal level. Examples of coagulation factor inhibitors include FVIII inhibitors, FIX inhibitors, and the like. Preferably, inhibitor positivity refers to a condition in which the inhibitor titer is 0.6 BU/mL or more. In the present specification, the inhibitor titer is also referred to simply as "titer," and the inhibitor titer in the present specification is expressed as Bethesda unit (BU/mL) .

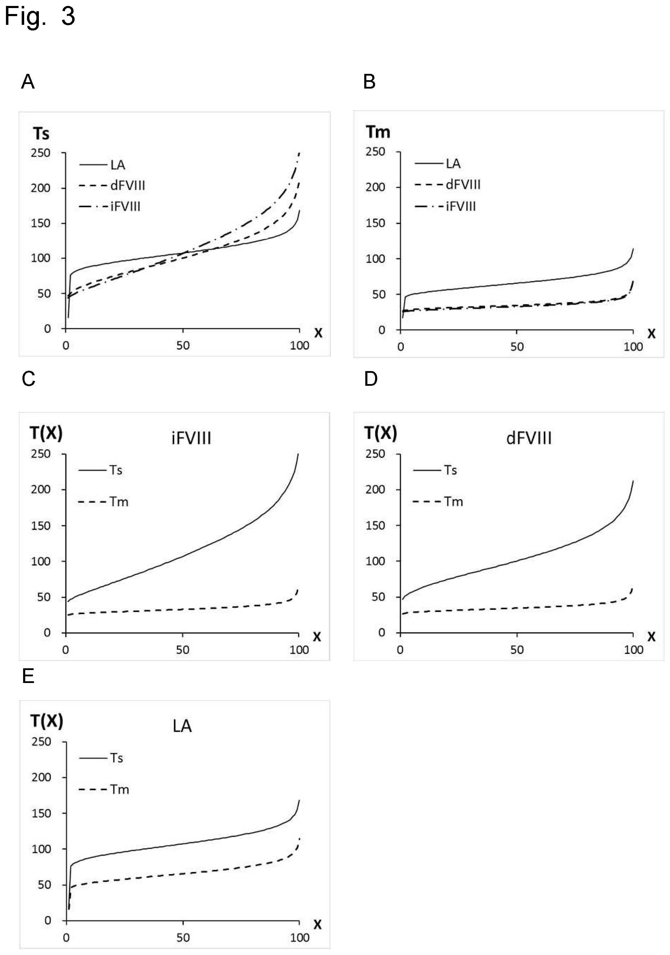

As shown in the Examples described later, Ts(X) may show different characteristics depending on the cause of coagulation disorders in the specimen (specimen type). Fig. 3A shows Ts(X) for each specimen type in which APTT is about 100 seconds. The solid line indicates LA, the dashed line indicates dFVIII (FVIII activity: less than 1%) as an example of Def, and the chain line indicates iFVIII (FVIII inhibitor-positive, titer: 0.8) as an example of Inh. APTT is Ts(X) when X = 50, and it is shown that all three specimen types reach this point at around 100 seconds. When comparing the three specimen types relatively, LA is high when X<50, while when X>50, the level increases in the order of LA, dFVIII, and iFVIII. The slope in the range of X from about 10 to about 90 also increases in the order of LA, dFVIII, and iFVIII. These characteristics indicate that even for specimens with about the same prolonged APTT (about 100 seconds), there are large differences in Ts(X) depending on the specimen type, that the slope of Ts(X) for iFVIII is the largest, and that the difference in Ts(X) increases as X increases when X>50, while when X = 100, Ts(X) for iFVIII is the largest. That is, Ts(X) for Inh indicates that the coagulation reaction of Inh is weaker than that of LA and Def. Therefore, based on Ts(X) of the specimen S with coagulation disorders (prolonged coagulation time), it is possible to estimate whether the coagulation disorders in the specimen S are due to Inh or other causes (LA or Def). For example, when the horizontal axis is X and the vertical axis is Ts(X) as shown in Fig. 3A, the slope of Ts(X) for Inh is larger than that for LA and Def. Therefore, Inh can be distinguished by using, as a parameter, an index Ps based on Ts(X) in a predetermined interval of X, which quantifies such characteristics of Ts(X).

Furthermore, Tm(X) can also show different characteristics depending on the cause of coagulation disorders in the specimen. Fig. 3B shows Tm(X) for each specimen type for mixed specimens of test and normal specimens (NP) at a mixing ratio of 1:1, and the solid line, dashed line, and chain line are the same as those in Fig. 3A. Tm(X) of the mixed specimens derived from dFVIII (Def) and iFVIII (Inh) showed a small increase with the increase in X because the coagulation reaction approached that of the normal specimen due to the correction effect of the coagulation factor derived from the normal specimen. On the other hand, in the mixed specimen derived from LA, NP-derived phospholipids mixed with the specimen S to prepare the mixed specimen immediately bound to autoantibodies against phospholipids contained in the specimen S, thereby inhibiting the coagulation reaction. As a result, Tm(X) increased significantly with the increase in X. That is, Tm(X) for LA includes data showing that LA inhibits the coagulation reaction more strongly than Def and Inh. However, it should be noted that even for high-titer Inh, the coagulation reaction is inhibited and Tm(X) increases. However, as described above, if Inh can be already (previously) distinguished based on Ts, then it is sufficient to distinguish between Def and LA; thus, Tm(X) can be data for distinguishing between LA and Def. For example, when the horizontal axis is X and the vertical axis is Tm(X) as shown in Fig. 3B, the slope of Tm(X) for LA is larger than that for Def. Therefore, LA can be distinguished by using, as a parameter, an index Pm based on Tm(X) in a predetermined interval of X, which quantifies such characteristics of Tm(X).

As described above, in the present invention, whether a specimen S is first estimated to be Inh based on Ts(X) of the specimen S. Next, the specimen S which has not been estimated to be Inh can be estimated to be LA or Def based on Tm(X).

More specifically, in the present invention, Ps and Pm described above are used to estimate the cause of coagulation disorders in a specimen S. More specifically, whether a specimen S is first estimated to be Inh based on Ps, and the specimen S which has not been estimated to be Inh is then estimated to be LA or Def based on Pm, or Ps and Pm.

In an embodiment, Ps is rTs(X1), rTs(X2)-rTs(X1), arTs(X1,X2), rSLs[X1:X2], wXs[X1:X2], vT2s(X1)/vT1s(X2), vT2s(X1)/vT2s(X2), (vT2s(X1)-vT1s(X2))/vT2s(Bs), (vT2s(X1)-vT1s(X2))/vT2s(Bs), (vT2s(X1)-vT1s(X2))/vT1s(Bs), (vT2s(X1)-vT2s(X2))/vT2s(Bs), or (vT2s(X1)-vT2s(X2)) /vT1s(Bs) described above. If Ps is equal to or larger than a threshold value, the specimen S is estimated to be Inh. In the present specification, the threshold value for Ps is set to Ls. The threshold value Ls can be determined prior to the implementation of the method of the present invention. Ls can be determined based on Ps calculated from a specimen group with a known cause of coagulation disorders. Ls can be set for each Ps, i.e., rTs(X1), rTs(X2)-rTs(X1), arTs(X1,X2), rSLs[X1:X2], wXs[X1:X2], vT2s(X1)/vT1s(X2), vT2s(X1)/vT2s(X2), (vT2s(X1)-vT1s(X2))/vT2s(Bs), (vT2s(X1)-vT1s(X2))/vT1s(Bs), (vT2s(X1)-vT2s(X2))/vT2s(Bs), and (vT2s(X1)-vT2s(X2))/vT1s(Bs). On the other hand, when Ps of a specimen S is not equal to or larger than Ls, the specimen S is not estimated to be Inh, and is estimated to be LA or Def in an estimation step based on Pm described later.

If necessary, two threshold values Ls1 and Ls2 related to Ps may be set. However, Ls1>Ls2. For example, when Ps of a specimen S is equal to or larger than Ls1, the specimen S is estimated to be Inh, whereas when Ps of a specimen S is equal to or less than Ls2, the specimen S is not estimated to be Inh, and is estimated to be LA or Def in an estimation step based on Pm described later.

In an example, Ps is calculated for each specimen from a specimen group with Inh, and Ls is set based on their statistics (e.g., average value - [k × standard deviation], k is preferably from 2 to 5). In another example, Ps is calculated for each specimen from a specimen group with Inh, and the minimum value × k (k is preferably 0<k<100%) is set to Ls. In another example, Ps of an Inh specimen whose titer exceeds the cut-off value (0.6 BU/mL) but is around 1 BU/mL is calculated, and this value × k (k is preferably 0<k≤100%) is set to Ls. These Ls may be set to Ls1 when threshold values Ls1 and Ls2 are set.

In another example, Ps is calculated for each specimen from a specimen group with a cause of prolongation other than Inh **(e.g.,** a specimen group with LA and/or Def), and Ls is set based on their statistics (e.g., average value + [k × standard deviation], k is preferably from 2 to 5). In another example, Ps is calculated for each specimen from a specimen group with a cause of prolongation other than Inh (e.g., a specimen group with LA and Def), and the maximum value × k (k is preferably 1<k) is set to Ls. In another example, Ps is calculated for each specimen from a group of normal specimens (NP), and Ls is set based on their statistics (e.g., average value + [k × standard deviation], k is preferably from 2 to 5). These Ls may be set to Ls2 when threshold values Ls1 and Ls2 are set. Ls2 is preferably set in accordance with the above criteria based on Ps of each specimen from a specimen group with LA and Def, or NP. In another example, Ls is set between the maximum value of Ps form a specimen group with LA and Def, and the minimum value of Ps from a specimen group with Inh.

In an embodiment, Pm is Tm(X1), rTm(X1), aTm(X1,X2), SLm[X1:X2], rSLm[X1:X2], wXm[X1:X2], wTm[X1:X2], wrTm[X1:X2], vT2m(X1)/vT1m(X2), vT2m(X1)/vT2m(X2), (vT2m(X1)-vT1m(X2))/vT2s(Bs), (vT2m(X1)-vT2m(X2))/vT2s(Bs), (vT2m(X1)-vT1m(X2))/vT1s(Bs), (vT2m(X1)-vT2m(X2))/vT1s(Bs), iCm(X1), or iwCm[X1:X2] described above.

In an embodiment, if Pm is equal to or larger than a threshold value, the specimen S is estimated to be LA, and if not, the specimen S is estimated to be Def. In the present specification, the threshold value for Pm is set to Lm. The threshold value Lm can be determined prior to the implementation of the method of the present invention. Lm can be determined based on Pm calculated from a specimen group with a known cause of coagulation disorders. Lm can be set for each Pm, i.e., Tm(X1), rTm(X1), aTm(X1,X2), SLm[X1:X2], rSLm[X1:X2], wXm[X1:X2], wTm[X1:X2], wrTm[X1:X2], vT2m(X1)/vT1m(X2), vT2m(X1)/vT2m(X2), (vT2m(X1)-vT1m(X2))/vT2s(Bs), (vT2m(X1)-vT2m(X2))/vT2s(Bs), (vT2m(X1)-vT1m(X2))/vT1s(Bs), (vT2m(X1)-vT2m(X2))/vT1s(Bs), iCm(X1), and iwCm[X1:X2].

If necessary, two threshold values Lm1 and Lm2 related to Pm may be set. However, Lm1>Lm2. For example, if Pm of a specimen S is equal to or larger than Lm1, the specimen S is estimated to be LA, whereas if Pm of a specimen S is equal to or less than Lm2, the specimen S is estimated to be Def.

In an example, Pm of each specimen is calculated from a specimen group with LA, and Lm is set based on their statistics (e.g., average value - [k × standard deviation], k is preferably from 2 to 5). In another example, Pm of each specimen is calculated from a specimen group with LA, and the minimum value × k (k is preferably 0<k<100%) is set to Lm. These Lm may be set to Lm1 when threshold values Lm1 and Lm2 are set.

In another example, Pm of each specimen is calculated from a specimen group with Def, and Lm is set based on their statistics (e.g., average value + [k × standard deviation], k is preferably from 2 to 5). In another example, Pm of each specimen is calculated from a specimen group with Def, and the maximum value × k (k is preferably 1<k) is set to Lm. In another example, Lm is set between the maximum value of Pm from a specimen group with Def and the minimum value of Pm from a specimen group with LA. In another example, Pm of each specimen is calculated from a group of normal specimens (NP), and Lm is set based on their statistics (e.g., average value + [k × standard deviation], k is preferably from 2 to 5). These Lm may be set to Lm2 when threshold values Lm1 and Lm2 are set.

In another embodiment, a threshold line Lc which separates LA and Def is set based on Pm and Ps. With Ps on the horizontal axis (X-axis) and Pm on the vertical axis (Y-axis), the threshold line (Y = aX+b) is determined using the following steps.
1) Calculate the slope SL and intercept IC of the regression line for the LA group.
2) Determine Pm for each in the LA group and Py (Py = SL×Ps +IC).
3) Determine the standard deviation SD of (Pm-Py) for the LA group.
4) Define the slope a and intercept b of the threshold line as a = SL and b = IC-k×SD (k is preferably 2 or 3), respectively.

In an embodiment, two-dimensional mapping of Ps and Pm of a specimen S enables the visualization of the cause of coagulation disorders in the specimen S. Specifically, Ps and Pm of the specimen S are plotted on a two-dimensional map with Ps on one axis and Pm on the other axis. For example, the plotted point of the specimen S is located at (Ps, Pm) on a two-dimensional map with Ps on the X-axis and Pm on the Y-axis. The relationship between Ps and Pm with respect to the coagulation reaction can be interpreted (explained) as follows: Ps is an index reflecting the weakness of the coagulation reaction of the specimen s, and Pm is an index reflecting the strength of inhibition of the coagulation reaction. Therefore, when comparing the relative positions of Inh, Def, and LA on the two-dimensional map, Ps for Inh (which has a coagulation factor inhibitor) is larger than that for Def and LA, and Pm for LA (which has autoantibodies against phospholipids which contribute to the coagulation reaction) is larger than that for Def. For Inh as well, the higher the titer, the stronger the inhibition of the coagulation reaction, so Pm for Inh is affected by the titer and increases in proportion to the titer. That is, for Inh, Pm for Inh-A, which has a relatively high titer, is larger than Pm for Inh-B, which has a relatively low titer. In addition, it can be inferred that (Ps, Pm) of a specimen whose APTT prolongation is caused by a combination of the weakness of the coagulation reaction and the strength of inhibition of the coagulation reaction is located on the two-dimensional map depending on the degree of influence of both factors. Since Ps and Pm have such characteristics, the specimen S can be estimated to be Inh, LA, or Def based on the position of the plotted point on the two-dimensional map.

Lines representing Ls and Lm (threshold lines or dividing lines) may be displayed on the two-dimensional map. For example, the two-dimensional map is divided into four sections by lines representing Ls and Lm. The cause of coagulation disorders in the specimen S can be visually estimated depending on in which the four sections the plotted point of the specimen S is located. For example, on a two-dimensional map with Ps on the x-axis and Pm on the Y-axis, if the specimen S is Inh, its plotted point (Ps, Pm) is located in a section to the right of the line representing Ls (also referred to as the section HX). Otherwise (when the plotted point is located in a section to the left of the line representing Ls, which is also referred to as the section LX), the specimen S is LA or Def. In that case, if the plotted point (Ps, Pm) is located in a section above the line representing Lm (also referred to as the section LH), the specimen S is LA. Otherwise (when the plotted point is located in a section below the line representing Lm, which is also referred to as the section LL), the specimen S is Def.

Alternatively, lines representing Ls and Lc may be displayed on the two-dimensional map. As described above, the specimen S can be estimated to be Inh, LA, or Def based on Ls. Of the specimens estimated to be LA or Def, if the plotted point is located in the section above the line representing Lc, the specimen S is LA; otherwise, the specimen S is Def.

### 9. Re-estimation of specimen estimated to be LA

Specimens with coagulation disorders include those derived from patients with acquired hemophilia A (AHA), although they are very rare. AHA is a disease presenting with bleeding symptoms due to the appearance of autoantibodies against coagulation factors (mostly FVIII) caused by acquired autoimmune abnormalities. AHA is characterized in that despite being positive for coagulation factor inhibitors, the activity of coagulation factors remains. AHA has normal PT but a prolonged APTT, and is accompanied by an increase in inhibitor titer and a decrease in coagulation factor activity. AHA is a disease which requires rapid and accurate diagnosis and treatment. Diagnosis of AHA generally requires cross-mixing tests, inhibitor titer testing, and the like. However, only a limited number of facilities are able to perform inhibitor titer testing, and many facilities rely on outsourced testing. In addition, cross-mixing tests are not popular tests, and are not always performed even in large-scale facilities where they are essentially required. That is, there is only a limited number of medical institutions which can perform tests for AHA diagnosis, and only a very limited number of facilities can provide test results on the day of blood collection. On the other hand, AHA requires rapid diagnosis, immediately followed by appropriate treatment (hemostatic therapy and immunosuppressive therapy). Since the treatment method differs from that for other coagulation disorders, the time it takes to obtain test results poses the risk of delayed initiation of appropriate treatment for AHA patients.

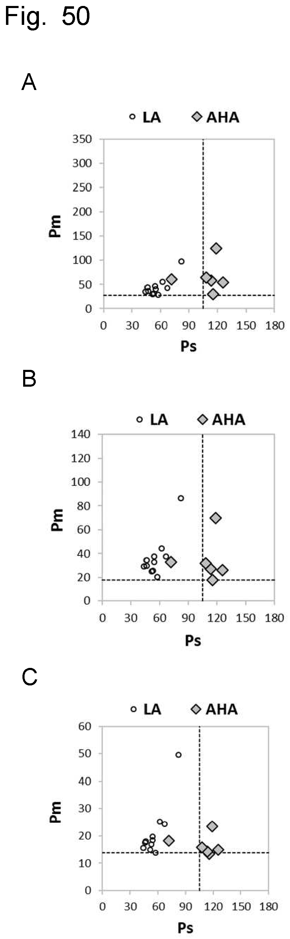

When examining the positions of the plotted points of AHA patient-derived specimens (hereinafter also referred to as "AHA") on a Ps-Pm two-dimensional map based on the method of the present invention described above, they may not enter the Inh section HX, despite being positive for coagulation factor inhibitors (see Fig. 50). Since the onset of AHA is extremely rare, the probability of its occurrence is also expected to be extremely low; however, AHA can pose a serious problem for the estimation results of the cause of coagulation disorders by the present invention. For example, if the plotted point of AHA is located in the LA section (e.g., the section LH described above) on the two-dimensional map, this AHA may be misestimated to be LA. However, since the treatment for LA differs from the treatment for AHA, misestimation as LA poses a risk for AHA patients. Therefore, it is clinically meaningful to examine the possibility that a specimen which has been estimated to be LA by the method of the present invention described above is a suspected AHA specimen. Further, it is at least beneficial to be able to quickly confirm the possibility of suspected AHA in subject specimens.

Therefore, in a further embodiment of the method of the present invention, for a specimen which has been estimated to be LA based on Ps and Pm calculated from the coagulation reaction in the APTT measurement described above, whether the specimen is suspected to be AHA is estimated again based on a parameter Pd. In the present embodiment, the parameter Pd is calculated for the specimen which has been estimated to be LA based on Ps and Pm. Pd is calculated based on Td(X), and Td(X) = Ts(X)-Tm(X) (0<X≤100). In the present specification and drawings, Td(X) may be referred to as "TdX" or simply as "Td."

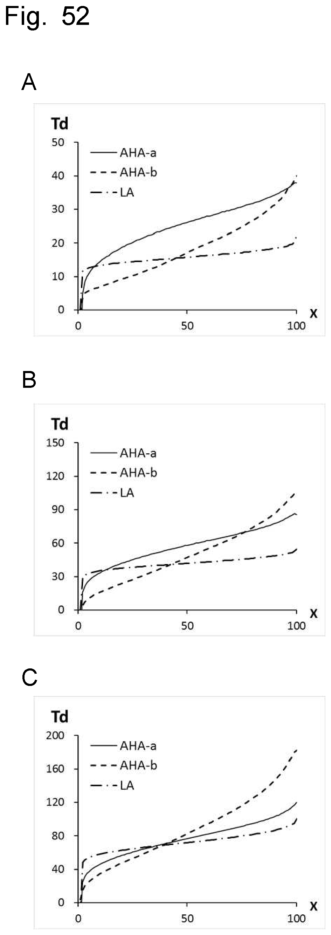

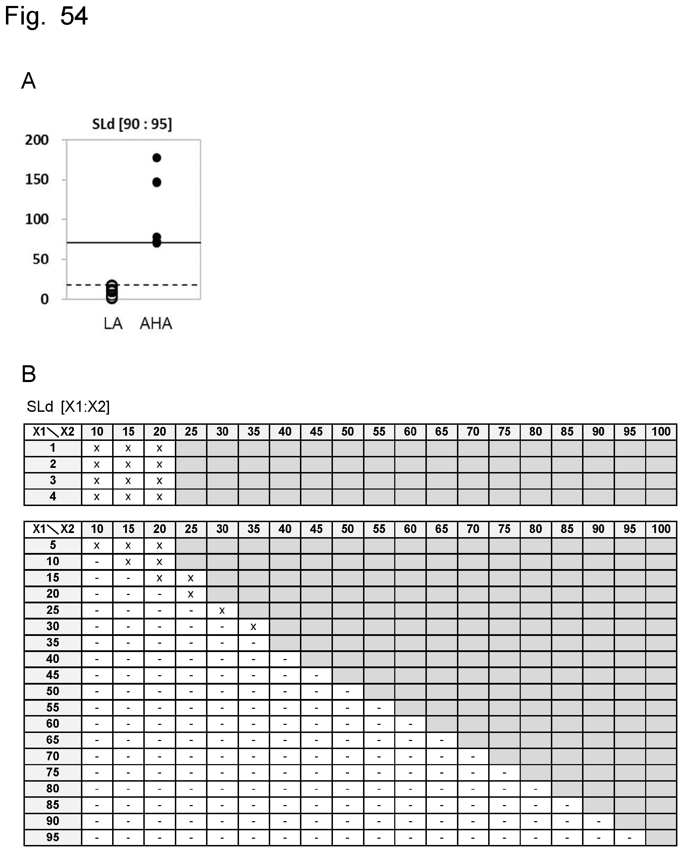

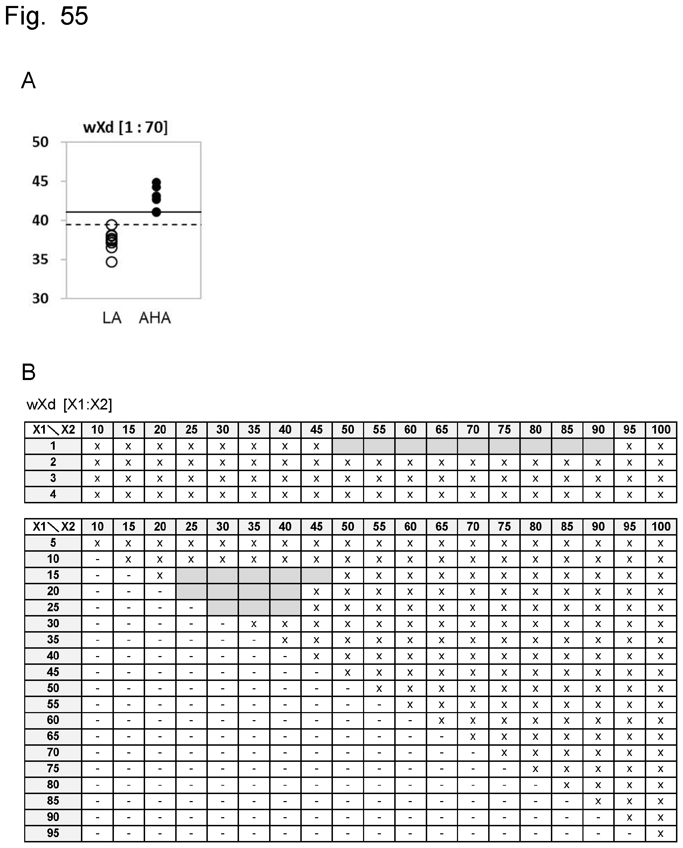

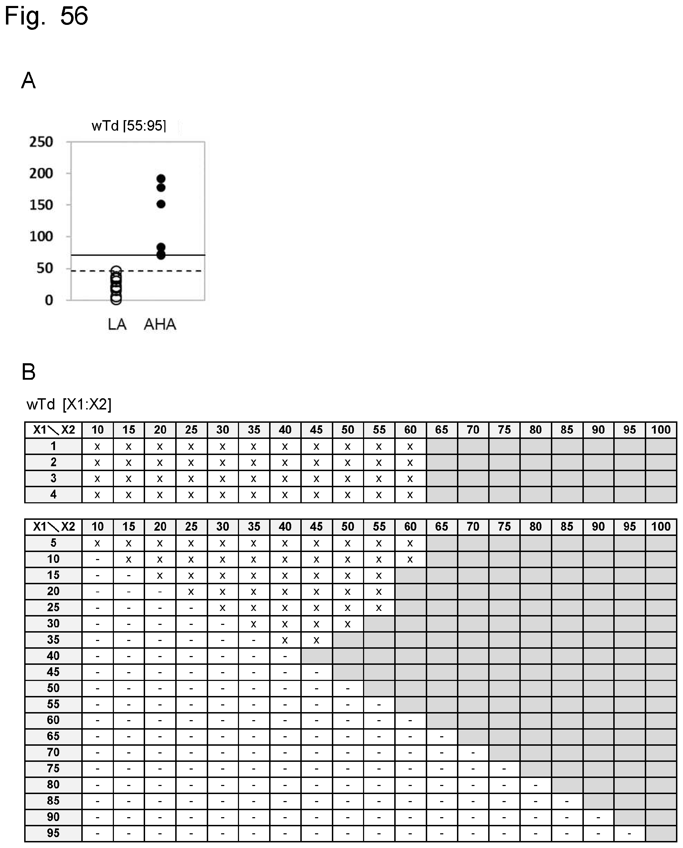

Examples of Pd include Td(X1), Td(X2)-Td(X1), SLd[X1:X2], wXd[X1:X2], and wTd[X1:X2] of the following equation, as shown in the Examples (Figs. 52 to 56) described later. In the following equation, Qd is a coefficient for adjusting the size of Pd, Qd>0, and it can be selected from, for example, a range from 1 to 100.
- Td(X1) (wherein preferably X1 = 43 to 100);
- Td(X2)-Td(X1) (wherein X1 = 1 to 80, X2 = 15 to 100, and X2-X1 = 5 to 99);
   - $SLd \left[X1:X2\right] = \frac{{\sum }_{X = X 1}^{X 2} \left\{\left(X-UX\right)\times \left(Td\left(X\right)-UTd\right)\right\}}{{\sum }_{X = X 1}^{X 2} {\left(X-UX\right)}^{2}} \times Qd$ (wherein X1 = 1 to 95, X2 = 25 to 100, X2-X1 = 5 to 99, UX is an average of X from X1 to X2, and UTd is an average of Td(X) from Td(X1) to Td(X2));
   - $wXd \left[X1:X2\right] = \frac{{\sum }_{X = X 1}^{X 2} \left\{X\times Td\left(X\right)\right\}}{{\sum }_{X = X 1}^{X 2} Td \left(X\right)} \times Qd$ (wherein X1 = 1, X2 = 50 to 90, or X1 = 15 to 25, X2 = 25 to 45, and X2-X1 = 5 to 30);
   - $wTd \left[X1:X2\right] = \frac{{\sum }_{X = X 1}^{X 2} \left\{X\times Td\left(X\right)\right\}}{{\sum }_{X = X 1}^{X 2} X} \times Qd$ (wherein X1 = 1 to 95, X2 = 45 to 100, and X2-X1 = 5 to 99).

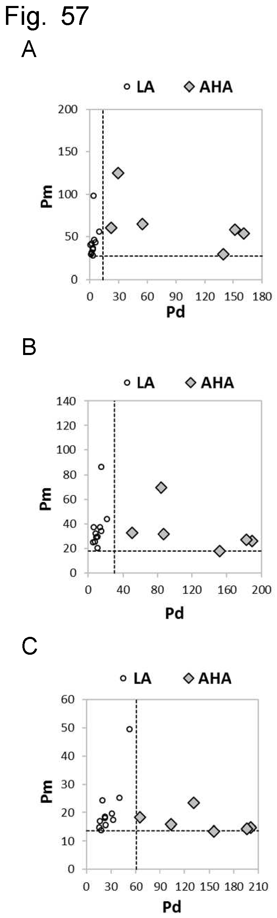

Based on the calculated Pd, whether the estimated LA specimen is suspected to be AHA (hereinafter also referred to as Inh-X) is estimated. As shown in the Examples described later, although the specimen is actually AHA, Pd of AHA located in the section LH on the Pd-Pm two-dimensional map tends to be larger than that of true LA (see Fig. 57). Therefore, if Pd of a specimen is larger than a threshold value Ld, the specimen is estimated to be Inh-X. For example, a section in which Pd>Ld and Pm>Lm on the Pd-Pm two-dimensional map is set to a section dHH, a specimen whose plotted point (Ps, Pm) is located in the section dHH is estimated to be Inh-X. The threshold value Ld can be determined prior to the implementation of the method of the present invention. For example, Ld can be determined based on Pd calculated from a specimen group with AHA, a specimen group with true LA, or a normal specimen group. Ld can be set for each Pd. On the other hand, if Pd of a specimen is equal to or less than Ld, the specimen is estimated to be LA.

If necessary, two threshold values Ld1 and Ld2 related to Pd may be set. However, Ld1>Ld2. For example, if Pd of a specimen located in the section LH on the Ps-Pm two-dimensional map is equal to or larger than Ld1, the specimen is estimated to be Inh-X, whereas if Pd of a specimen is equal to or less than Ld2, the specimen is estimated to be LA.

In an example, Pd of each specimen is calculated from a specimen group with true LA, and Ld is set based on their statistics (e.g., average value + [k × standard deviation], k is preferably from 2 to 5). In another example, Pd of each specimen is calculated from a specimen group with true LA, and the maximum value × k (k is preferably 1<k) is set to Ld. These Ld may be set to Ld2 when threshold values Ld1 and Ld2 are set.

In another example, Pd of each specimen is calculated from a specimen group with AHA, and Ld is set based on their statistics (e.g., average value - [k × standard deviation], k is preferably from 2 to 5). In another example, Pd of each specimen is calculated from a specimen group with AHA, and the minimum value × k (k is preferably 0<k<100%) is set to Ld. These Ld may be set to Ld1 when threshold values Ld1 and Ld2 are set.

In another example, Ld is set between the maximum value of Pd from a specimen group with true LA, and the minimum value of Pd from a specimen group with AHA.

In another example, Pd of each specimen is calculated from a normal specimen group, and Ld is set based on their statistics (e.g., average value + [k × standard deviation], k is preferably from 2 to 5).

### 10. Estimation flow

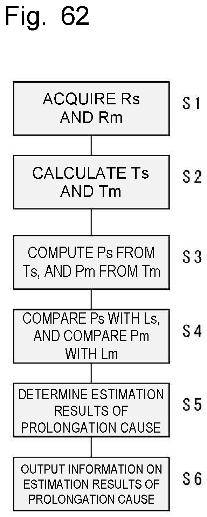

Figs. 62 to 66 each illustrate an exemplary flow for estimating the cause of coagulation disorders in a subject specimen by the method of the present invention. As an embodiment, Fig. 62 illustrates a flow for estimating the cause of coagulation disorders in a specimen with a known APTT prolongation (specimen S). The detailed steps of the flow are shown below.
S1: Acquire Rs of the specimen S. Perform APTT measurement on a mixed specimen of the specimen S and a normal specimen (specimen M) to acquire Rm.
S2: Calculate Ts from Rs, and calculate Tm from Rm.
S3: Compute a parameter Ps from Ts, and compute a parameter Pm from Tm.
S4: Compare Ps with Ls, and compare Pm with Lm.
S5: Determine the estimation results of the cause of prolongation as one of the following four options based on the comparison in S4.

If Ps>Ls and Pm>Lm, the subject specimen is estimated to be Inh-A.

Note: Inh-A is an inhibitor specimen with a relatively high strength of inhibition of the coagulation reaction.

If Ps>Ls and Pm≤Lm, the subject specimen is estimated to be Inh-B.

Note: Inh-B is an inhibitor specimen with a relatively low strength of inhibition of the coagulation reaction.

If Ps≤Ls and Pm>Lm, the subject specimen is estimated to be LA.

If Ps≤Ls and Pm≤Lm, the subject specimen is estimated to be Def.
S6: Output information related to the estimation results of the cause of prolongation.

Note: If necessary, Ps and Pm may be output on a two-dimensional map.

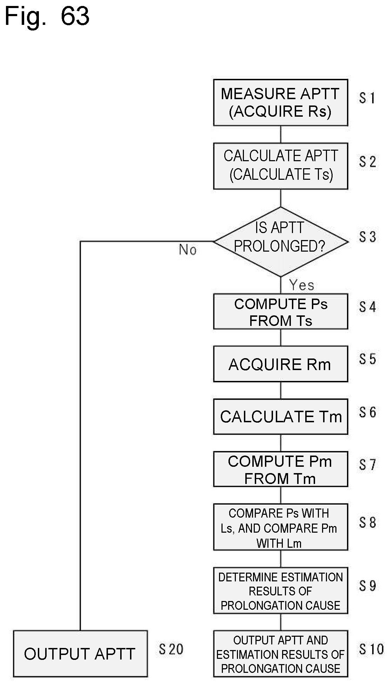

As an embodiment, Fig. 63 illustrates a flow for measuring APTT and estimating the cause of coagulation disorders in a specimen with an unknown APTT. The detailed steps of the flow are shown below.
S1: Measure APTT of a subject specimen (specimen S candidate) (acquire Rs).
S2: Calculate APTT from Rs (calculate Ts).
S3: Determine whether APTT is prolonged.

If it is determined that APTT is prolonged, then proceed to S4.

If it is not determined that APTT is prolonged, then proceed to S20.
S4: Compute a parameter Ps from Ts.
S5: Measure APTT of a mixed specimen of the subject specimen and a normal specimen (specimen M) (acquire Rm).
S6: Calculate Tm from Rm.
S7: Compute a parameter Pm from Tm.
S8: Compare Ps with Ls, and compare Pm with Lm.
S9: Determine the estimation results of the cause of prolongation as one of the following four options based on the comparison in S8.

If Ps>Ls and Pm>Lm, the subject specimen is estimated to be Inh-A.

Note: Inh-A is an inhibitor specimen with a relatively high strength of inhibition of the coagulation reaction.

If Ps>Ls and Pm≤Lm, the subject specimen is estimated to be Inh-B.

Note: Inh-B is an inhibitor specimen with a relatively low strength of inhibition of the coagulation reaction.

If Ps≤Ls and Pm>Lm, the subject specimen is estimated to be LA.

If Ps≤Ls and Pm≤Lm, the subject specimen is estimated to be Def.
S10: Output information related to APTT and the estimation results of the cause of prolongation.

Note: If necessary, Ps and Pm may be output on a two-dimensional map.
S20: Output APTT.

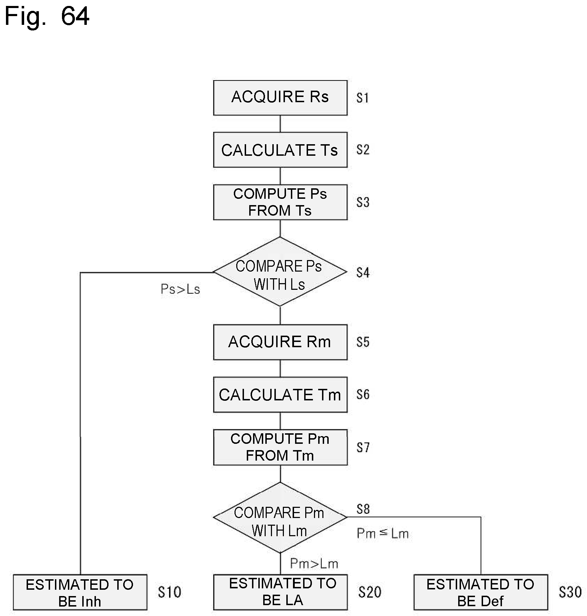

As an embodiment, Fig. 64 illustrates a flow for, after finding an APTT prolongation specimen (specimen S) in normal APTT measurement, subsequently estimating the cause of coagulation disorders in the specimen. The detailed steps of the flow are shown below.
S1: Acquire Rs from the APTT measurement data of the specimen S.
S2: Calculate Ts from Rs.
S3: Compute a parameter Ps from Ts.
S4: Compare Ps with Ls.

If Ps>Ls, then proceed to S10.

If Ps≤Ls, then proceed to S5.
S5: Measure APTT of a mixed specimen of the specimen S and a normal specimen (specimen M) (acquire Rm).
S6: Calculate Tm from Rm.
S7: Compute a parameter Pm from Tm.
S8: Compare Pm with Lm.

If Pm>Lm, then proceed to S20.

If Pm≤Lm, then proceed to S30.
S10: Estimate the subject specimen to be Inh.
S20: Estimate the subject specimen to be LA.
S30: Estimate the subject specimen to be Def.

Note: In S10 to S30, if necessary, APTT may be output, and Ps and Pm may be output on a two-dimensional map.

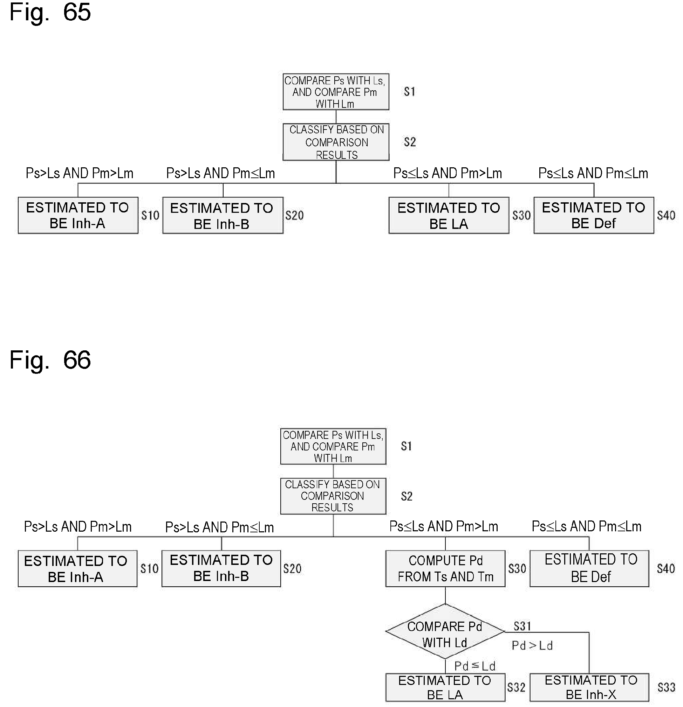

As an embodiment, Fig. 65 illustrates a detailed flow for comparing parameters and estimating the cause of coagulation disorders in S4 and S5 of Fig. 62, or S8 and S9 of Fig. 63.

As an embodiment, Fig. 66 illustrates a flow prepared by further adding a step of Inh-X estimation using Pd to the flow of Fig. 65. The flow of Fig. 66 is the same as that of Fig. 65, except for the steps following S30. The detailed steps of the flow following S30 are shown below.
S30: When Ps≤Ls and Pm>Lm:
   Calculate Td from Ts and Tm, and compute a parameter Pd.
S31: Compare Pd with Ld.

If Pd≤Ld, then proceed to S32.

If Pd>Ld, then proceed to S33.
S32: Estimate the subject specimen to be LA.
S33: Estimate the subject specimen to be Inh-X.

In order to identify each section, a section code (HH, HL, LH, LL, and dHH) is assigned to each section, and section information using the section codes can be expressed as estimation results. In addition, it is possible to output the corresponding section information as estimation results.

### 11. Application to other coagulation reaction measurement methods

The method for estimating the cause of coagulation disorders according to the present invention is described above using coagulation reaction measurement based on the amount of scattered light as an example. However, a person skilled in the art would be able to apply the method based on the amount of scattered light described above to methods using other coagulation reaction measurement methods (e.g., blood coagulation reaction measurement methods based on transparency, absorbance, viscosity, and the like). Therefore, this application is included in the scope of the present invention.

### 12. Others

In the procedure for estimating the cause of coagulation disorders described above, a person skilled in the art would understand that the phrase "above the threshold value" may be used in place of "equal to or larger than the threshold value," or the phrase "below the threshold value" may be used in place of "equal to or less than the threshold value," as long as different causes of coagulation disorders can be estimated in a distinguishing manner. Further, in the estimation using a two-dimensional map, a person skilled in the art would understand that "the section to the right of (above) the line representing the threshold value" can be defined as a section which contains or does not contain the part on the line, as long as different causes of coagulation disorders can be estimated in a distinguishing manner.

The estimation results of the cause of coagulation disorders in subject specimens obtained by the above procedure can be output in any format. For example, the estimation results can be output in the form of electronic data, printout, or display on a screen. The estimation results preferably include information that the subject specimen is any of Inh, LA, and Def. Alternatively, when no prolonged coagulation time is found in the subject specimen, information that the subject specimen is normal or does not have coagulation disorders (or prolonged coagulation time) may be output. If necessary, together with the estimation results, the coagulation time of the subject specimen, and further R(i), Ts(X), Tm(X), vT1s(X) and vT2s(X), vT1m(X) and vT2m(X), Ps, Pm, Pd, and the like for the subject specimen may also be output. Further, Ps, Pm, and Pd may be output as a relative ratio to the statistics of a specimen group of normal specimens (NP) (when the mean of Ps, Pm, or Pd of the NP group is M, and the standard deviation is SD, denominator of relative ratio = M+k×SD, k = 2 or 3). Alternatively, a relative ratio based on Ps, Pm, and Pd (when the Ps, Pm, or Pd value of NP is N, denominator of relative ratio = N×k, k = 100 to 200%) of the selected NP (e.g., pooled plasma from multiple normal specimens and normal plasma for cross-mixing tests) may be output. Further, as information corresponding to the value of each relative ratio, for example, + or ++ may be output as a flag indicating the size of the value. In addition, information on a section (e.g., HH, HL, LH, LL, or dHH) in which the plotted point (Ps, Pm) or (Pd, Pm) of the subject specimen is located on a two-dimensional map may be output.

### 13. Program and device

The method for estimating the cause of coagulation disorders according to the present invention described above can be performed automatically using a computer program. Therefore, an embodiment of the present invention is a program for performing the method for estimating the cause of coagulation disorders according to the present invention. In an embodiment, the program of the present invention is a program for running a process of calculating Ps and Pm described above; a process of estimating whether a specimen S is Inh based on the calculated Ps; and a process of estimating a specimen S which has not been estimated to be Inh to be LA or Def based on the calculated Pm. In an embodiment, the program of the present invention further runs a process of calculating Ts(X), or vT1s(X) and vT2s(X), as well as Tm(X), or vT1m(X) and vT2m(X) before the process of calculating Ps and Pm. In an embodiment, the program of the present invention further runs a process of calculating R(i) or V(i) for specimen S and specimen M. The resulting R(i) or V(i) can be used to calculate Ts(X) and the like, or calculate the coagulation time of the specimen S. In an embodiment, the program of the present invention further runs a process of calculating the coagulation time of the specimen S. In an embodiment, when the calculated coagulation time of the specimen S is prolonged, the program of the present invention runs a process of calculating Ts(X), or vT1s(X) and vT2s(X), as well as Tm(X), or vT1m(X) and vT2m(X) described above. In an embodiment, the program of the present invention further runs a process of outputting the estimation results obtained in the process of estimating the cause of coagulation disorders (Inh, LA, or Def) in the specimen S described above.

In an embodiment, the program of the present invention further runs a process of calculating Td(X) and Pd of a specimen S which has been estimated to be LA. In an embodiment, the program of the present invention further runs a process of estimating whether the specimen S is Inh-X based on the calculated Pd. In an embodiment, the program of the present invention further runs a process of outputting the estimation results of the cause of coagulation disorders (Inh, LA, Def, or Inh-X) in the specimen S obtained in the estimation process described above.

In a preferred embodiment, the program of the present invention is a program for performing the flows of Figs. 62 to 66 described above.

An embodiment of the present invention is a device for performing the method for estimating the cause of coagulation disorders according to the present invention described above. The device comprises a calculator for running the program of the present invention described above to estimate the cause of coagulation disorders in subject specimens. If necessary, the device comprises a measurement device for measuring the coagulation reactions of specimens, or an output device for outputting the estimation results of the cause of coagulation disorders.

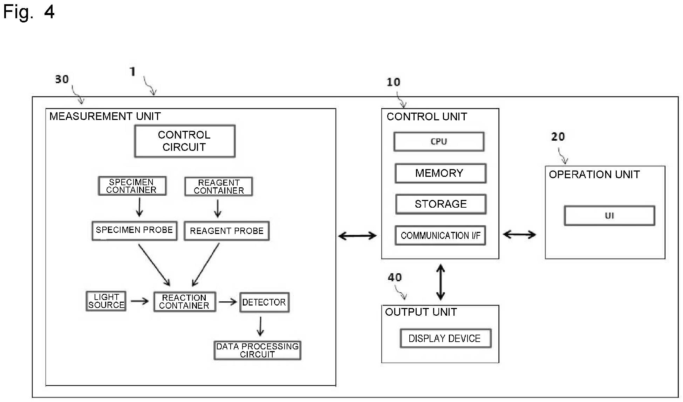

An embodiment of the device of the present invention will be described below. An embodiment of the device of the present invention is an automated analyzer 1 as shown in Fig. 4. The automated analyzer 1 comprises a control unit 10, an operation unit 20, a measurement unit 30, and an output unit 40.

The control unit 10 controls the overall operation of the automated analyzer 1. The control unit 10 can be composed of, for example, a calculator (e.g., a personal computer). The control unit 10 comprises a CPU, memory, storage, communication interface (I/F), and the like, and processes commands from the operation unit 20, controls the operation of the measurement unit 30, stores and analyzes the measurement data received from the measurement unit 30, stores the analysis results, and controls the output of the measurement data and analysis results by the output unit 40. Furthermore, the control unit 10 may be connected to other devices, such as external media and a host computer. In the control unit 10, the calculator which controls the operation of the measurement unit 30 and the calculator which analyzes the measurement data may be the same or different from each other.

The operation unit 20 obtains the input from the operator, and transmits the obtained input information to the control unit 10. For example, the operation unit 20 comprises user interfaces (UI), such as a keyboard and a touch panel. The output unit 40 outputs, under the control of the control unit 10, the measurement data from the measurement unit 30, the coagulation reaction curve R, coagulation rate curve V, coagulation reaction end point E, coagulation time, T(X), vT1(X), vT2(X), Ps, Pm, and Pd based on the measurement data, the estimation results of the cause of coagulation disorders (e.g., Inh, LA, or Def) in the specimen, a two-dimensional map showing the plotted point (Ps, Pm) of the specimen, information on the section (e.g., HH, HL, LH, LL, or dHH) in which the plotted point (Ps, Pm) of the specimen is located on the two-dimensional map, information related to the estimation results (e.g., flag), and other analysis results. For example, the output unit 40 comprises a display device, such as a display.

The measurement unit 30 executes a series of operations for a blood coagulation test, and obtains the measurement data of the coagulation reactions of specimens including blood specimens. The measurement unit 30 comprises various equipment and analysis modules necessary for the blood coagulation test, such as a specimen container for containing a blood specimen, a reagent container for containing a testing reagent, a reaction container for reacting the specimen and reagent, probes for dispensing the specimen, diluent, reagent, and the like into the reaction container, a light source, a detector for detecting scattered light or transmitted light from the specimen in the reaction container, a data processing circuit which sends the data from the detector to the control unit 10, and a control circuit which controls the operation of the measurement unit 30 upon receiving instructions from the control unit 10.

The control unit 10 estimates the cause of coagulation disorders in the specimen based on the data measured by the measurement unit 30. This analysis may include the calculation of Ps and Pm, and the estimation of the cause of coagulation disorders in the specimen using Ps and Pm. Furthermore, the control unit 10 may acquire the coagulation reaction curve R, coagulation rate curve V, coagulation time, coagulation reaction end point E, T(X), vT1(X), vT2(X), and the like. Alternatively, the coagulation reaction curve R and E may be acquired by the control unit 10 based on the measurement data from the measurement unit 30, or may be acquired by another device, such as the measurement unit 30, and sent to the control unit 10. The control unit 10 may store the coagulation time of specimens, and various parameters used for calculating T(X), V, vT1(X), vT2(X), Ps, Pm, and Pd **(e.g.,** equations for calculating Ps, Pm, and Pd). The control unit 10 may also store parameters used to estimate the cause of coagulation disorders in specimens **(e.g.,** the threshold values Ls, Lm, and Ld described above). Alternatively, the control unit 10 may import the parameters and reference values stored in an external device or on a network during analysis.

The above analysis can be performed by the program for performing the method of the present invention described above. Therefore, the control unit 10 may comprise a program for performing the method for estimating the cause of coagulation disorders according to the present invention.

The analysis results in the control unit 10 are sent to the output unit 40 and output. The output can take any form, such as display on a screen, sending to the host computer, or printing. The output information from the output unit may include the coagulation reaction curve R, coagulation rate curve V, coagulation reaction end point E, coagulation time, T(X), vT1(X), vT2(X), Ps, Pm, Pd, and the estimation results of the cause of coagulation disorders in specimens. The type of information output from the output unit can be controlled by the program of the present invention.

### Examples

The present invention will be described in more detail below with reference to Examples; however, the present invention is not limited to these Examples.

### Example 1: Estimation of cause of coagulation disorders

### 1. Method

### 1) Specimen

The following specimens with a prolonged coagulation time were used as subject specimens.
- Coagulation factor inhibitor-positive specimens (Inh): plasma of patients (N = 15 in total) who were diagnosed as positive for FVIII inhibitors (N = 11, titer: from 0.8 to 260 BU/mL), and positive for FIX inhibitors (N = 4, titer: from 0.6 to 3.2 BU/mL).
- LA-positive specimens (LA): plasma of patients (N = 11) who were diagnosed as positive for LA.
- Coagulation factor-deficient specimens (Def): plasma of patients (N = 65 in total) who were diagnosed as FVIII-deficient (N = 37), FIX-deficient (N = 23), and FV-deficient (N = 5), listed in Table 1 below, and Congenital Factor V Deficient Plasma (N = 1) of George King Bio-Medical, Inc., which had been confirmed to have an FV activity value of less than 1%.

**[Table 1]**

| Factor activity (IU/dL) | FVIII | FIX | FV |
|---|---|---|---|
| Activity < 1 | 10 | 5 | 1 |
| 1 ≤ activity < 5 | 21 | 11 | 1 |
| 5 ≤ activity < 40 | 6 | 7 | 3 |

The patients' blood was collected in a blood collection tube containing a 3.2% sodium citrate aqueous solution so that the ratio of the blood and the sodium citrate aqueous solution was 9:1. Next, the blood collection tube was centrifuged (at 1,500 g for 15 minutes), and the separated plasma was collected in a storage container and stored at -80°C. The frozen plasma was thawed at 37°C immediately before measurement to prepare a subject specimen. The normal plasma used for preparing mixed specimens was a pooled plasma of 20 normal people (NPP) in which no coagulation disorders were observed. The mixed specimens were prepared by mixing each subject specimen and NPP at a mixing ratio (capacity ratio of subject specimen to NPP) of 9:1, 1:1, and 1:9.

### 2) Coagulation reaction measurement

The APTT measurement reagents used were Coagpia APTT-N (manufactured by SEKISUI MEDICAL CO., LTD.) as a first reagent and Coagpia APTT-N Calcium Chloride Solution (25 mM calcium chloride solution, manufactured by SEKISUI MEDICAL CO., LTD.) as a second reagent. The coagulation reaction measurement was performed by using a blood coagulation automated analyzer CP3000 (manufactured by SEKISUI MEDICAL CO., LTD.). After 50 µL of a specimen was heated at 37°C for 45 seconds in a cuvette, 50 µL of the first reagent at about 37°C was added, and 50 µL of the second reagent was further added after the lapse of 171 seconds to start the coagulation reaction. The reaction was performed at 37°C. In the measurement of the coagulation reaction, the cuvette was irradiated with light with a wavelength of 660 nm using an LED light source, and the amount of 90-degree side scattered light was measured at intervals of 0.1 seconds. The measurement time was 600 seconds.

### 3) Acquisition of coagulation reaction curve

The photometric data from each specimen were subjected to smoothing processing including noise removal, and then to zero-point adjustment processing so that the amount of scattered light at the photometry start point was 0, thereby creating a coagulation reaction curve R(i).

### 4) Detection of coagulation reaction end point E and determination of APTT

R(i) at the earliest time point at which the integration ratio Z(i) of R(i) (see WO 2021/132552) was less than an integration ratio threshold value Zs was detected as a coagulation reaction end point E. The integration ratio threshold value Zs used was 1.001. A time point T(50) at which R(i) reached 50% of E was calculated and determined as APTT. The integration ratio Z(i) at the time point i was calculated as follows: $Integration ratio Z \left(i\right) = Rb \left(i\right) / Ra \left(i\right)$ $Ra \left(i\right) = sum of from R \left(i-20\right) to R \left(i-1\right)$ $Rb \left(i\right) = sum of from R \left(i+1\right) to R \left(i+20\right)$

### 5) Calculation of T(X)

For each specimen, a time T(X) when R(i) reached X% of E detected in 4) was calculated based on the following equations. Specifically, based on the following equations, R(i), E, and T(X) of the subject specimen and mixed specimen were calculated as Rs(i), Es, and Ts(X) for the subject specimen, and as Rm(i), Em, and Tm(X) for the mixed specimen. X was set in increments of 1 from 1 to 100, and T(1) to T(100) were calculated. APTT was defined as T(50) when X = 50. $Rs \left(Ts\left(X\right)\right) = Es \times X %$ $Rm \left(Tm\left(X\right)\right) = Em \times X %$

### 6) Calculation of function related to V(i)

Rs(i) and Rm(i) were first differentiated, and coagulation rate curves Vs(i) and Vm(i) were calculated, respectively. The maximum values of Vs(i) and Vm(i) were obtained as Vmax_s and Vmax_m, respectively. vT1s(X), vT2s(X), vT1m(X), and vT2m(X) were calculated in accordance with the following equations (Xa) to (Xd), respectively. X was set to, in increments of 5, from 3 to 98, and to 100. $Vs \left(vT1s\left(X\right)\right) = Vmax _ s \times X %$ (provided that vT1s(X)≤VmaxT_s) $Vs \left(vT2s\left(X\right)\right) = Vmax _ s \times X %$ (provided that vT2s(X)>_VmaxT_s) $Vm \left(vT1m\left(X\right)\right) = Vmax _ m \times X %$ (provided that vT1m(X)≤VmaxT_m) $Vm \left(vT2m\left(X\right)\right) = Vmax _ m \times X %$ (provided that vT2m(X)≤VmaxT_m)

### 2. Coagulation reaction of specimen

### 1) T(X)

Fig. 3 illustrates T(X) (X is time (sec)) of specimens with about the same APTT (about 100 seconds) selected from an LA group, a Def group, and an Inh group. The specimen selected from the Inh group was an FVIII inhibitor-positive specimen (iFVIII) with a titer of 0.8 BU/mL. Further, the specimen selected from the Def group was an FVIII-deficient specimen (dFVIII) with a factor activity of less than 1%. Fig. 3A illustrates Ts(X) of each specimen, and Fig. 3B illustrates Tm(X) of mixed specimens with a mixing ratio (subject specimen:NPP) of 1:1. In both Figs. 3A and B, the solid line indicates LA, the dashed line indicates dFVIII (Def), and the chain line indicates iFVIII (Inh). Figs. 3C to E illustrate Ts (solid line) and Tm (dashed line) for iFVIII (Inh), dFVIII (Def), and LA, respectively.

As shown in Fig. 3A, even if Ts(X) was about the same (APTT was about 100 seconds) when X = 50, the slope of Ts(X) for Inh was significantly large, and when X>50, the difference between Ts(X) for Inh and Ts(X) for LA and Def increased with the increase in X. Therefore, it was suggested that specimens which are estimated to be Inh can be distinguished from subject specimens with a prolonged coagulation time based on Ts(X).

Further, as shown in Fig. 3B, the increase in Tm(X) of the Def-derived mixed specimen with the increase in X was small. This was presumably because the coagulation reaction approached that of the normal specimen due to the action of coagulation factors derived from NPP which was mixed with the specimen S for the preparation of the mixed specimen. On the other hand, Tm(X) of the LA-derived mixed specimen increased with the increase in X. This was presumably because phospholipids derived from NPP mixed with the specimen S for the preparation of the mixed specimen immediately bound to autoantibodies against phospholipids contained in the specimen S, thus inhibiting the coagulation reaction.

### 3. Estimation of Inh based on Ps

### 1) rTs(X1)

As Ps, rTs(X1) of each specimen was calculated in accordance with the following equation (IIa'). $rTs \left(X1\right) = Ts \left(X1\right) / Ts \left(As\right) \times Qs$ (X1 = 10, 60, 95, or 100, As = 50, Qs = 1)

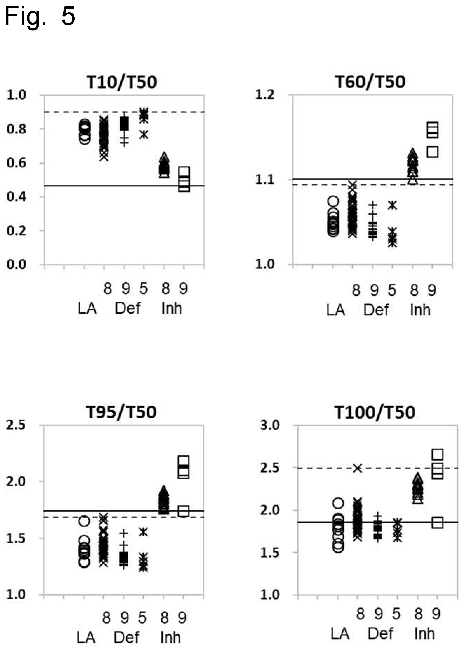

Fig. 5 illustrates the calculated rTs(X1) of each specimen. In the figure, the solid line indicates the minimum value of rTs(X1) in the Inh group, and the dashed line indicates the maximum value of rTs(X1) in the LA and Def groups. When X1>As, rTs(X1) for Inh tended to be larger than that for LA and Def. Therefore, it was indicated that a threshold value which can distinguish Inh from LA and Def can be set based on rTs(X1), and that when X1>As, a specimen in which rTs(X1) is larger than the threshold value can be estimated to be Inh.

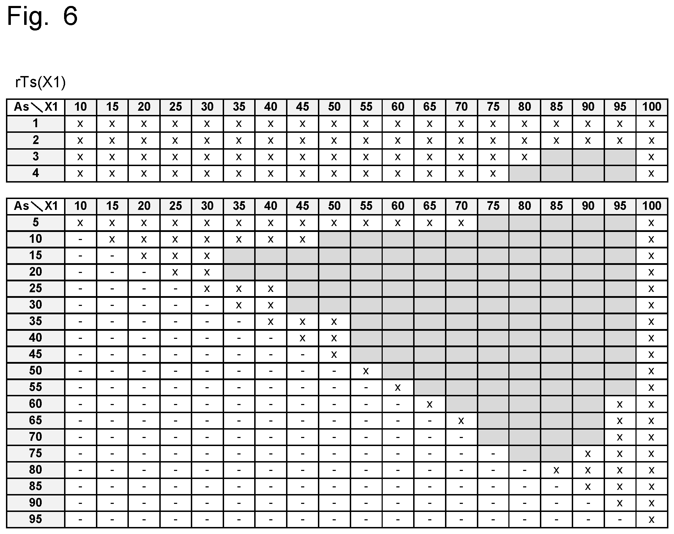

Fig. 6 illustrates the difference between the minimum value of rTs(X1) in the Inh group and the maximum value of rTs(X1) in the LA and Def groups when X1 and As (X1>As) were changed in various ways. In the figure, when the minimum value in the Inh group was larger than the maximum value in the LA and Def specimen groups, it was indicated in the gray column; otherwise, it was indicated with ×. It was confirmed that when X1>As, the minimum value of rTs(X1) in the Inh group tended to be larger than the maximum value of rTs(X1) in the LA and Def groups. However, this tendency did not appear when As≥80. These results indicated that rTs(X1) in the range of X1 = 35 to 95, As = 3 to 75, and X1-As = 5 to 92 was preferred for the estimation of Inh.

### 2) rTs(X2)-rTs(X1)

As Ps, rTs(X2)-rTs(X1) of each specimen was calculated in accordance with the following equation (IIIa'). $\begin{matrix}rTs \left(X2\right) - rTs \left(X1\right) \\ = \left(Ts\left(X2\right)-Ts\left(X1\right)\right) / Ts \left(As\right) \times Qs\end{matrix}$ (X1 = 20 and X2 = 95, As = 50, Qs = 1)

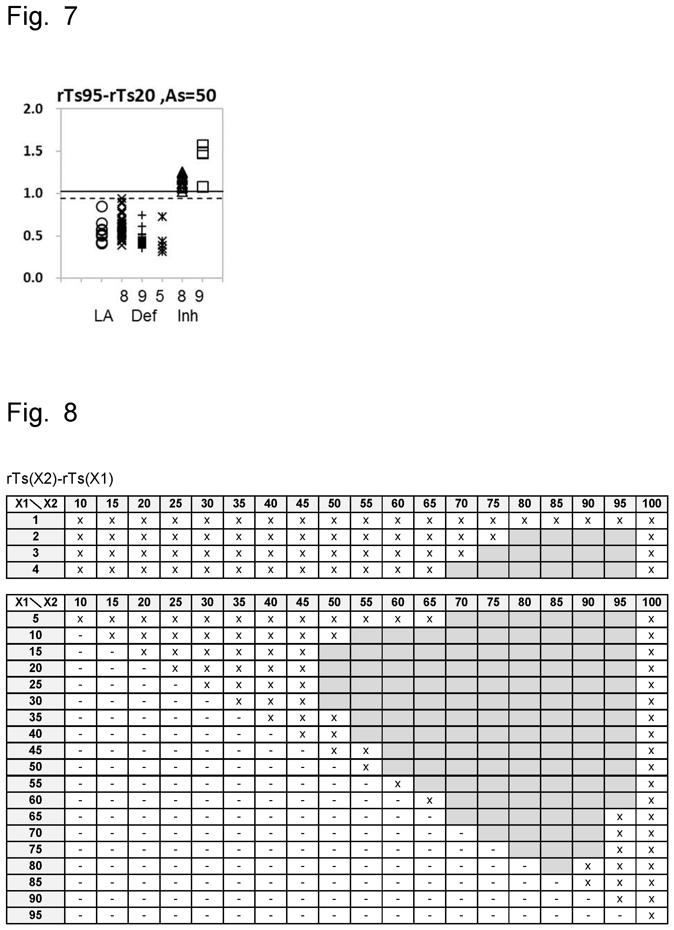

Fig. 7 illustrates the calculated rTs(X2)-rTs(X1) of each specimen. In the figure, the solid line indicates the minimum value of rTs(X2)-rTs(X1) in the Inh group, and the dashed line indicates the maximum value of rTs(X2)-rTs(X1) in the LA and Def groups. When X2>X1, rTs(X2)-rTs(X1) for Inh tended to be larger than that for LA and Def. Therefore, it was indicated that a threshold value which can distinguish Inh from LA and Def can be set based on rTs(X2)-rTs(X1), and that a specimen in which rTs(X2)-rTs(X1) is larger than the threshold value can be estimated to be Inh.

Fig. 8 illustrates the difference between the minimum value of rTs(X2)-rTs(X1) in the Inh group and the maximum value thereof in the LA and Def groups when X1 and X2 were changed in various ways at As = 50. In the figure, when the minimum value in the Inh group was larger than the maximum value in the LA and Def groups, it was indicated in the gray column; otherwise, it was indicated with ×. When X2>X1, the minimum value of rTs(X2)-rTs(X1) in the Inh group tended to be larger than the maximum value thereof in the LA and Def groups. However, this tendency did not appear when X1≥85, and when X2≤45 or X2 = 100. These results indicated that rTs(X2)-rTs(X1) in the range of X1 = 2 to 80, X2 = 50 to 95, and X2-X1 = 5 to 93 was preferred for the estimation of Inh.

### 3) arTs(X1,X2)

As Ps, arTs(X1:, X2), which is the ratio of the difference between rTs(X1) and rTs(X2) to the difference between X1 and X2, was calculated for each specimen in accordance with the following equation (IVa'). $\begin{matrix}arTs \left(X1,X2\right) \\ = \left\{rTs\left(X2\right)-rTs\left(X1\right)\right\} / \left(X2-X1\right) \\ = \left[\left\{Ts\left(X2\right)-Ts\left(X1\right)\right\}/Ts\left(As\right)\times Qs\right] / \left(X2-X1\right)\end{matrix}$ (X1 = 50, X2 = 95, As = 5, 50, or 95, Qs = 1)

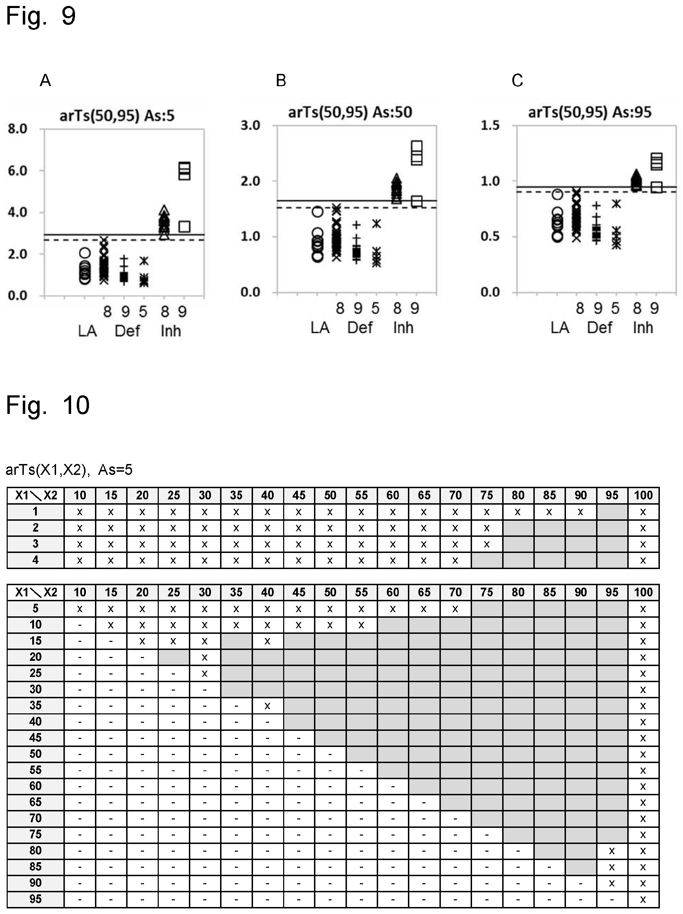

Figs. 9A to C illustrate the calculated arTs(X1,X2) of each specimen. In each figure, the solid line indicates the minimum value of arTs(X1,X2) in the Inh group, and the dashed line indicates the maximum value of arTs(X1,X2) in the LA and Def groups. arTs(X1,X2) for Inh tended to be larger than that for LA and Def. Therefore, it was indicated that a threshold value which can distinguish Inh from LA and Def can be set based on arTs(X1,X2), and that a specimen in which arTs(X1,X2) is larger than the threshold value can be estimated to be Inh.

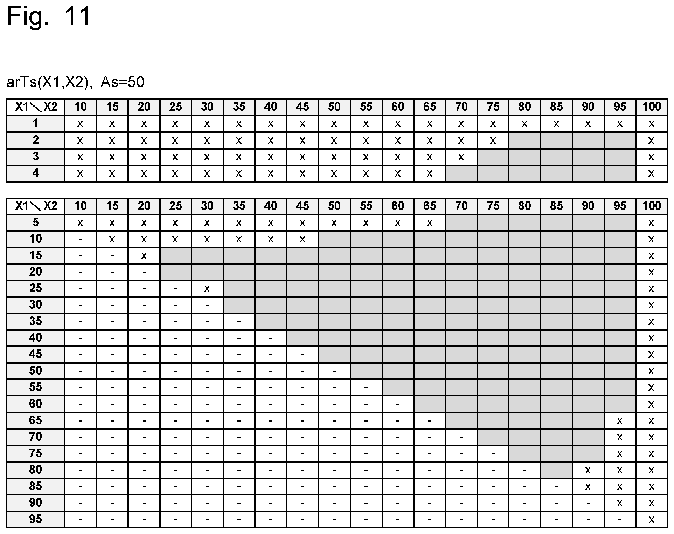

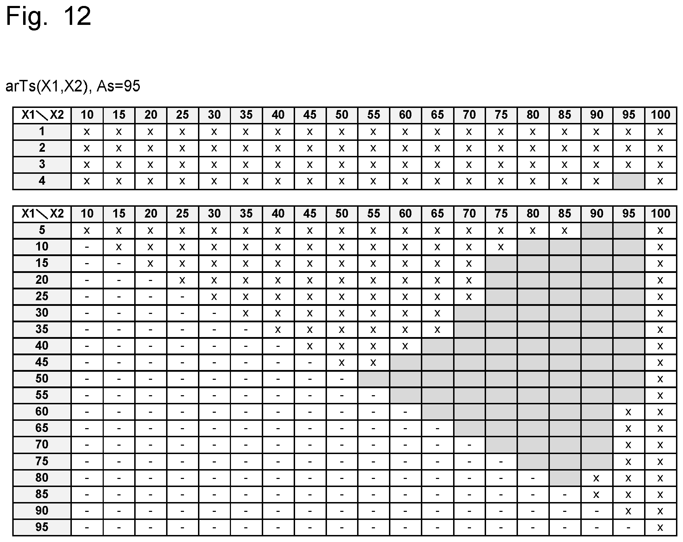

Figs. 10 to 12 illustrate the difference between the minimum value of arTs(X1,X2) in the Inh group and the maximum value thereof in the LA and Def groups when X1 and X2 were changed in various ways at As = 5, 50, and 95. In the figures, when the minimum value in the Inh group was larger than the maximum value in the LA and Def groups, it was indicated in the gray column; otherwise, it was indicated with ×. The conditions in which the minimum value of arTs(X1,X2) in the Inh group was larger than the maximum value of arTs(X1,X2) in the LA and Def groups were confirmed. These results indicated that arTs(X1:X2) in the range of As = 5 to 95, X1 = 1 to 85, X2 = 25 to 95, and X2-X1 = 5 to 94 can be used for the estimation of Inh, but arTs(X1,X2) in the range of X1 = 4 to 80, X2 = 55 to 95, and X2-X1 = 5 to 91 is preferred.

### 4) rSLs[X1:X2]

As Ps, rSLs[X1:X2], which is the slope of the regression line of rTs(X), was calculated for each specimen in accordance with the following equation (Va'). $\begin{matrix}rSLs \left[X1:X2\right] \\ = \frac{{\sum }_{X = X 1}^{X 2} \left\{\left(X-UX\right)\times \left(rTs\left(X\right)-UrTs\right)\right\}}{{\sum }_{X = X 1}^{X 2} {\left(X-UX\right)}^{2}} \times Qs\end{matrix}$

However, UX is an average of X from X1 to X2, and UrTs is an average of rTs(X) from rTs(X1) to rTs(X2).
(X1 = 10, X2 = 90, As = 5, 50, or 95, Qs = 100)

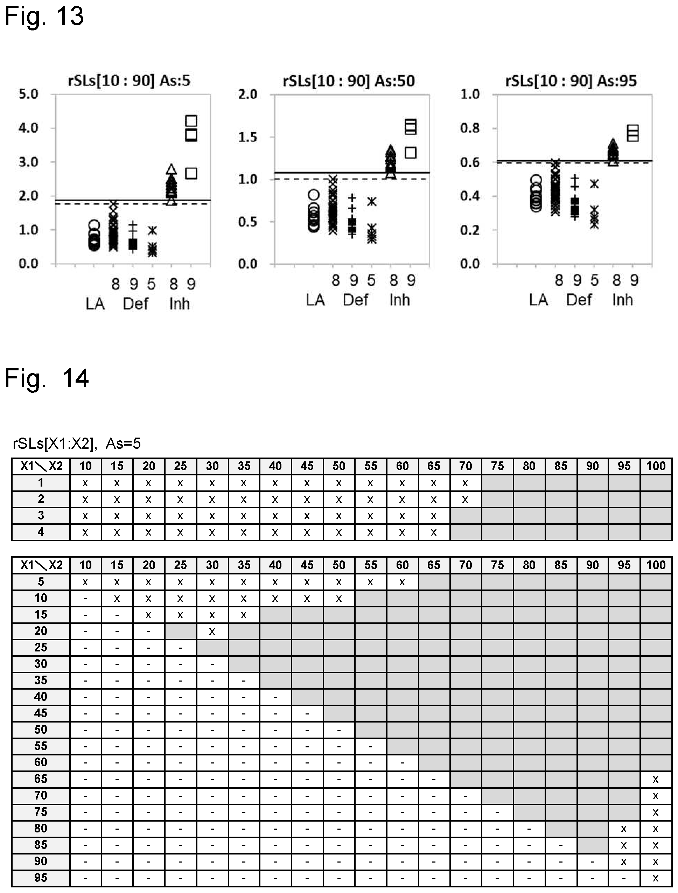

Fig. 13 illustrates the calculated rSLs[X1:X2] of each specimen. In the figure, the solid line indicates the minimum value of rSLs[X1:X2] in the Inh group, and the dashed line indicates the maximum value of rSLs[X1:X2] in the LA and Def groups. An example in which rSLs[X1:X2] for Inh was larger than that for LA and Def was confirmed. Therefore, it was indicated that a threshold value which can distinguish Inh from LA and Def can be set based on rSLs[X1:X2], and that a specimen in which rSLs[X1:X2] is larger than the threshold value can be estimated to be Inh.

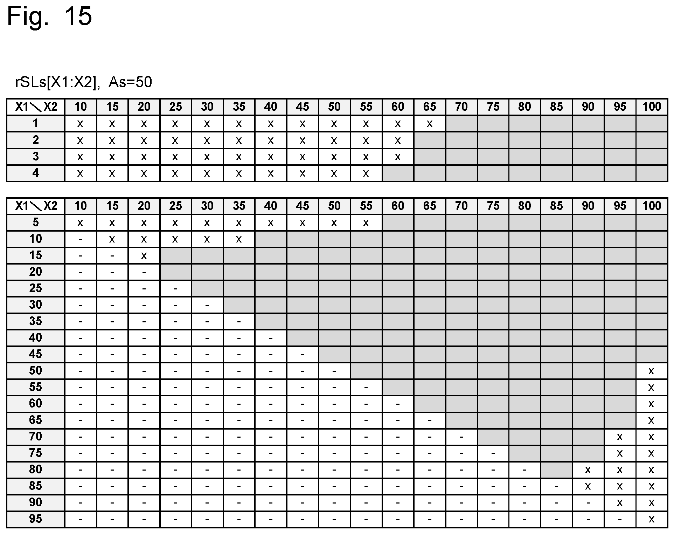

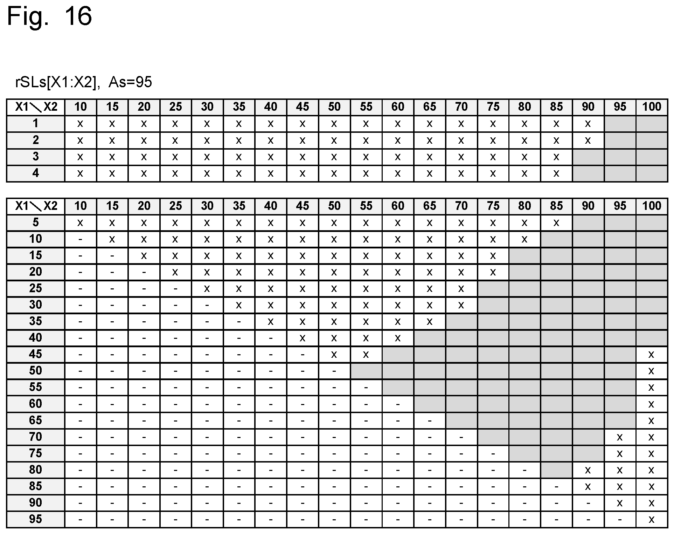

Figs. 14 to 16 illustrate the difference between the minimum value of rSLs[X1:X2] in the Inh group and the maximum value of rSLs[X1:X2] in the LA and Def groups when X1 and X2 were changed in various ways at As = 5, 50, and 95. In each figure, when the minimum value in the Inh group was larger than the maximum value in the LA and Def groups, it was indicated in the gray column; otherwise, it was indicated with ×. The minimum value of rSLs[X1:X2] in the Inh group tended to be larger than the maximum value of rSLs[X1:X2] in the LA and Def groups. However, this tendency did not appear when X2<40, and when As was large, this tendency did not appear unless the difference between X1 and X2 was large. These results indicated that rSLs[X1:X2] in the range of As = 5 to 95, X1 = 1 to 85, X2 = 25 to 100, and X2-X1 = 5 to 99 can be used for the estimation of Inh, but irSLs[X1:X2] in the range of X1 = 1 to 80, X2 = 55 to 100, and X2-X1 = 5 to 99 is preferred.

### 5) wXs[X1:X2]

As Ps, wXs[X1:X2], which is the weighted average of X in the interval from Ts(X1) to Ts(X2), was calculated for each specimen in accordance with the following equation (VIa). $wXs \left[X1:X2\right] = \frac{{\sum }_{X = X 1}^{X 2} \left\{X\times Ts\left(X\right)\right\}}{{\sum }_{X = X 1}^{X 2} Ts \left(X\right)} \times Qs$ (X1 = 1, X2 = 100, Qs = 1)

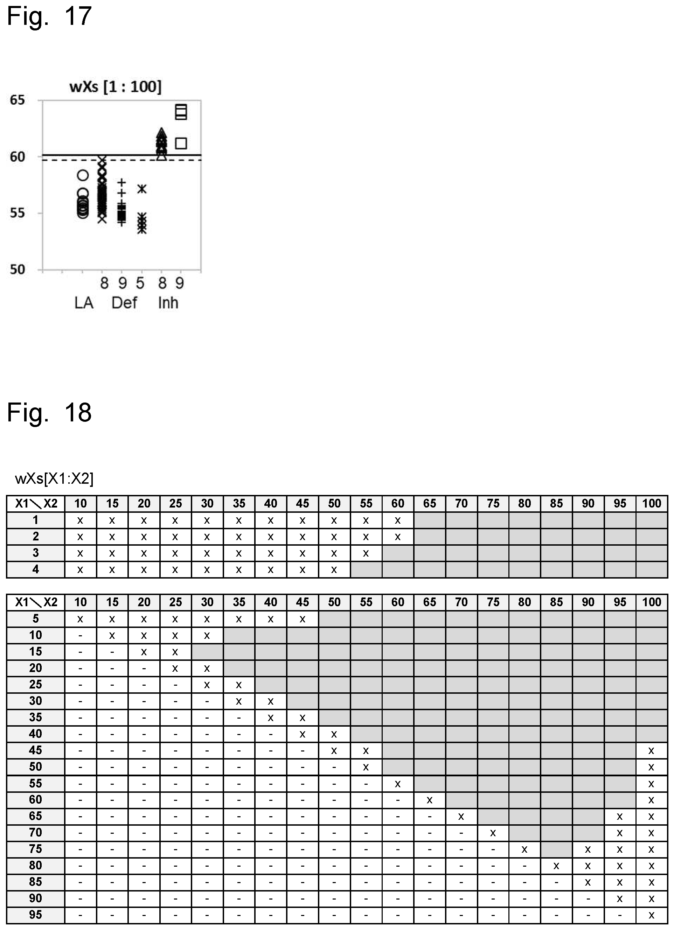

Fig. 17 illustrates the calculated wXs[X1:X2] of each specimen. In the figure, the solid line indicates the minimum value of wXs[X1:X2] in the Inh group, and the dashed line indicates the maximum value of wXs[X1:X2] in the LA and Def groups. An example in which wXs[X1:X2] for Inh was larger than that for LA and Def was confirmed. Therefore, it was indicated that a threshold value which can distinguish Inh from LA and Def can be set based on wXs[X1:X2], and that a specimen in which wXs[X1:X2] is larger than the threshold value can be estimated to be Inh.

Fig. 18 illustrates the difference between the minimum value of wXs[X1:X2] in the Inh group and the maximum value of wXs[X1:X2] in the LA and Def groups when X1 and X2 were changed in various ways. In the figure, when the minimum value in the Inh group was larger than the maximum value in the LA and Def groups, it was indicated in the gray column; otherwise, it was indicated with ×. The conditions in which the minimum value of wXs[X1:X2] in the Inh group was larger than the maximum value of wXs[X1:X2] in the LA and Def groups were confirmed. These results indicated that wXs[X1:X2] in the range of X1 = 1 to 75, X2 = 30 to 100, and X2-X1 = 10 to 99 is preferred for the estimation of Inh.

### 6) Ratio of vTs(X)

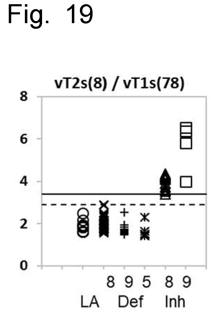

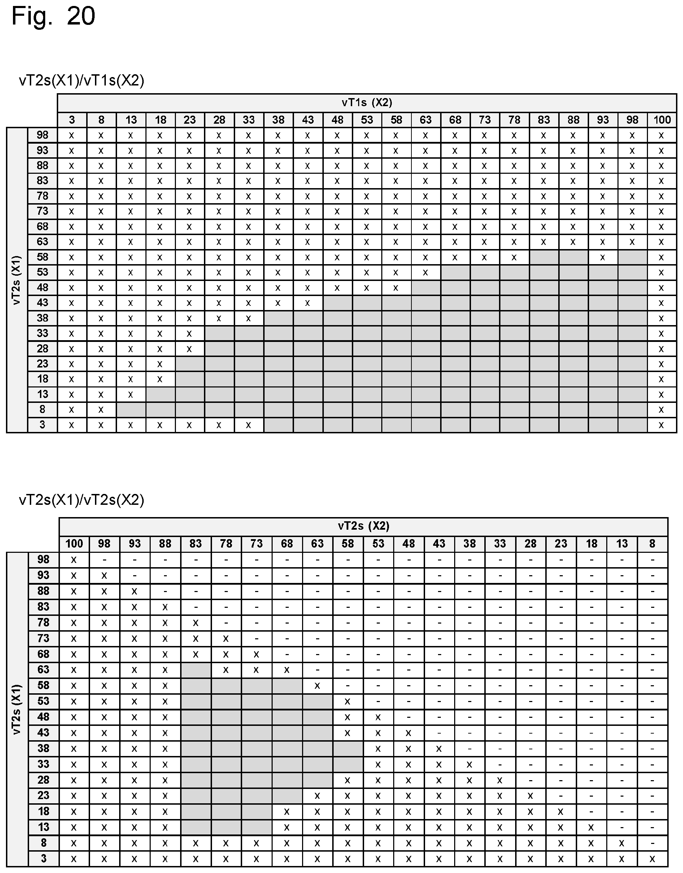

Fig. 19 illustrates an example of vT2s(X1)/vT1s(X2) (X1 = 8 and X2 = 78) for each specimen. In the figure, the solid line indicates the minimum value in the Inh group, and the dashed line indicates the maximum value in the LA and Def groups. The minimum value for Inh was larger than the maximum value for LA and Def. Fig. 20 illustrates the difference between the minimum value in the Inh group and the maximum value in the LA and Def groups for vT2s(X1)/vT1s(X2) and vT2s(X1)/vT2s(X2) when X1 and X2 were changed in various ways. In the figure, when the minimum value in the Inh group was larger than the maximum value in the LA and Def groups, it was indicated in the gray column; otherwise, it was indicated with ×. These results indicated that vT2s(X1)/vT1s(X2) in the range of X1 = 3 to 58 and X2 = 13 to 98, and vT2s(X1)/vT2s(X2) in the range of X1 = 13 to 63 and X2 = 58 to 83 (provided that X1<X2) are preferred for the estimation of Inh Ps.

### 7) Relative value of difference in vTs(X)

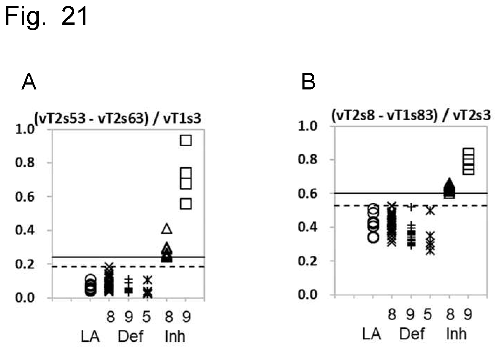

Fig. 21 illustrates the relative value of the difference in vTs(X) for each specimen. Fig. 21A illustrates (vT2s(X1)-vT2s(X2))/vT1s(Bs) (X1 = 53 and X2 = 63, Bs = 3). Fig. 21B illustrates (vT2s(X1)-vT1s(X2))/vT2s(Bs) (X1 = 8 and X2 = 83, Bs = 3) of each specimen. In each figure, the solid line indicates the minimum value in the Inh group, and the dashed line indicates the maximum value in the LA and Def groups. It was confirmed that the minimum value for Inh was larger than the maximum value for LA and Def.

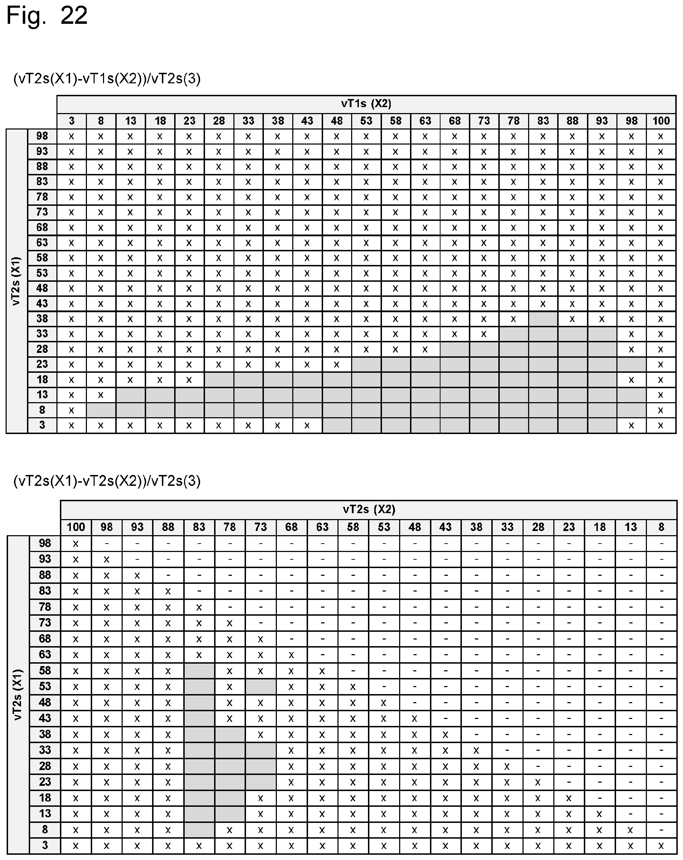

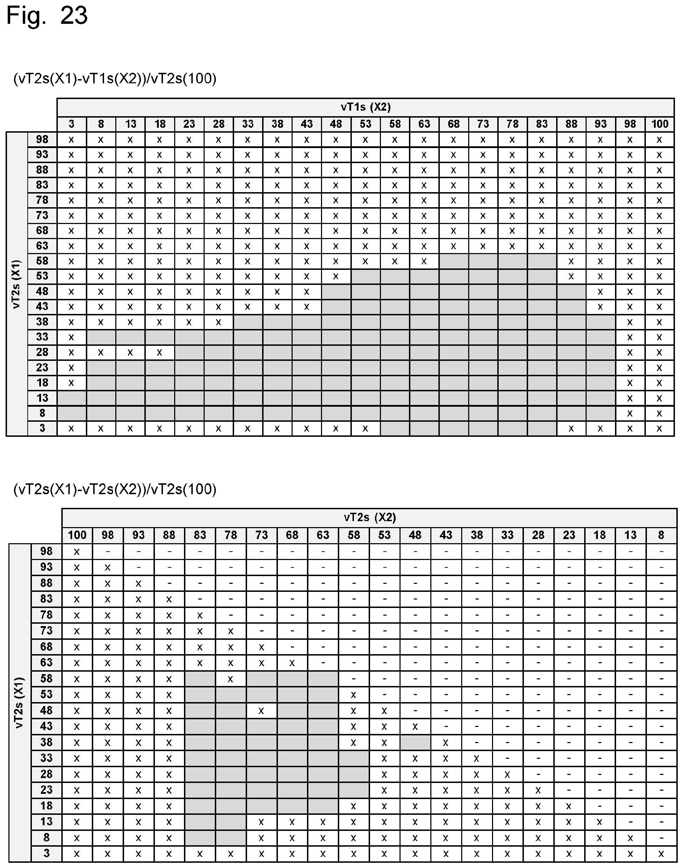

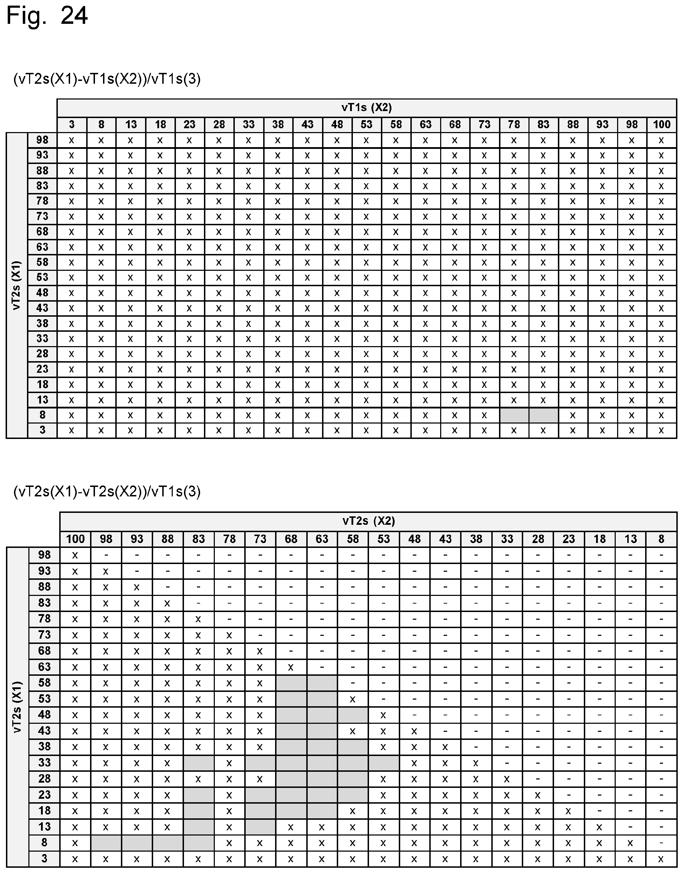

Fig. 22 illustrates the difference between the minimum value in the Inh group and the maximum value in the LA and Def groups for (vT2s(X1)-vT1s(X2))/vT2s(Bs) and (vT2s(X1)-vT2s(X2))/vT2s(Bs) (Bs = 3). Fig. 23 illustrates the difference between the minimum value in the Inh group and the maximum value in the LA and Def groups for (vT2s(X1)-vT1s(X2))/vT2s(Bs) and (vT2s(X1)-vT2s(X2))/vT2s(Bs) (Bs = 100). Fig. 24 illustrates the difference between the minimum value in the Inh group and the maximum value in the LA and Def groups for (vT2s(X1)-vT1s(X2))/vT1s(Bs) and (vT2s(X1)-vT2s(X2))/vT1s(Bs) (Bs = 3). In Figs. 22 to 24, when the minimum value in the Inh group was larger than the maximum value in the LA and Def groups, it was indicated in the gray column; otherwise, it was indicated with ×. These results indicated that the relative value of the difference in vTs(X) is preferred as Ps for the estimation of Inh.

### 4. Estimation of LA and Def based on Pm

### 1) Tm(X1)

As Pm, Tm(X1) (X1 = 1, 25, 50, 75, or 100) of each specimen was calculated in accordance with the following equation.

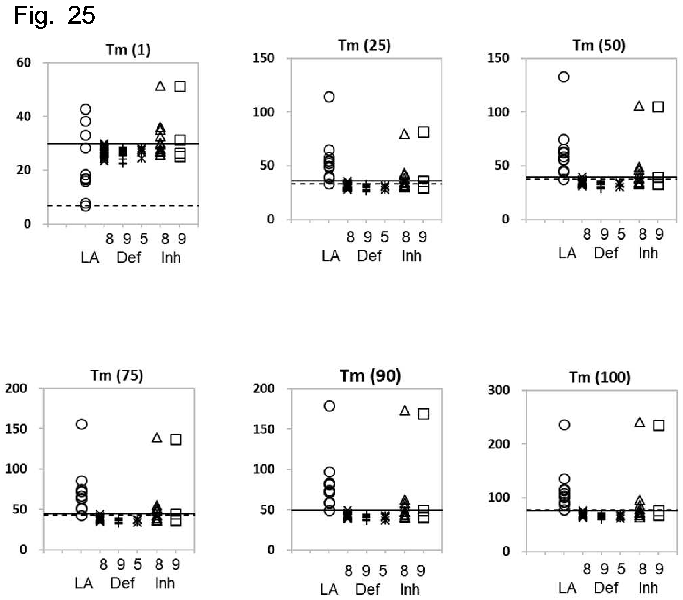

Fig. 25 illustrates Tm(X1) of each specimen. In the figure, the solid line indicates the maximum value of Tm(X1) in the Def group, and the dashed line indicates the minimum value of Tm(X1) in the LA group. Tm(X1) for LA tended to be larger than that for Def. Therefore, it was indicated that a threshold value which can distinguish between LA and Def can be set based on Tm(X1), and that from the subject specimens except for Inh, specimens in which Tm(X1) is larger than the threshold value can be estimated to be LA. As Pm for the estimation of LA, Tm(X1) is preferably in the range of X1 = 90 to 100.

### 2) rTm(X1)

As Pm, rTm(X1) of each specimen was calculated in accordance with the following equation (IIb'). $rTm \left(X1\right) = Tm \left(X1\right) / Tm \left(Am\right) \times Qm$ (X1 = 90, Am = 3, Qm = 1)

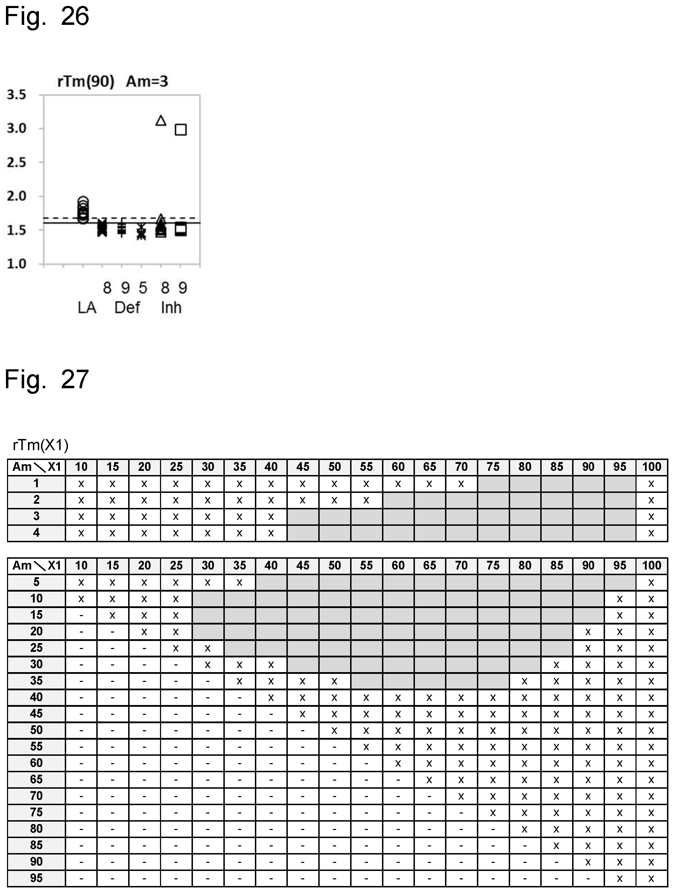

Fig. 26 illustrates the calculated rTm(X1) of each specimen. In the figure, the solid line indicates the maximum value of rTm(X1) in the Def group, and the dashed line indicates the minimum value of rTm(X1) in the LA group. An example in which rTm(X1) for LA was larger than that for Def was confirmed. Therefore, it was indicated that a threshold value which can distinguish between LA and Def can be set based on rTm(X1), and that from the subject specimens except for Inh, specimens in which rTm(X1) is larger than the threshold value can be estimated to be LA.

Fig. 27 illustrates the difference between the minimum value of rTm(X1) in the LA group and the maximum value of rTm(X1) in the Def group when X1 and Am (X1>Am) were changed in various ways. In the figure, when the minimum value in the LA group was larger than the maximum value in the Def group, it was indicated in the gray column; otherwise, it was indicated with ×. These results indicated that rTm(X1) in the range of Am = 1 to 35, X1 = 30 to 95, and X1-Am = 10 to 94 is preferred for the estimation of LA and Def.

### 3) SLm[X1:X2]

As Pm, SLm[X1:X2], which is the slope of the regression line of Tm(X), was calculated for each specimen in accordance with the following equation (Vb). $SLm \left[X1:X2\right] = \frac{{\sum }_{X = X 1}^{X 2} \left\{\left(X-UX\right)\times \left(Tm\left(X\right)-UTm\right)\right\}}{{\sum }_{X = X 1}^{X 2} {\left(X-UX\right)}^{2}} \times Qm$

However, UX is an average of X from X1 to X2, and UTm is an average of Tm(X) from Tm(X1) to Tm(X2) .
(X1 = 5, X2 = 100, Qm = 1)

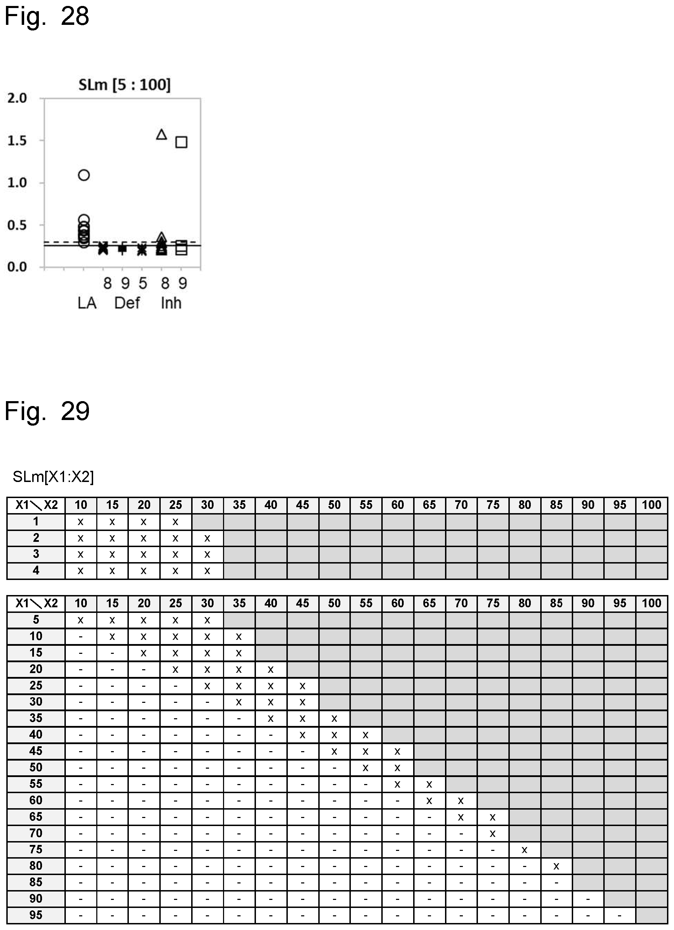

Fig. 28 illustrates the calculated SLm[X1:X2] of each specimen. In the figure, the solid line indicates the maximum value of SLm[X1:X2] in the Def group, and the dashed line indicates the minimum value of SLm[X1:X2] in the LA group. An example in which SLm[X1:X2] for LA was larger than that for Def was confirmed. Therefore, it was indicated that a threshold value which can distinguish between LA and Def can be set based on SLm[X1:X2], and that from the subject specimens except for Inh, specimens in which SLm[X1:X2] is larger than the threshold value can be estimated to be LA.

Fig. 29 illustrates the difference between the minimum value of SLm[X1:X2] in the LA group and the maximum value of SLm[X1:X2] in the Def group when X1 and X2 were changed in various ways. In the figure, when the minimum value in the LA group was larger than the maximum value in the Def group, it was indicated in the gray column; otherwise, it was indicated with ×. These results indicated that SLm[X1:X2] in the range of X1 = 1 to 95, X2 = 30 to 100, and X2-X1 = 5 to 99 is preferred for the estimation of LA and Def.

### 4) rSLm[X1:X2]

As Pm, rSLm[X1:X2], which is the slope of the regression line of rTm(X), was calculated for each specimen in accordance with the following equation (Vb'). $\begin{matrix}rSLm \left[X1:X2\right] \\ = \frac{{\sum }_{X = X 1}^{X 2} \left\{\left(X-UX\right)\times \left(rTm\left(X\right)-UrTm\right)\right\}}{{\sum }_{X = X 1}^{X 2} {\left(X-UX\right)}^{2}} \times Qm\end{matrix}$

However, rTm(X) is rTm(X) = Tm(X)/Tm(Am)×Qm, UX is an average of X from X1 to X2, and UrTm is an average of Tm(X) from rTm(X1) to rTm(X2).
(X1 = 20, X2 = 40, As = 5, 50, or 95, Qm = 1)

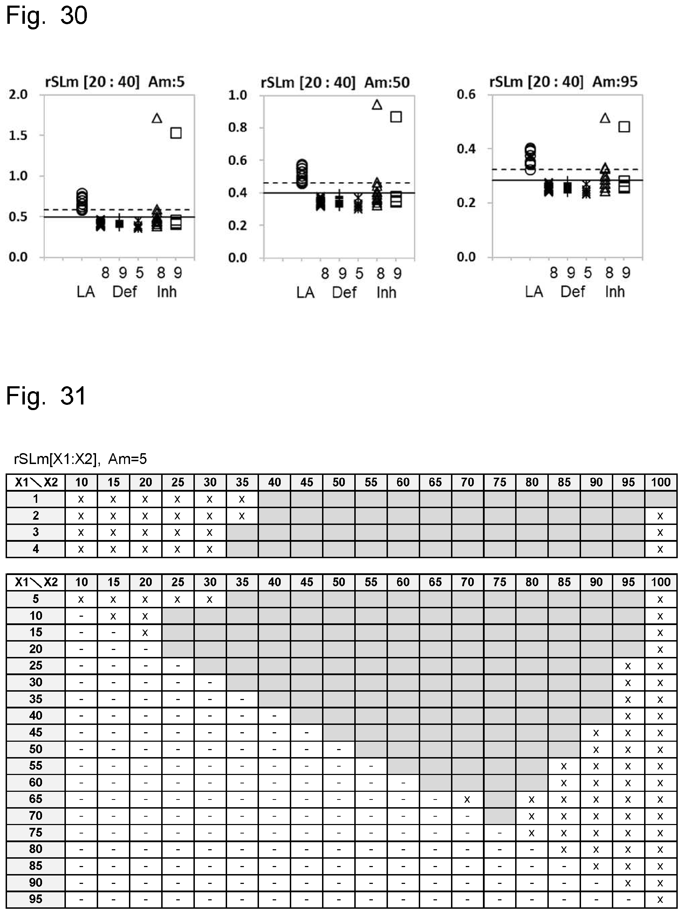

Fig. 30 illustrates the calculated rSLm[X1:X2] of each specimen. In the figure, the solid line indicates the maximum value of rSLm[X1:X2] in the Def group, and the dashed line indicates the minimum value of rSLm[X1:X2] in the LA group. An example in which rSLm[X1:X2] for LA was larger than that for Def was confirmed. Therefore, it was indicated that a threshold value which can distinguish between LA and Def can be set based on rSLm[X1:X2], and that from the subject specimens except for Inh, specimens in which rSLm[X1:X2] is larger than the threshold value can be estimated to be LA.

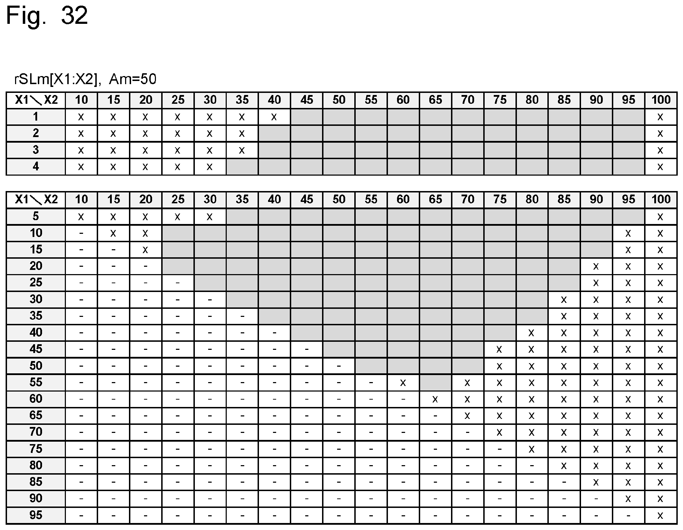

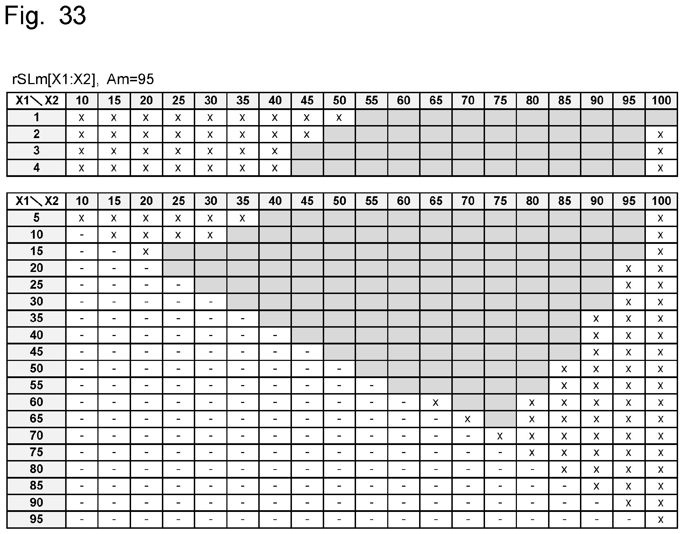

Figs. 31 to 33 each illustrate the difference between the minimum value of rSLm[X1:X2] in the LA group and the maximum value of rSLm[X1:X2] in the Def group when X1 and X2 were changed in various ways. In each figure, when the minimum value in the LA group was larger than the maximum value in the Def group, it was indicated in the gray column; otherwise, it was indicated with ×. These results indicated that rSLm[X1:X2] in the range of Am = 5 to 95, X1 = 1 to 70, X2 = 25 to 100, and X2-X1 = 5 to 99 can be used for the estimation of LA and Def, but rSLm[X1:X2] in the range of X1 = 1 to 55, X2 = 25 to 95, and X2-X1 = 5 to 94 is preferred.

### 5) Weighted average

As Pm, wXm[X1:X2], which is a weighted average of X in the interval from Tm(X1) to Tm(X2), was calculated for each specimen in accordance with the following equation (VIb). Similarly, the weighted average wTm[X1:X2] of Tm(X) in the interval from X1 to X2 was calculated for each specimen in accordance with the following equation (VIIb). Similarly, the weighted average wrTm[X1:X2] of rTm(X) in the interval from X1 to X2 was calculated for each specimen in accordance with the following equation (VIIb'). $wXm \left[X1:X2\right] = \frac{{\sum }_{X = X 1}^{X 2} \left\{X\times Tm\left(X\right)\right\}}{{\sum }_{X = X 1}^{X 2} Tm \left(X\right)} \times Qm$ (X1=1, X2=95, Qm=1) $wTm \left[X1:X2\right] = \frac{{\sum }_{X = X 1}^{X 2} \left\{X\times Tm\left(X\right)\right\}}{{\sum }_{X = X 1}^{X 2} X} \times Qm$ (X1=95, X2=100, Qm=1) $\begin{matrix}wrTm \left[X1:X2\right] = \frac{{\sum }_{X = X 1}^{X 2} \left\{X\times rTm\left(X\right)\right\}}{{\sum }_{X = X 1}^{X 2} X} \\ = \frac{{\sum }_{X = X 1}^{X 2} \left\{X\times Tm\left(X\right)/Tm\left(Am\right)\times Qm\right\}}{{\sum }_{X = X 1}^{X 2} X}\end{matrix}$ (X1=65, X2=80, Am=3, 10, or 34)

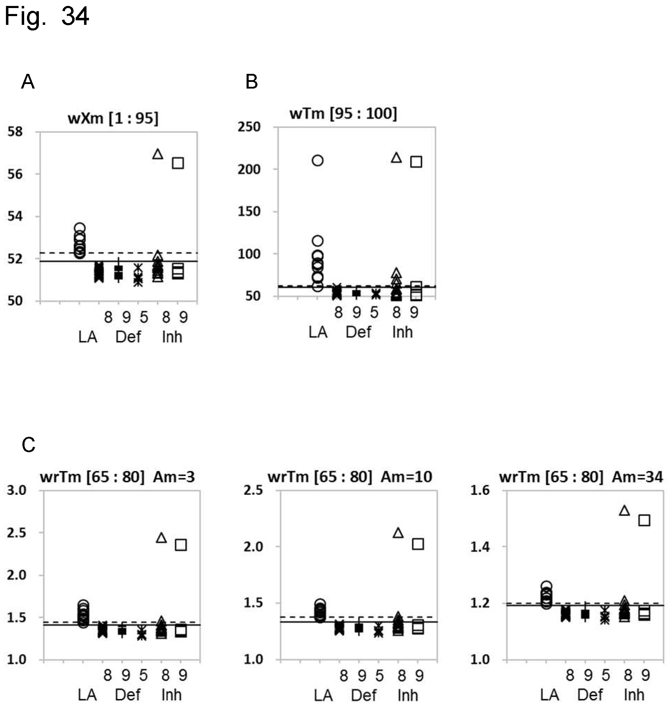

Figs. 34A to C illustrate the calculated wXm[X1:X2], wTm[X1:X2], and wrTm[X1:X2] of each specimen. In each figure, the solid line indicates the maximum value in the Def group, and the dashed line indicates the minimum value in the LA group. The parameters for LA were larger than those for Def. On the other hand, Inh could not be distinguished from LA or Def based on these parameters. Therefore, it was indicated that threshold values which can distinguish between LA and Def can be set based on wXm[X1:X2], wTm[X1:X2], and wrTm[X1:X2], and that from the subject specimens except for Inh, specimens in which these parameters are larger than the threshold values can be estimated to be LA.

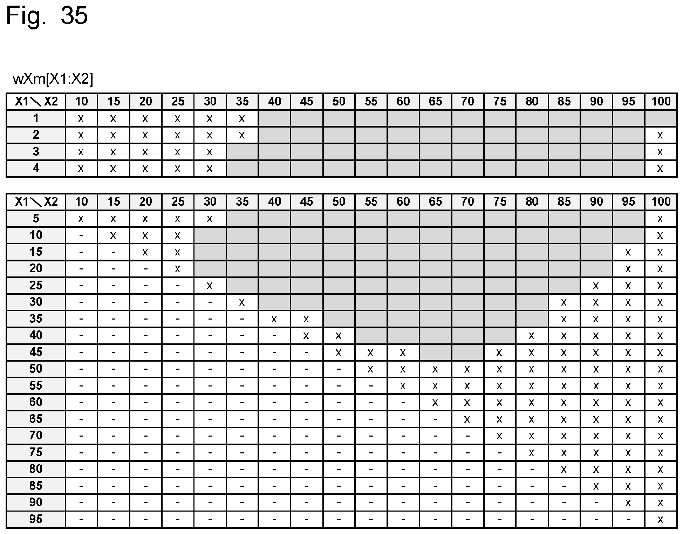

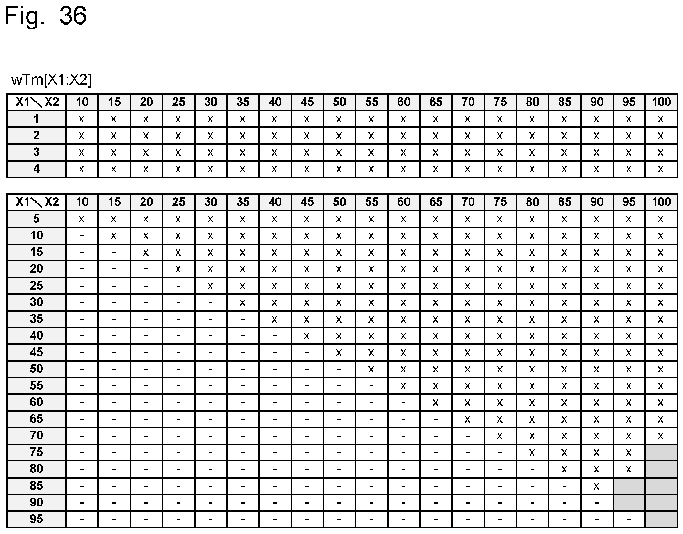

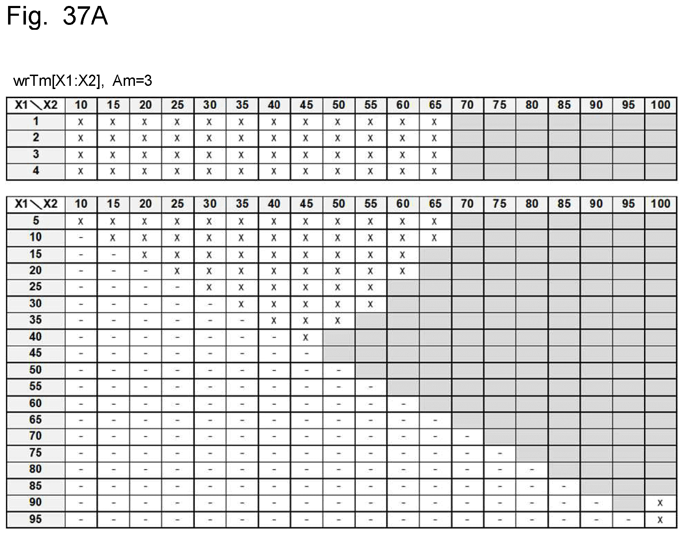

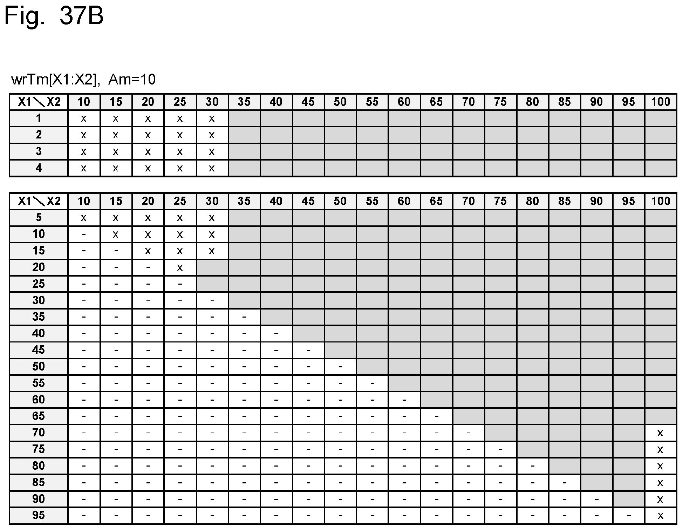

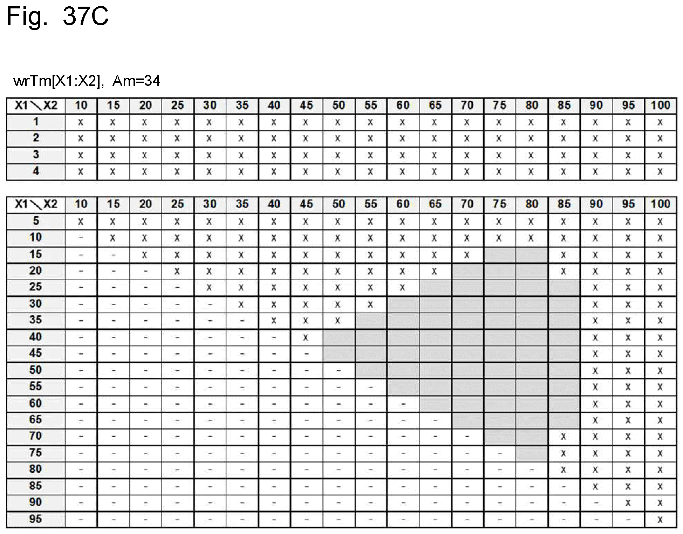

Figs. 35, 36, and 37A to C each illustrate the difference between the minimum values of wXm[X1:X2], wTm[X1:X2], and wrTm[X1:X2] in the LA group and the maximum values of the same parameters in the Def group when X1 and X2 were changed in various ways. In each figure, when the minimum value in the LA group was larger than the maximum value in the Def group, it was indicated in the gray column; otherwise, it was indicated with ×. These results indicated that wXm[X1:X2] in the range of X1 = 1 to 45, X2 = 30 to 100, and X2-X1 = 10 to 99 is preferred for the estimation of LA and Def. It was also indicated that the range of wTm[X1:X2] for the estimation of LA and Def is preferably X1 = 75 to 95, X2 = 95 to 100, and X2-X1 = 5 to 25. It was also indicated that the range of wrTm[X1:X2] for the estimation of LA and Def is preferably X1 = 1 to 90, X2 = 30 to 100, and X2-X1 = 5 to 99, and more preferably X1 = 15 to 75, X2 = 50 to 85, X2-X1 = 5 to 65, and Am = 3 to 34.

### 6) aTm(X1,X2)

As Pm, aTm(X1,X2) was calculated in accordance with the following equation (IVb). $\begin{matrix}aTm \left(X1,X2\right) \\ = \left\{Tm\left(X2\right)-Tm\left(X1\right)\right\} / \left(X2-X1\right)\end{matrix}$ (X1 = 85, X2 = 90)

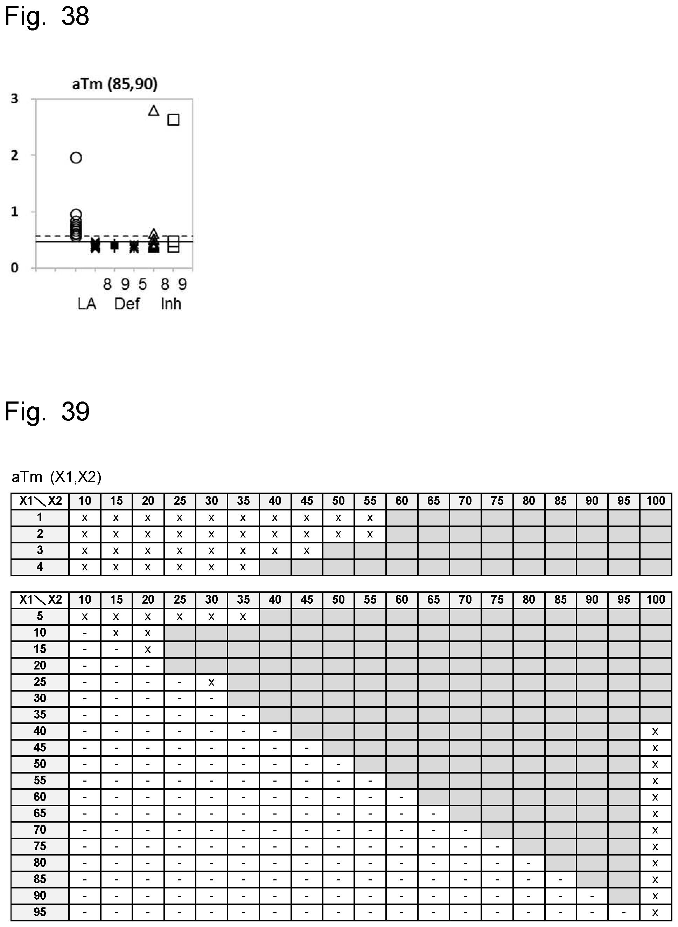

Fig. 38 illustrates the calculated aTm(X1,X2) of each specimen. In the figure, the solid line indicates the maximum value of aTm(X1,X2) in the Def group, and the dashed line indicates the minimum value of aTm(X1,X2) in the LA group. An example in which aTm(X1,X2) for LA was larger than that for Def was confirmed. On the other hand, Inh could not be distinguished from LA or Def based on aTm(X1,X2). Therefore, it was indicated that a threshold value which can distinguish between LA and Def can be set based on aTm(X1,X2), and that from the subject specimens except for Inh, specimens in which aTm(X1,X2) is larger than the threshold value can be estimated to be LA.

Fig. 39 illustrates the difference between the minimum value of aTm(X1,X2) in the LA group and the maximum value thereof in the Def group when X1 and X2 were changed in various ways. In the figure, when the minimum value in the LA group was larger than the maximum value in the Def group, it was indicated in the gray column; otherwise, it was indicated with ×. These results indicated that aTm(X1,X2) in the range of X1 = 1 to 90, X2 = 25 to 100, and X2-X1 = 5 to 99 is preferred for the estimation of LA and Def.

### 7) vTm(X)

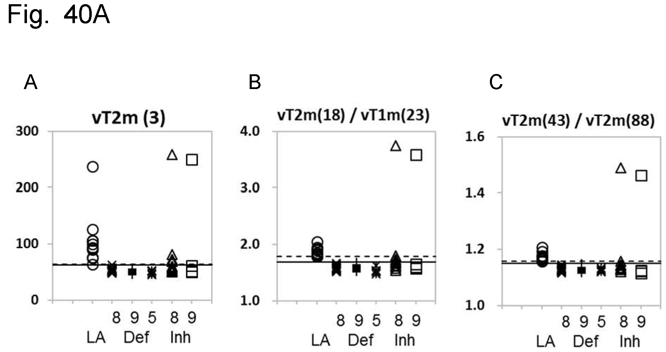

A to C in Figs. 40A illustrate vT2m(X1) (X1 = 3) of each specimen as an example of vTm(X), and vT2m(X1)/vT1m(X2) (X1 = 18, X2 = 23) and vT2m(X1)/vT2m(X22) (X1 = 43, X2 = 88) as examples of the vTm ratio. In each figure, the solid line indicates the maximum value in the Def group, and the dashed line indicates the minimum value in the LA group. The values for LA were larger than those for Def. Therefore, it was suggested that from the subject specimens except for Inh, LA and Def can be estimated based on vTm(X) or its ratio. The preferred range of vT2m(X1) was X1 = 3 to 18.

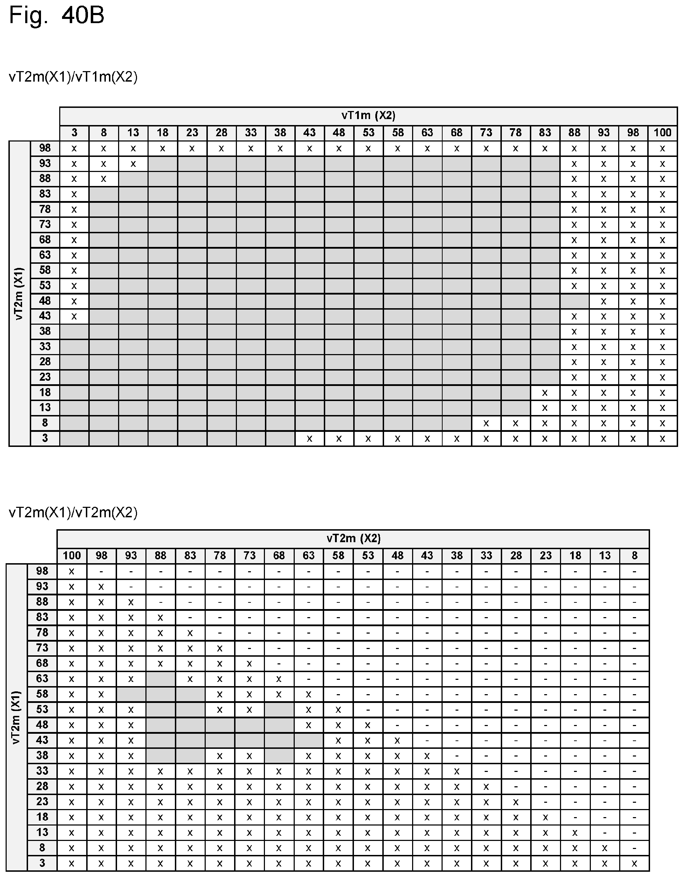

Fig. 40B illustrates the results of examining the vTm ratio when changing X1 and X2 in various ways. In the figure, when the minimum value in the LA group was larger than the maximum value in the Def group, it was indicated in the gray column; otherwise, it was indicated with ×. The preferred range of vT2m(X1)/vT1m(X2) was X1 = 3 to 93, X2 = 3 to 88, and the preferred range of vT2m(X1)/vT2m(X2) was X1 = 38 to 63 and X2 = 63 to 93. Therefore, it was indicated that the vTm ratio can be used for the estimation of LA and Def.

### 8) Relative value of difference in vTm(X)

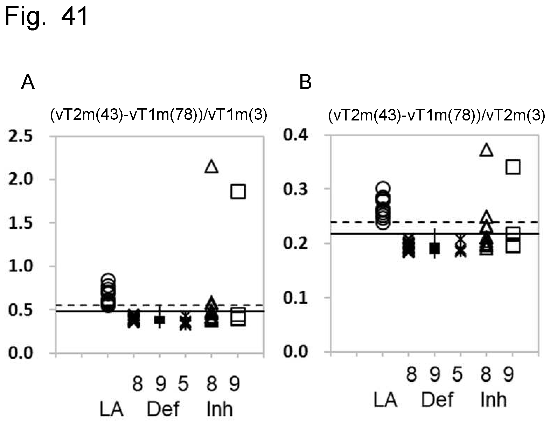

Fig. 41A illustrates (vT2m(X1)-vT1m(X2))/vT1m(Bm) (X1 = 43 and X2 = 78, Bm = 3) of each specimen, and Fig. 41B illustrates (vT2m(X1)-vT1m(X2))/vT2m(Bm) (X1 = 43 and X2 = 78, Bm = 3) of each specimen. In each figure, the solid line indicates the maximum value in the Def group, and the dashed line indicates the minimum value in the LA group. The values for LA were larger than those for Def.

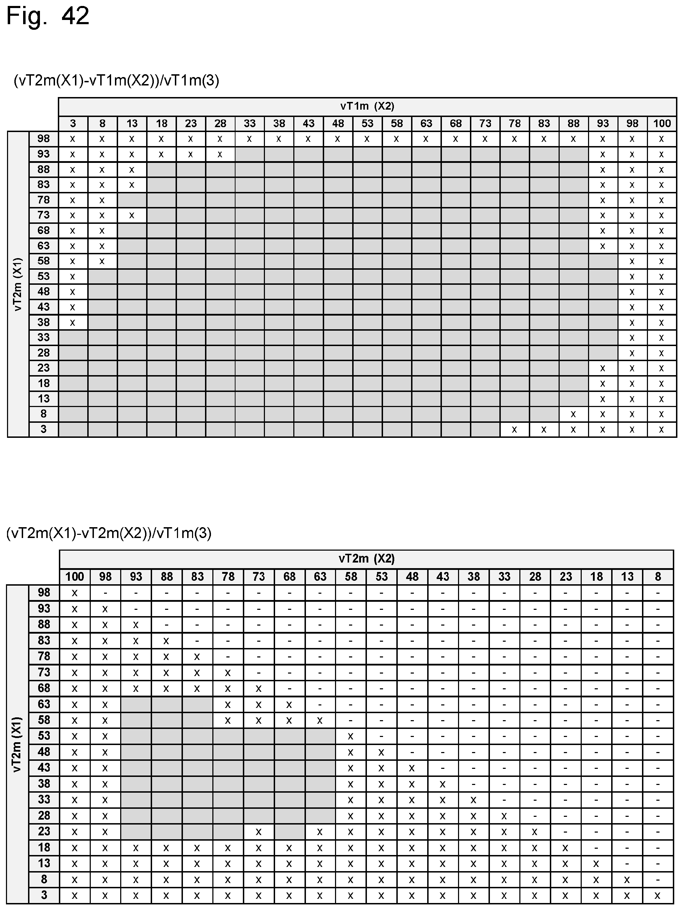

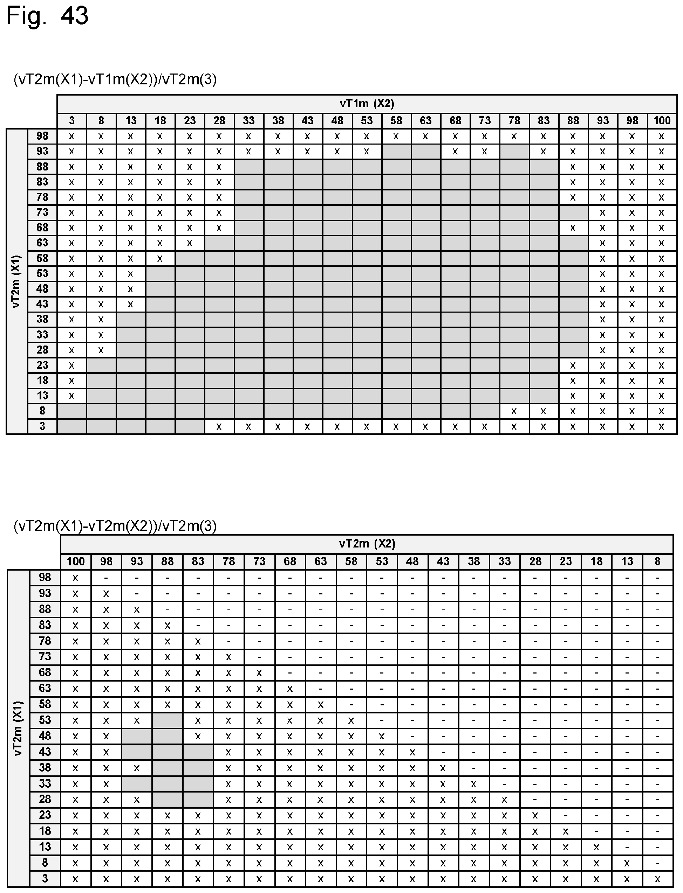

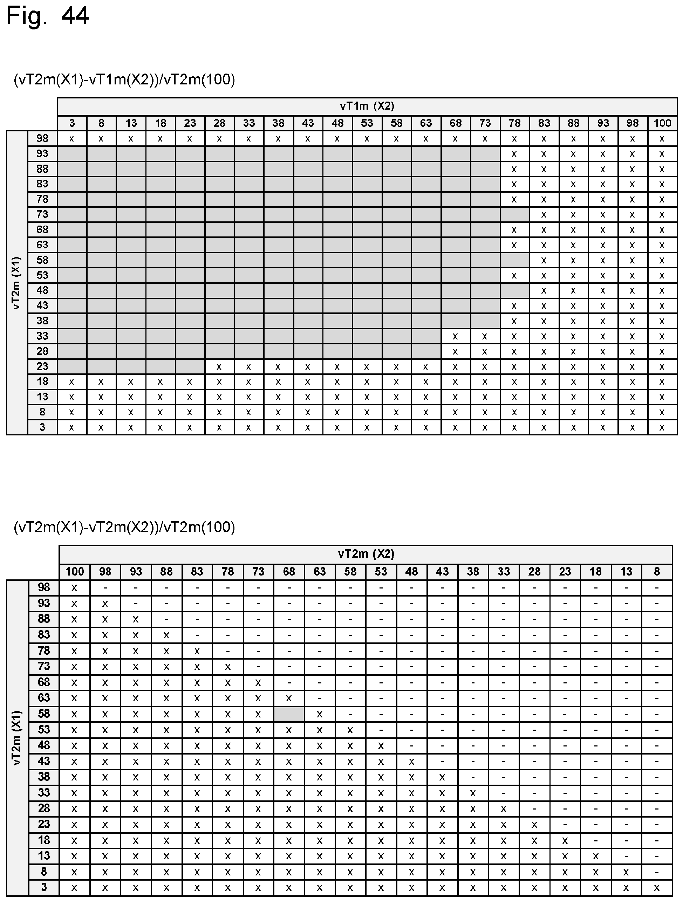

Figs. 42 and 43 each illustrate the difference between the minimum value in the LA group and the maximum value in the Def group for the relative value of the difference in vTm(X). In each figure, when the minimum value in the LA group was larger than the maximum value in the Def group, it was indicated in the gray column; otherwise, it was indicated with ×. Fig. 42 illustrates the results for (vT2m(X1)-vT1m(X2))/vT1m(Bm) and (vT2m(X1)-vT2m(X2))/vT1m(Bm), and Fig. 43 illustrates the results for (vT2m(X1)-vT1m(X2))/vT2m(Bm) and (vT2m(X1)-vT2m(X2))/vT2m(Bm). Fig. 44 illustrates the results when Bm = 100. These results indicated that the relative value of the difference in vTm(X) was preferred for the estimation of LA or Def. These results also indicated that Bm = 3 to 100, X1 = 3 to 93, and X2 = 3 to 93 were preferred for (vT2m(X1)-vT1m(X2))/vT1m(Bm). It was indicated that Bm = 3 to 100, X1 = 3 to 93, and X2 = 3 to 88 were preferred for (vT2m(X1)-vT1m(X2))/vT2m(Bm). It was indicated that Bm = 3 to 100, X1 = 23 to 63, and X2 = 63 to 93 were preferred for (vT2m(X1)-vT2m(X2))/vT1m(Bm). It was indicated that Bm = 3 to 100, X1 = 28 to 63, and X2 = 68 to 93 were preferred for (vT2m(X1)-vT2m(X2))/vT2m(Bm).

### 9) Cm(i)

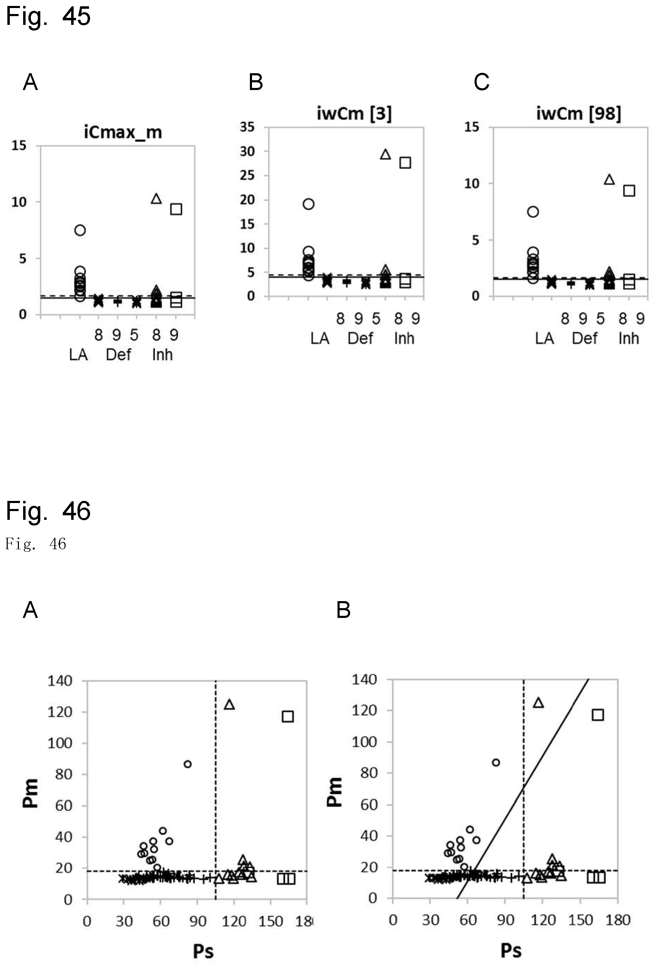

As correction parameters for Vm(i), (Q = 1) and Cm(i) were calculated in accordance with the above equation (XIb). The maximum value Cmax_m of Cm(i) was determined, and its reciprocal iCmax_m was calculated. Further, iwCm[X1:X2] was determined in accordance with the above equation (XIIb). In the equation (XIIb), X1 and X2 were points satisfying Cm(X1) = Cm(X2) = {maximum value of Cm(i)×S%} (S = 3 or 98, X1<VmaxT_m<X2). Figs. 45A, B, and C illustrate iCmax_m, and iwCm[X1:X2] when S = 3 and 98.

### 5. Estimation of cause of coagulation disorders using two-dimensional map

As shown in Figs. 46A and B, Pm of 1:1 (subject specimen:NPP) mixed specimens was used to plot (Ps, Pm) of each subject specimen on a Ps-Pm two-dimensional map with Ps on the X-axis and Pm on the Y-axis. Ps is rSLs[X1:X2] when As = 50, Pm is SLm[X1:X2], and X1 = 10 and X2 = 90 for both Ps and Pm. The plotted points of Inh, LA, and Def were distributed in different sections on the two-dimensional maps. In Figs. 46A and B, the dotted line parallel to the Y-axis indicates a threshold value Ls (the average value of Ps in the Def group + 3SD), and all the plotted points of Inh were located on the right side of this line (section HX). In Fig. 46A, the dotted line parallel to the X-axis indicates a threshold value Lm (the average value of Ps in the Def group + 4SD), and the distribution areas of the LA and Def plotted points were divided by this dotted line. In Fig. 46B, the solid line (diagonal line) indicates a threshold line Lc. The calculation procedure of the threshold line Lc (Y = aX+b) with Ps on the X-axis and Pm on the Y-axis is as follows: First, the slope SL and intercept IC of the regression line in the LA group were determined, and Py was determined (Py = SL×X+IC). Subsequently, the standard deviation SD of (Pm-Py) in the LA group was determined. With slope a = SL and intercept b = IC-3×SD, Lc:Y = aX+b was determined. The distribution areas of the LA and Def plotted points were divided by this threshold line Lc. It was indicated that plotting Ps and Pm of a subject specimen on a two-dimensional map allows visualization of whether the cause of coagulation disorders in the subject specimen is Inh, LA, or Def.

### 6. Comparative example

### 1) Coagulation time

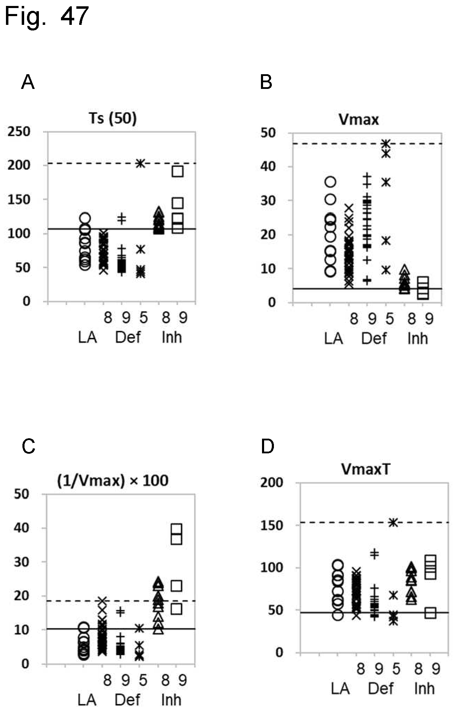

T(50) is a time point at which R(i) reaches 50% of E, and conventionally and generally calculated as a coagulation time. As shown in Fig. 47A, Inh, LA, and Def could not be distinguished based on Ts(50) of the specimen.

### 2) Parameter related to coagulation rate curve

The coagulation reaction curve R(i) of the specimen was differentiated to obtain a coagulation rate curve V(i). The maximum value (maximum reaction rate) Vmax of V(i), its reciprocal 1/Vmax, and time VmaxT at which V(i) reached Vmax were calculated. As shown in Figs. 47B to D, Inh, LA, and Def could not be distinguished based on any of Vmax, 1/Vmax, and VmaxT. For example, in the case of Vmax shown in Fig. 47B, Vmax tended to decrease in the order of LA, Def, and Inh; however, the distribution areas of the specimen types partially overlapped.

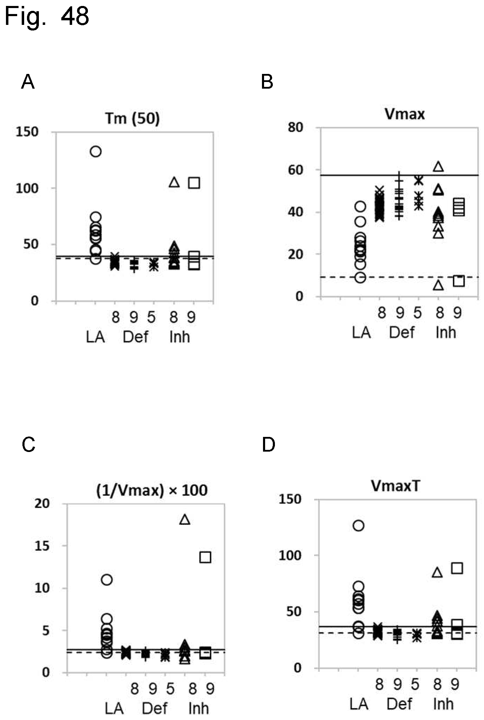

For the mixed specimen, Tm(50), Vmax, 1/Vmax, and VmaxT were similarly calculated. As shown in Figs. 48A to D, Inh, LA, and Def could not be distinguished based on any of Tm(50), Vmax, 1/Vmax, and VmaxT.

### 7. Effect of mixing ratio on mixed specimen

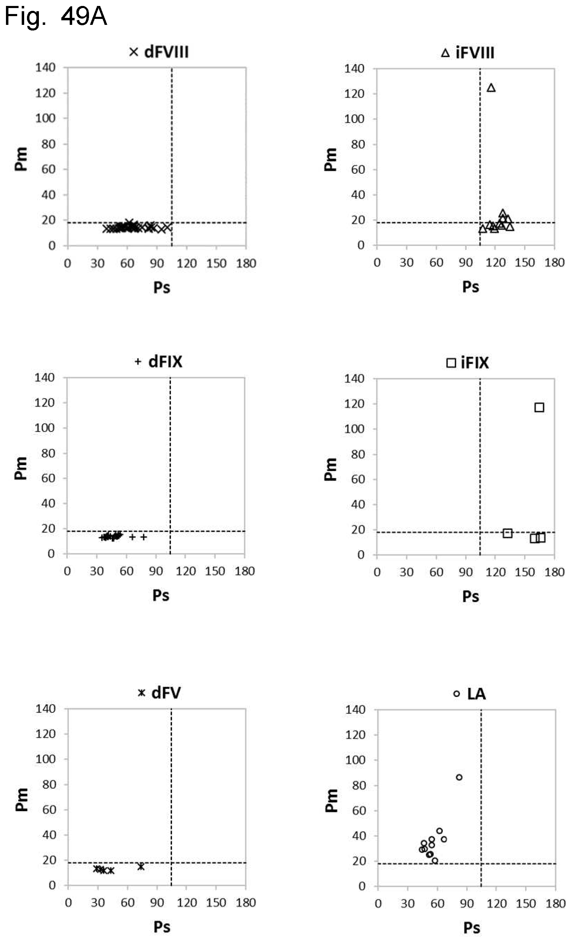

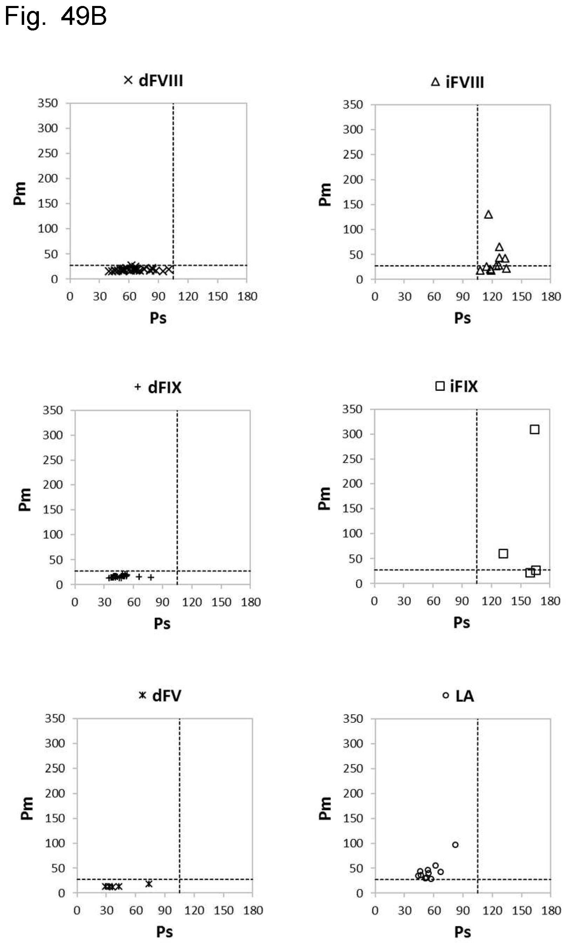

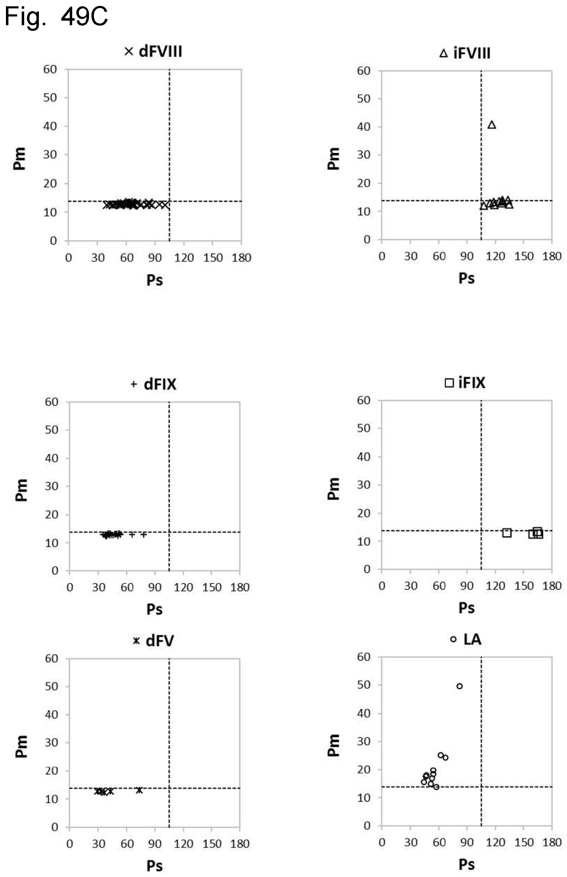

Mixed specimens were prepared in the same manner as in 5 described above, except that the mixing ratio of subject specimen and NPP was 1:9 or 9:1 (subject specimen:NPP), and Pm of the resulting mixed specimens was determined. Fig. 49 illustrates two-dimensional maps plotting (Ps, Pm) of each subject specimen by specimen type. Ps, Pm, Ls, and Lm are the same as those in Fig. 46A. Fig. 49Aillustrates Pm of a 1:1 (subject specimen:NPP) mixed specimen, Fig. 49B illustrates Pm of a 1:9 (subject specimen:NPP) mixed specimen, and Fig. 49C illustrates Pm of a 9:1 (subject specimen:NPP). In the figures, dFVIII, dFIX, and dFV indicate FVIII-deficient, FIX-deficient, and FV-deficient Def, respectively, and iFVIII and iFIX indicate FVIII inhibitor-positive and FIX inhibitor-positive Inh, respectively. The dotted line parallel to the Y-axis indicates a threshold value Ls, and the dotted line parallel to the X-axis indicates a threshold value Lm. Regardless of the mixing ratio of the mixed specimen, the distribution trends of Inh and Def on the two-dimensional map were almost the same, and Pm of Def was equal to or less than Lm. These results indicate that Pm enables the estimation of LA and Def, regardless of the mixing ratio of the mixed specimen.

### Example 2: Estimation of AHA

Acquired hemophilia A (AHA) is a special clinical condition in which VIII factor activity remains regardless of the appearance of VIII factor inhibitors. For this reason, the positions of (Ps, Pm) of specimens form AHA patients on a two-dimensional map was examined.

Ps and Pm were calculated for specimens derived from AHA patients (AHA; N = 6) in the same manner as in Example 1. Ps was {Ts(X2)-Ts(X1)}/Ts(As)×Qs of the equation (IIIa'). Pm was SLm[X1:X2] of the equation (Vb). Ps and Pm were also calculated similarly for the LA-positive specimens (LA; N = 11) used in Example 1. Mixed specimens with mixing ratios of 1:9, 1:1, and 9:1 (subject specimen:NPP) were used for the calculation of Pm. Fig. 50 illustrates two-dimensional maps plotting (Ps, Pm) of each subject specimen. In each figure, the dotted line parallel to the Y-axis indicates a threshold value Ls (Ls = M+3SD, wherein M is the average value of Ps in the Def group, and SD is the standard deviation), and the dotted line parallel to the X-axis indicates a threshold value Lm (Lm = M+K×SD, wherein M is the average value of Pm in the Def group, SD is the standard deviation, and K is 3, 3.5, or 4). Although Ps for AHA tended to be larger than that for LA, some the plotted points of AHA were located in a section estimated to be LA. This indicated the possibility that AHA could be misestimated to be LA in the estimation of the cause of coagulation disorders using Ps and Pm as in Example 1. The probability of such a misestimation is very low because the onset of AHA is very rare. However, considering that the treatment regimens for LA and AHA are quite different, such a misestimation poses a significant risk to AHA patients.

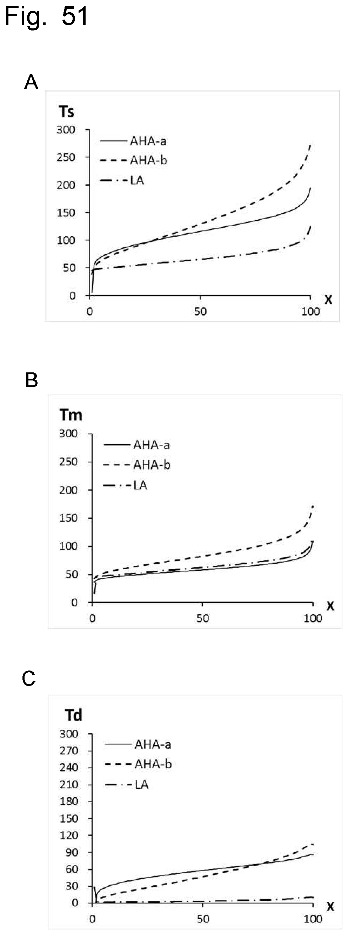

Fig. 51 illustrates Ts (Fig. A), Tm (Fig. B), and Td (Fig. C) of the following three specimens. AHA-a (solid line) is AHA whose (Ps, Pm) is located in the section LH, which is estimated to be LA, in the two-dimensional plot of Fig. 50. AHA-b (dashed line) is AHA whose Pm is about the same as that of AHA-a and whose Ps is larger. LA (chain line) is LA whose Ps is the closest to that of AHA-a. In Fig. 50, the reason why Ps for AHA-a is about the same as that in the LA group is that the slope of Ts for AHA-a is almost parallel to that of Ts for LA in Fig. 51A. Further, the reason why Pm for AHA-a is about the same as that for LA is that Tm for AHA-a is approximate to Tm for LA in Fig. 51B. On the other hand, as shown in Fig. 51C, Td for AHA-a and Td for AHA-b both increase with the increase in X, whereas Td for LA hovers around zero. Such differences in Td(X) between AHA and LA suggest that the AHA group including AHA-a whose (Ps, Pm) is located in the section LH, which is estimated to be LA, can be distinguished from the LA group.

Figs. 52 to 56 illustrate Pd of each specimen in the AHA group and LA group used in Example 1. Figs. 52A to C illustrate Td(X) when the mixing ratios (specimen S:NPP) of mixed specimens are 9:1 (A), 1:1 (B), and 1:9 (C), respectively. AHA-a (solid line) in Figs. 52A to C refers to AHA whose (Ps, Pm) is located in the section LH (i.e., minimum Ps), which is estimated to be LA, in the two-dimensional plots of Figs. 50A to C. AHA-b (dashed line) in Figs. 52A to C refers to AHA specimens whose Ps is the next (second) smallest after AHA-a on the two-dimensional plots of Figs. 50A to C. LA (chain line) in Figs. 52A to C refers to LA whose Ps is the closest to that of AHA-a in the two-dimensional plots of Figs. 50A to C. In all of Figs. 52A to C, Td(X) for AHA-a and AHA-b was larger than that for LA in the range where X exceeded about 40. These results indicated that Td(X) can be used as Pd in a predetermined interval of X.

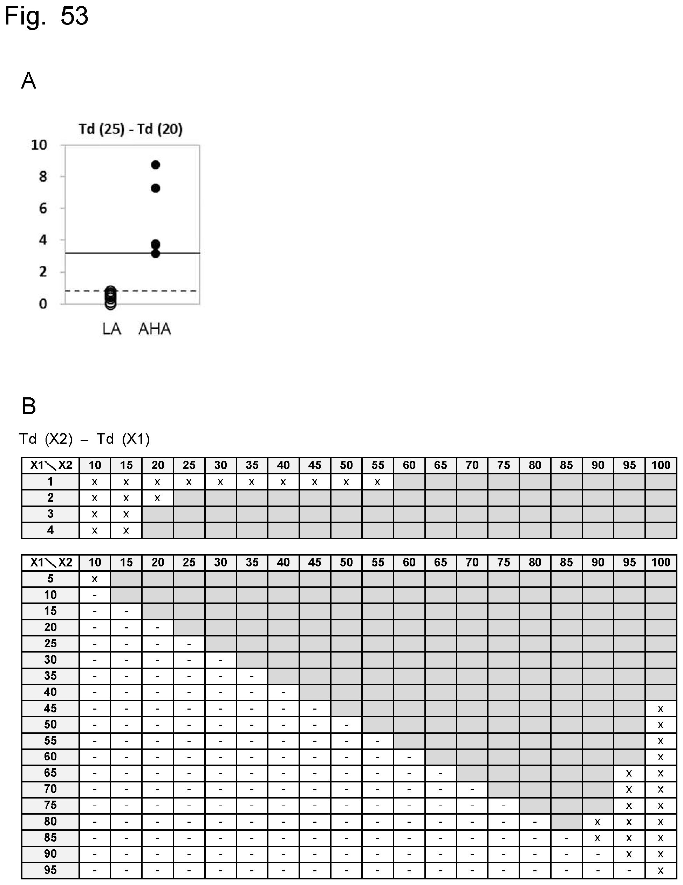

Figs. 53 to 56 illustrate Td(X2)-Td(X1), SLd[X1:X2], wXd[X1:X2], and wTd[X1:X2] of each specimen, respectively. In the tables of Figs. 53 to 56, the gray columns indicate that the minimum value in the AHA group is larger than the maximum value in the LA group; otherwise, it is indicated with ×.

Fig. 57 illustrates two-dimensional maps plotting (Pd, Pm) of each subject specimen for AHA and LA. In Figs. A, B, and C, the mixing ratios of mixed specimens (subject specimen:NPP) are 9:1, 1:1, and 1:9, respectively. Pd is SLd[X1:X2], Pm is SLm[X1:X2], and X1 = 10 and X2 = 90 were used for both Pd and Pm. In each figure, the dotted line parallel to the Y-axis indicates a threshold value Ld (Ld = Md+kd×SDd, wherein Md is the average value of Pd in the LA group, SDd is the standard deviation, and kd is the coefficient). Further, the dotted line parallel to the X-axis indicates a threshold value Lm (Lm = Mm+km×SDm, wherein Mm is the average value of Pm in the Def group, SDm is the standard deviation, and km is the coefficient). In Figs. A, B, and C, kd is 4, 4, and 3, respectively, and km is 3.5, 4, and 3, respectively. All the AHA specimens could be distinguished from LA based on the threshold values Ld and Lm. It was indicated that AHA latent in specimens estimated to be LA by the procedure of Example 1 can be distinguished from LA based on Pd.

### Example 3: Display of estimation results using index ratio

The specimens used were normal plasma of healthy persons (N = 10). The coagulation reaction measurement, acquisition of a coagulation reaction curve, detection of a coagulation reaction end point E, determination of APTT, and calculation of T(X) were performed in the same manner as in Example 1.

Calculation of index ratio $Index ratio = \left(\begin{matrix}Ps , Pm , or Pd of subject \\ specimen\end{matrix}\right) / \left(control index value\right)$ 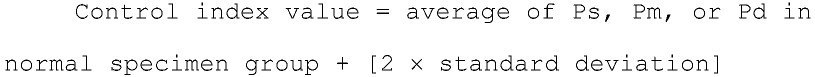 Control index value = average of Ps, Pm, or Pd in normal specimen group + [2 × standard deviation]

The same indices as in Example 1 were used for both Ps on the horizontal axis and Pm on the vertical axis to calculate index ratios, and a two-dimensional plot was created. Examples of units for index ratios include ratio and no unit. The use of index ratios made it possible to relatively show the size of Ps and Pm of the specimen S in comparison to those of the normal specimen group.

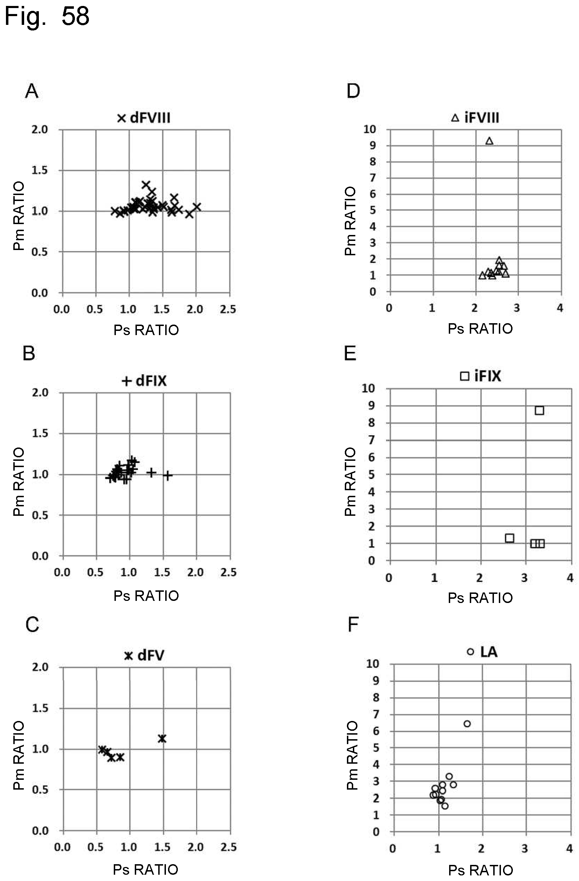

Fig. 58 illustrates two-dimensional maps created by converting the two-dimensional maps of Fig. 49A into index ratios (Ps ratio and Pm ratio). Control index values of the index ratios were calculated as average value + [2 × standard deviation] using each Ps and Pm in the normal plasma group (N = 10). Ps and Pm used are the same as those in Fig. 49A. Fig. 58 illustrates the distribution locations of all specimens on a single two-dimensional map by specimen type. Figs. A, B, and C show dFVIII, dFIX, and dFV of Def, respectively, Figs. D and E show iFVIII and iFIX of Inh, respectively, and Fig. F shows LA. The ranges of the Ps ratio for LA, Def, and Inh were from 0.9 to 1.6, from 0.6 to 2.0, and from 2.1 to 3.3, respectively, and the ranges of the Pm ratio were from 1.5. to 6.5, from 0.9 to 1.3, and from 1.0 to 9.3, respectively. From these results, it was possible to distinguish between Inh when the Ps ratio exceeded 2.0 and LA when the Pm ratio exceeded 1.3. In addition, it was possible to distinguish Def when the Ps ratio was 2.0 or less and Pm was 1.3 or less.

### 1) Relationship between Ps ratio and factor activity

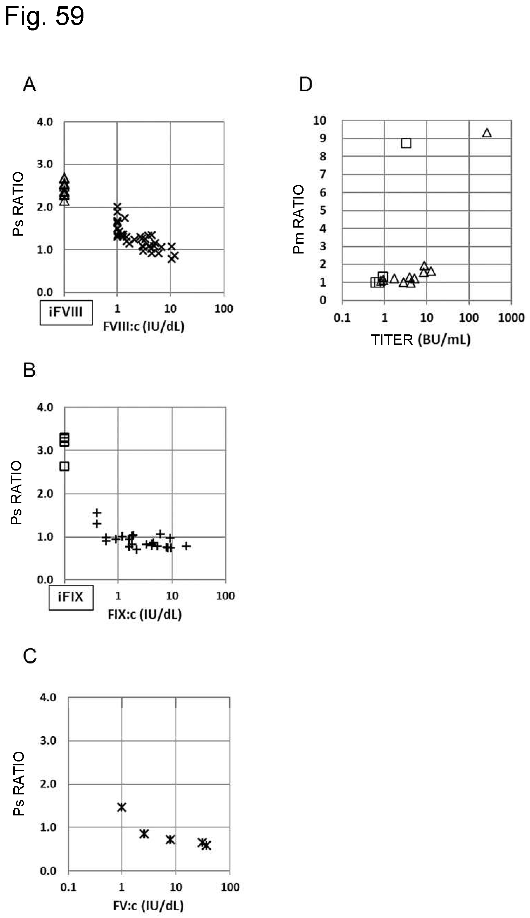

Fig. 59 illustrates the relationship between the Ps ratio and coagulation factor activity, with the factor activity (IU/dL) on the horizontal axis (logarithmic scale) and the Ps ratio on the vertical axis. The Ps ratio was calculated in the same manner as in Fig. 58. Fig. A shows dFVIII, Fig. B shows dFIX, and Fig. C shows dFV. In Figs. A and C, severe patient specimens with a factor activity of less than 1% are plotted on a scale of 1 on the horizontal axis. Further, in order to compare the relationship with coagulation factor inhibitors, Inh (iFVIII in Fig. A, iFIX in Fig. B) is plotted on a scale of 0.1 on the horizontal axis. Figs. 59A to C show that the Ps ratio is inversely related to the factor activity. It was revealed that specimens with a Ps ratio of larger than 2.0 can be distinguished as Inh, and that specimens with a Ps ratio of less than 2.0 can be distinguished as Def.

### 2) Relationship between Pm ratio and inhibitor titer

Fig. 59D illustrates the relationship between the Pm ratio and inhibitor titer for iFVIII (triangle) and iFIX (square), with the inhibitor titer (BU/mL) on the horizontal axis (logarithmic scale) and the Pm ratio on the vertical axis. The Pm ratio was calculated in the same manner as in Fig. 58. Fig. 59D indicates that for iFVIII, when the titer is higher than about 5, the Pm ratio correlates with the titer. This correlation suggests that the Pm ratio can be used to estimate the degree of titer because a specimen with a titer of 260 (Pm ratio: about 9) was covered.

### 3) Advantage of index ratios

The index ratios are considered to be a practical display method because it is expected that the effects of differences between reagent lots and differences between automated analyzers can be avoided by measuring the normal plasma group used for the control index value (denominator) using the same analyzer and the same reagent lot as the subject specimen (Specimen S).

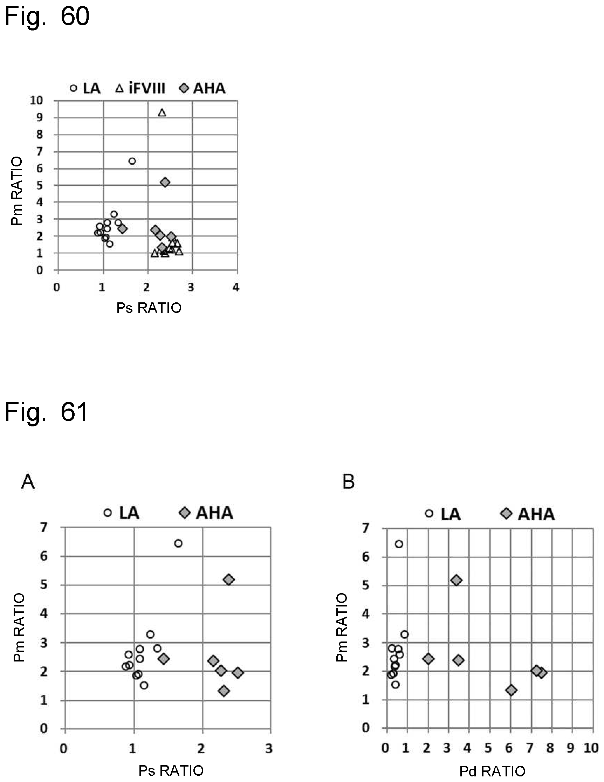

Fig. 60 illustrates the difference in the distribution location of each specimen type for LA (circle), iFVIII (triangle), and AHA (diamond) in a two-dimensional map with the Ps ratio and Pm ratio. Six AHA cases (Ps ratio and Pm ratio) were separated in the following three locations: three cases between the LA group and the iFVIII group; two cases close to the iFVIII group between the LA group and the iFVIII group; and one case close to the LA group between the LA group and the iFVIII group. The reason why all the AHA cases are distributed between the LA group and the iFVIII group can be explained by the fact that in AHA, VIII factor activity remains despite the presence of VIII factor inhibitors. That is, this can be explained by the fact that although the Ps ratio is smaller than that for iFVIII in which factor VIII activity has almost disappeared, the Pm ratio is larger because they are high-titer specimens with an inhibitor titer of larger than 5 BU/mL. The above results indicated that regarding the distribution locations (coordinates) of Ps and Pm on the two-dimensional map, Ps reflects the factor activity (its weakness), and Pm reflects the inhibition of the coagulation reaction (its strength).

### 4) Relationship between AHA specimens and index ratios

LA and AHA were used as specimen types to examine the positional relationship between the specimen types in a two-dimensional map using index ratios on the horizontal axis and vertical axis. Fig. 61 illustrates two-dimensional maps. In each figure, the circle represents LA, and the diamond represents AHA. The index ratios of the horizontal axis and vertical axis are the Ps ratio and Pm ratio in Fig. 61A, and the Pd ratio and Pm ratio in Fig. 61B. Ps is rSLs[X1:X2] when As = 50, Pm is SLm[X1:X2], Pd is SLd[X1:X2], and X1 = 10 and X2 = 90 were used for all of Ps, Pm, and Pd. The control index value used for the calculation of the Ps ratio and Pm ratio was average in normal specimen group + [2 × standard deviation], and the control index value used for the calculation of the Pd ratio was average in LA group + [3 × standard deviation]. Further, the mixing ratio of mixed specimens (subject specimen:NPP) was 1:1. In the AHA group, the Pd ratio of a specimen with the lowest Ps ratio (1.4) was 2.0. This specimen was AHA-a whose (Ps, Pm) on the two-dimensional plot was located in the section LH, which was estimated to be LA. On the other hand, in the LA group, the Pd ratio of a specimen with the maximum value of Ps ratio (1.6) was 0.6. These results indicated that AHA-a whose (Ps ratio, Pm ratio) on the two-dimensional plot is located in the section LH, which is estimated to be LA, can be distinguished from LA based on Pd. For example, when the threshold value Ld of the Pd ratio is 1.5, if the Pd ratio of a specimen whose (Ps, Pm) on the two-dimensional plot is located in the section LH, which is estimated to be LA, is larger than Ld, it can be estimated that this specimen is not LA or is suspected to be AHA.

## Claims

1. A method for estimating a cause of coagulation disorders in a blood specimen, the method comprising:
1) calculating a parameter Ps related to a coagulation reaction of a specimen S, and a parameter Pm related to a coagulation reaction of a specimen M,
wherein
the specimen S is a subject blood specimen with a prolonged coagulation time,
the specimen M is a mixed specimen of the specimen S and a specimen N,
the specimen N is a normal blood specimen,
Ps is calculated based on Ts(X), or vT1s(X) and vT2s(X), and
Pm is calculated based on Tm(X), vT2m(X), vT1m(X), and vT2m(X), or based on a first derivative curve of a coagulation reaction curve of the specimen M,
wherein
Ts(X) represents a measurement point or time at which a coagulation reaction curve of the specimen S reaches X% of Es, and Es is a coagulation reaction end point in the coagulation reaction curve of the specimen S,
vT1s(X) represents a measurement point or time at which a coagulation rate curve of the specimen S reaches X% of a maximum value before reaching the maximum value,
vT2s(X) represents a measurement point or time at which the coagulation rate curve of the specimen S reaches X% of a maximum value after reaching the maximum value,
Tm(X) represents a measurement point or time at which a coagulation reaction curve of the specimen M reaches X% of Em, and Em is a coagulation reaction end point in the coagulation reaction curve of the specimen M,
vT1m(X) represents a measurement point or time at which a coagulation rate curve of the specimen M reaches X% of a maximum value before reaching the maximum value,
vT2m(X) represents a measurement point or time at which the coagulation rate curve of the specimen M reaches X% of a maximum value after reaching the maximum value, and $0 < X \leq 100 ;$
2) estimating whether the specimen S is coagulation factor inhibitor-positive based on Ps; and
3) estimating that the specimen S which has not been estimated to be coagulation factor inhibitor-positive in 2) is lupus anticoagulant-positive or coagulation factor-deficient based on Pm or based on Ps and Pm.

2. The method according to claim 1, wherein Ps is selected from the group consisting of the following (a) to (j):
(a) rTs(X1)
(wherein $rTs \left(X1\right) = Ts \left(X1\right) / Ts \left(As\right) \times Qs$ X1 = 35 to 95, As = 3 to 75, X1>As, and Qs>0);
(b) $\left\{rTs\left(X2\right)-rTs\left(X1\right)\right\} / \left(X2-X1\right) ,$ (wherein rTs(X1) and rTs(X2) are defined in accordance with the above equation (IIa'), provided that X1 = 1 to 85, X2 = 25 to 95, (X2-X1) = 5 to 94, As = 5 to 95, and Qs>0);
(c) rSLs[X1:X2]
(wherein $rSLs \left[X1:X2\right] = \frac{{\sum }_{X = X 1}^{X 2} \left\{\left(X-UX\right)\times \left(rTs\left(X\right)-UrTs\right)\right\}}{{\sum }_{X = X 1}^{X 2} {\left(X-UX\right)}^{2}} \times Qs$
UX is an average from X1 to X2,
UrTs is an average from rTs(X1) to rTs(X2),
rTs(X2 is defined in accordance with the above equation (IIa'),
X1 = 1 to 85, X2 = 25 to 100, (X2-X1) = 5 to 99, As = 5 to 95, and Qs>0);
(d) wXs[X1:X2]
(wherein $wXs \left[X1:X2\right] = \frac{{\sum }_{X = X 1}^{X 2} \left\{X\times Ts\left(X\right)\right\}}{{\sum }_{X = X 1}^{X 2} Ts \left(X\right)} \times Qs$ X1 = 1 to 75, X2 = 30 to 100, (X2-X1) = 10 to 99, and Qs>0);
(e) $vT 2 s \left(X1\right) / vT 1 s \left(X2\right)$ (wherein X1 = 3 to 58 and X2 = 13 to 98);
(f) $vT 2 s \left(X1\right) / vT 2 s \left(X2\right)$ (wherein X1 = 13 to 63, X2 = 58 to 83, and X2>X1);
(g) $\left(vT2s\left(X1\right)-vT1s\left(X2\right)\right) / vT 2 s \left(Bs\right)$ (wherein X1 = 3 to 58, X2 = 13 to 98, and Bs = 3 to 100);
(h) $\left(vT2s\left(X1\right)-vT1s\left(X2\right)\right) / vT 1 s \left(Bs\right)$ (wherein X1 = 3 to 58, X2 = 3 to 93, and Bs = 3 to 100);
(i) $\left(vT2s\left(X1\right)-vT2s\left(X2\right)\right) / vT 2 s \left(Bs\right)$ (wherein X1 = 8 to 58, X2 = 48 to 83, X2>X1, and Bs = 3 to 100); and
(j) $\left(vT2s\left(X1\right)-vT2s\left(X2\right)\right) / vT 1 s \left(Bs\right)$ (wherein X1 = 8 to 58, X2 = 48 to 98, X2>X1, and Bs = 3 to 100), and
wherein Pm is selected from the group consisting of the following (a') to (p'):
(a') Tm(X1) (wherein X1 = 90 to 100);
(b') rTm(X1)
(wherein $rTm \left(X1\right) = Tm \left(X1\right) / Tm \left(Am\right) \times Qm$ X1 = 30 to 95, Am = 1 to 35, X1-Am = 10 to 94, and Qm>0);
(c') aTm(X1,X2)
(wherein $aTm \left(X1,X2\right) = \left\{Tm\left(X2\right)-Tm\left(X1\right)\right\} / \left(X2-X1\right) \times Qm ,$ X1 = 1 to 90, X2 = 25 to 100, X2-X1 = 5 to 99, and Qm>0);
(d') SLm[X1:X2]
(wherein $SLm \left[X1:X2\right] = \frac{{\sum }_{X = X 1}^{X 2} \left\{\left(X-UX\right)\times \left(Tm\left(X\right)-UTm\right)\right\}}{{\sum }_{X = X 1}^{X 2} {\left(X-UX\right)}^{2}} \times Qm$
UX is an average from X1 to X2,
UTm is an average from Tm(X1) to Tm(X2),
X1 = 1 to 95, X2 = 30 to 100, (X2-X1) = 5 to 99, and Qm>0) ;
(e') rSLm[X1:X2]
(wherein $rSLm \left[X1:X2\right] = \frac{{\sum }_{X = X 1}^{X 2} \left\{\left(X-UX\right)\times \left(rTm\left(X\right)-UrTm\right)\right\}}{{\sum }_{X = X 1}^{X 2} {\left(X-UX\right)}^{2}} \times Qm$
rTm(X) is defined in accordance with the above equation (IIb'),
UX is an average of X from X1 to X2,
UrTm is an average of rTm(X) from rTm(X1) to rTm(X2),
X1 = 1 to 70, X2 = 25 to 100, (X2-X1) = 5 to 99, Am = 5 to 95, and Qm>0);
(f') wXm[X1:X2]
(wherein $wXm \left[X1:X2\right] = \frac{{\sum }_{X = X 1}^{X 2} \left\{X\times Tm\left(X\right)\right\}}{{\sum }_{X = X 1}^{X 2} Tm \left(X\right)} \times Qm$ X1 = 1 to 45, X2 = 30 to 100, (X2-X1) = 10 to 99, and Qm>0) ;
(g') wTm[X1:X2]
(wherein $wTm \left[X1:X2\right] = \frac{{\sum }_{X = X 1}^{X 2} \left\{X\times Tm\left(X\right)\right\}}{{\sum }_{X = X 1}^{X 2} X} \times Qm$ X1 = 75 to 95, X2 = 95 to 100, (X2-X1) = 5 to 25, and Qm>0) ;
(h') wrTm[X1:X2]
(wherein $wrTm \left[X1:X2\right] = \frac{{\sum }_{X = X 1}^{X 2} \left\{X\times rTm\left(X\right)\right\}}{{\sum }_{X = X 1}^{X 2} X}$
rTm(X) is defined in accordance with the above equation (IIb'),
X1 = 1 to 90, X2 = 30 to 100, (X2-X1) = 5 to 99, Am = 3 to 34, and Qm>0);
(i') $vT 2 m \left(X1\right) / vT 1 m \left(X2\right)$ (wherein X1 = 3 to 93 and X2 = 3 to 88);
(j') $vT 2 m \left(X1\right) / vT 2 m \left(X2\right)$ (wherein X1 = 38 to 63, X2 = 63 to 93, and X2>X1);
(k') $\left(vT2m\left(X1\right)-vT1m\left(X2\right)\right) / vT 2 s \left(Bs\right)$ (wherein X1 = 3 to 93, X2 = 3 to 88, and Bm = 3 to 100);
(l') $\left(vT2m\left(X1\right)-vT2m\left(X2\right)\right) / vT 2 s \left(Bs\right)$ (wherein X1 = 28 to 63, X2 = 68 to 93, X2>X1, and Bm = 3 to 100);
(m') $\left(vT2m\left(X1\right)-vT1m\left(X2\right)\right) / vT 1 s \left(Bs\right)$ (wherein X1 = 3 to 93, X2 = 3 to 93, and Bm = 3 to 100);
(n') $\left(vT2m\left(X1\right)-vT2m\left(X2\right)\right) / vT 1 s \left(Bs\right)$ (wherein X1 = 23 to 63, X2 = 63 to 93, X2>X1, and Bm = 3 to 100);
(o') iCmax_m
(wherein iCmax_m is a reciprocal of Cmax_m,
Cmax_m is a maximum value of Cm(i), $Cm \left(i\right) = Vm \left(i\right) \times \left(Q/Em\right)$
in the equation (XIb), Vm(i) is a first derivative curve of a coagulation reaction curve of the specimen M, i is time or the number of measurement points, and Q>0); and
(p') $iwCm \left[X1:X2\right]$ (wherein $iwCm \left[X1:X2\right] = \frac{{\sum }_{i = X 1}^{X 2} i}{{\sum }_{i = X 1}^{X 2} \left\{i\times Cm\left(i\right)\right\}} \times Qm$
Cm(i) is defined in accordance with the above equation (XIb),
X1 and X2 satisfy Cm(X1) = Cm(X2) = Vm(i)×[Q/(Em×S%) ], provided that X1<VmaxT_m<X2 and Qm>0, $S = 1 to 100 ,$ and
VmaxT_m is time or a measurement point at which the first derivative curve of the coagulation reaction curve of the specimen M reaches a maximum value).

3. The method according to claim 1, wherein
2) comprises estimating that the specimen S is coagulation factor inhibitor-positive when Ps is equal to or larger than a threshold value for Ps; and
3) comprises estimating that the specimen S is lupus anticoagulant-positive when Pm is equal to or larger than a threshold value for Pm, or that the specimen S is coagulation factor-deficient when Pm is equal to or less than the threshold value for Pm.

4. The method according to claim 1, which comprises, as 2) and 3), plotting Ps and Pm of the specimen S on a two-dimensional map, and estimating that the specimen S is coagulation factor inhibitor-positive, lupus anticoagulant-positive, or coagulation factor-deficient, based on the position of the plotted point on the two-dimensional map.

5. The method according to claim 1, which comprises:
calculating a parameter Pd for a specimen S which has been estimated to be lupus anticoagulant-positive, and
estimating whether the specimen S is suspected to be derived from a patient with acquired hemophilia A based on Pd,
wherein
Pd is calculated based on Td(X), $Td \left(X\right) = Ts \left(X\right) - Tm \left(X\right) ,$ and $0 < X \leq 100 .$

6. The method according to claim 5, wherein Pd is selected from the group consisting of the following (a") to (e"):
(a") Td(X1) (wherein X1 = 43 to 100);
(b") Td(X2)-Td(X1) (wherein X1 = 1 to 80, X2 = 15 to 100, and X2-X1 = 5 to 99);
(c") SLd[X1:X2]
(wherein $SLd \left[X1:X2\right] = \frac{{\sum }_{X = X 1}^{X 2} \left\{\left(X-UX\right)\times \left(Td\left(X\right)-UTd\right)\right\}}{{\sum }_{X = X 1}^{X 2} {\left(X-UX\right)}^{2}} \times Qd$ X1 = 1 to 95, X2 = 25 to 100, X2-X1 = 5 to 99, UX is an average of X from X1 to X2, UTd is an average of Td(X) from Td(X1) to Td(X2), and Qd>0);
(d") wXd[X1:X2]
(wherein $wXd \left[X1:X2\right] = \frac{{\sum }_{X = X 1}^{X 2} \left\{X\times Td\left(X\right)\right\}}{{\sum }_{X = X 1}^{X 2} Td \left(X\right)} \times Qd$ X1 = 1 and X2 = 50 to 90, or X1 = 15 to 25, X2 = 25 to 45, and X2-X1 = 5 to 30, and Qd>0); and
(e") wTd[X1:X2]
(wherein $wTd \left[X1:X2\right] = \frac{{\sum }_{X = X 1}^{X 2} \left\{X\times Td\left(X\right)\right\}}{{\sum }_{X = X 1}^{X 2} X} \times Qd$ X1 = 1 to 95, X2 = 45 to 100, X2-X1 = 5 to 99, and Qd>0).

7. The method according to claim 5, which comprises estimating that the specimen S is suspected to be derived from a patient with acquired hemophilia A when Pd is equal to or larger than a threshold value for Pd.

8. A program for implementing the method according to any one of claims 1 to 7.

9. A device for implementing the method according to any one of claims 1 to 7.
